# EUROPEAN PATENT APPLICATION

(11) **EP 4 549 436 A1**
(43) Date of publication of application: **07.05.2025**
(21) Application number: 23831936.2
(22) Date of filing: 29.06.2023
(51) Int. Cl.: C07D 417/10, A61K 31/4439, A61K 31/426, A61K 31/5377, A61K 31/4436, A61P 25/28, C07D 277/22, C07D 409/10, C07D 401/10, A23L 33/10

(54) **NOVEL INDENONE DERIVATIVES AND USES THEREOF**

(30) Priority: 29.06.2022 KR 20220079766
(71) Applicant: Amyloid Solution Inc., Seongnam-si, Gyeonggi-do 13486 (KR)
(72) Inventor: KIM, Seong Muk, Seoul 07651 (KR); KIM, Hye Ju, Hwaseong-si, Gyeonggi-do 18444 (KR)
(74) Representative: Vossius & Partner Patentanwälte Rechtsanwälte mbB
(86) International application number: PCT/KR2023/009159
(87) International publication number: WO 2024/005578

(57) **Abstract**

The present disclosure relates to a novel indenone derivative or an isomer or pharmaceutically acceptable salt thereof, and use thereof. The indenone derivative according to the present disclosure may be effectively used for the prevention or treatment of a neurodegenerative brain disease by (i) inhibiting amyloid-beta aggregation and/or disaggregating amyloid-beta aggregates, (ii) inhibiting tau protein aggregation and/or disaggregating tau protein aggregates, and/or (iii) inhibiting the hyperphosphorylation of tau protein.

## Description

### Technical Field

The present disclosure relates to a novel indenone derivative or an isomer or pharmaceutically acceptable salt thereof, and use thereof for (i) inhibiting amyloid-beta aggregation and/or disaggregating amyloid-beta aggregates, (ii) inhibiting tau protein aggregation and/or disaggregating tau protein aggregates, and/or (iii) inhibiting the hyperphosphorylation of tau protein, and use thereof for the prevention or treatment of a neurodegenerative brain disease.

### Background Art

Neurodegenerative brain diseases are diseases that cause degenerative changes in nerve cells of the central nervous system and thus leads to various symptoms such as impaired motor and sensory functions, and inhibition of higher-order causative functions such as memory, learning, and arithmetic reasoning. Common neurodegenerative brain diseases include Alzheimer's disease, Parkinson's disease, memory disorders, and the like. In neurodegenerative brain diseases, neuronal cell death occurs due to rapid or slow progression of necrosis or apoptosis. Therefore, for the development of a method of preventing, regulating, and treating central nervous system diseases, the mechanism of death of neurons needs to be understood.

As causative agents for neurodegenerative brain diseases, two types of proteins, namely amyloid-beta and tau protein, are attracting attention.

Human amyloid-beta is a peptide molecule containing about 36-43 amino acids, and the self-assembly of amyloid-beta into oligomers or aggregates is known to be involved in the development of neurological diseases such as Alzheimer's disease. Specifically, amyloid-beta peptide molecules are obtained by cleaving amyloid precursor protein (APP; UniProtKB P05067) with beta secretase and gamma secretase, and these amyloid-beta peptide molecules aggregate to form neurotoxic oligomers, thereby causing neurodegenerative brain diseases.

In addition, tau protein consists of four parts: the N-terminal overhang, the proline aggregation domain, the microtubule-binding domain, and the C-terminus, and abnormal hyperphosphorylation or aggregation of tau protein in neurons of the central nervous system is known to cause neurodegenerative brain diseases such as Parkinson's disease and tauopathy.

Therefore, a material that inhibits amyloid-beta aggregation or disaggregating amyloid-beta aggregates, inhibiting tau protein aggregation or disaggregating tau protein aggregates, or inhibits the hyperphosphorylation of tau protein can be suggested as a therapeutic agent for neurodegenerative brain diseases.

### Detailed Description of the Disclosure

### Technical Problem

An objective of the present disclosure is to provide a novel indenone derivative or an isomer or pharmaceutically acceptable salt thereof that is effective in (i) inhibiting amyloid-beta aggregation and/or disaggregating amyloid-beta aggregates, ii) inhibiting tau protein aggregation and/or disaggregating tau protein aggregates, and/or (iii) inhibiting the hyperphosphorylation of tau protein.

Another objective of the present disclosure is to provide a pharmaceutical composition for preventing or treating a neurodegenerative brain disease, including the novel indenone derivative or the isomer or pharmaceutically acceptable salt thereof as an active ingredient.

Another objective of the present disclosure is to provide a health functional food for preventing or ameliorating a neurodegenerative brain disease, including the novel indenone derivative or the isomer or sitologically acceptable salt thereof as an active ingredient.

Another objective of the present disclosure is to provide a composition including the novel indenone derivative or the isomer or acceptable salt thereof.

Another objective of the present disclosure is to provide a pharmaceutical composition including the novel indenone derivative or the isomer or pharmaceutically acceptable salt thereof.

Another objective of the present disclosure is to provide a method of (i) inhibiting amyloid-beta aggregation and/or disaggregating amyloid-beta aggregates, (ii) inhibiting tau protein aggregation or disaggregating tau protein aggregates, and/or inhibiting the phosphorylation of tau protein, or (iii) treating or preventing a neurodegenerative brain disease, the method including administering the novel indenone derivative or the isomer or pharmaceutically acceptable salt thereof to a subject in need thereof.

Another objective of the present disclosure is to provide use of the novel indenone derivative or the isomer or pharmaceutically acceptable salt thereof for (i) inhibiting amyloid-beta aggregation and/or disaggregating amyloid-beta aggregates, (ii) inhibiting tau protein aggregation or disaggregating tau protein aggregates and/or inhibiting the phosphorylation of tau protein, or (iii) treating or preventing a neurodegenerative brain disease.

### Technical Solution

Hereinafter, the present disclosure will be described in more detail.

Throughout the specification, "including" an element means that other elements may be further included rather than excluding other elements, unless specifically stated otherwise.

The term "isomer" as used herein refers to a compound of the present disclosure or a salt thereof that has the same chemical formula or molecular formula but is structurally or sterically different. Such isomers include structural isomers such as tautomers, R or S isomers having an asymmetric carbon center, stereoisomers such as geometric isomers (trans, cis), and optical isomers (enantiomers). All these isomers and mixtures thereof also fall within the scope of the present disclosure.

The term "halogen" refers to F, Cl, Br, or I, unless otherwise specified.

The term "C₁₋₆ alkyl" refers to a linear or branched saturated hydrocarbon moiety having 1 to 6 carbon atoms. Specific examples of C₁₋₆ alkyl include, but are not limited to, methyl, ethyl, n-propyl, isopropyl, n-butyl, isobutyl, tert-butyl, sec-butyl, n-pentyl, isopentyl, neopentyl, tert-pentyl, 1-methylbutyl, n-hexyl, 1-methylpentyl, 2-methylpentyl, 4-methyl-2-pentyl, 3,3-dimethylbutyl, and 2-ethylbutyl. In an embodiment, the alkyl group may be substituted with one or more substituent, for example, 1 to 3 halogens or C₁₋₆ alkyls.

The term "C₁₋₆ alkoxy" refers to -O-C₁₋₆ alkyl, and examples thereof include, but are not limited to, methoxy, ethoxy, n-propoxy, isopropoxy, n-butoxy, isobutoxy, tert-butoxy, n-pentoxy, isopentoxy, sec-pentoxy, neopentoxy, and hexyloxy. In an embodiment, the alkoxy group may be substituted with one or more substituent, for example, 1 to 3 halogens or C₁₋₆ alkyls.

The term "aryl" refers to a monocyclic or bicyclic hydrocarbon aromatic ring. That is, the aryl as used herein may include biaryls such as phenyl, naphthyl, and the like, unless otherwise defined. In an embodiment of the present disclosure, C₆₋₁₀ aryl refers to an aromatic ring having 6 to 10 carbon atoms. In an embodiment, 0, 1, 2, 3, 4, 5 or 6 atoms of each ring of the aryl group may be substituted with a substituent.

The term "heteroaryl" refers to an aromatic 5- to 10-membered monocyclic or bicyclic heterocycle containing 1 to 4 heteroatoms selected from N, O, and S. That is, heteroaryl refers to a 5- or 6-membered aromatic heterocycle containing 1 to 4 heteroatoms selected from N, O, and S, or a bicyclic ring in which the heteroaryl ring is fused to a benzene ring or another heteroaryl ring. In an embodiment, 0, 1, 2, 3, or 4 atoms of each ring of the heteroaryl group may be substituted with a substituent. Examples of monocyclic heteroaryls may include, but are not limited to, thiazolyl, oxazolyl, thiophenyl, furanyl, pyrrolyl, imidazolyl, isoxazolyl, isothiazolyl, pyrazolyl, triazolyl, triazinyl, thiadiazolyl, tetrazolyl, oxadiazolyl, pyridinyl, pyridazinyl, pyrimidinyl, pyrazinyl, and similar groups. Examples of bicyclic heteroaryls may include, but are not limited to, indolyl, azaindolyl, indolinyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, quinoxalinyl, purinyl, furopyridinyl, and similar groups.

The term "heterocyclyl" refers to a 5- to 10-membered saturated or partially unsaturated carbocyclic ring containing 1 to 4 heteroatoms selected from N, O, and S, other than carbon atoms. In an embodiment, the heterocyclyl may be a 5- or 6-membered aliphatic heterocycle, or a bicyclic ring in which the heterocyclyl ring is fused to a benzene ring or another heterocyclyl ring. In an embodiment, 0, 1, 2, 3, or 4 atoms of each ring of the heterocyclyl group may be substituted with a substituent. For example, heterocyclyl may be azetidinyl, pyrrolidinyl, tetrahydrofuranyl, tetrahydro-thienyl, pyrazolidinyl, imidazolidinyl, oxazolidinyl, isoxazolidinyl, thiazolidinyl, dioxolyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, indolyl, isoindolyl, dihydroindolyl, dioxoisoindolinyl, dihydrofuryl, dihydroimidazolinyl, dihydroxazolyl, dihydrobenzodioxynyl, tetrahydropyridinyl, dihydropyranyl, dihydrobenzofuranyl, benzodioxolyl, or benzodioxanyl.

The term "substitution" refers to replacement of a hydrogen atom in a molecular structure with a substituent such that a chemically stable compound results from such substitution without exceeding the valence on the designated atom. For example, "Group A is substituted with substituent B" may mean that a hydrogen atom bonded to an atom such as carbon constituting the skeleton of group A is substituted with substituent B, so that group A and group B form a covalent bond.

The present disclosure provides a compound of Formula 1 below or an isomer or pharmaceutically acceptable salt thereof: 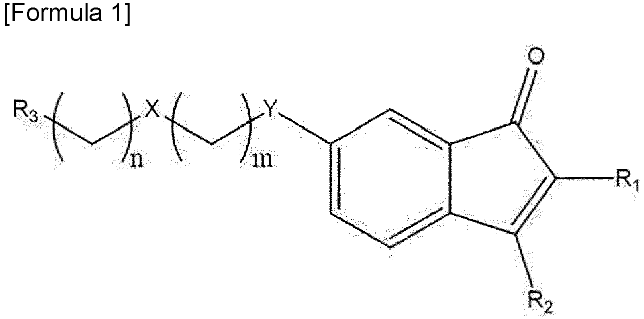
wherein, in Formula 1,
X is a direct bond or O;
Y is O or S;
n is an integer from 0 to 5;
m is an integer from 1 to 5;
R₁ is halogen, C₁₋₆ alkyl, C₆₋₁₀ aryl, -C₁₋₆ alkyl-C₆₋₁₀ aryl, or 5- or 6-membered heteroaryl, wherein the C₁₋₆ alkyl, the C₆₋₁₀ aryl, the -C₁₋₆ alkyl-C₆₋₁₀ aryl, and the 5- or 6-membered heteroaryl may each independently be unsubstituted or substituted with 1 to 4 halogens, hydroxy, CN, -NH₂, -CF₃, C₁₋₆ alkyl, -C₁₋₆ alkyl-CN, -O-C₁₋₆ alkyl, -NH-C₁₋₆ alkyl, or -N(C₁₋₆ alkyl)₂;
R₂ is 5- or 6-membered heteroaryl, wherein the 5- or 6-membered heteroaryl may be unsubstituted or substituted with 1 to 4 halogens, -CF₃, or C₁₋₆ alkyl;
R₃ is C₆₋₁₀ aryl, 5- or 6-membered heteroaryl, or 5- to 10-membered heterocyclyl, wherein the C₆₋₁₀ aryl, the 5- or 6-membered heteroaryl, and the 5-to 10-membered heterocyclyl may each independently be unsubstituted or substituted with 1 to 4 halogens, hydroxy, CN, -NH₂, -CF₃, C₁₋₆ alkyl, -C₁₋₆ alkyl-CN, -O-C₁₋₆ alkyl, -NH-C₁₋₆ alkyl, or -N(C₁₋₆ alkyl)₂; and

The heteroaryl is an aromatic heterocycle containing 1 to 4 heteroatoms selected from N, O, and S, and the heterocyclyl is an aliphatic heterocycle containing 1 to 4 heteroatoms selected from N, O, and S.

In an embodiment, in the compound of Formula 1, R₁ is halogen, phenyl, pyridinyl, pyrimidinyl, or thiophenyl, wherein the phenyl, the pyridinyl, the pyrimidinyl, and the thiophenyl may each independently be unsubstituted or substituted with 1 to 4 halogens, hydroxy, CN, -CF₃, C₁₋₆ alkyl, -C₁₋₆ alkyl-CN, or -O-C₁₋₆ alkyl.

Specifically, R₁ may be, for example, bromo, 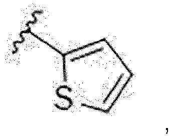 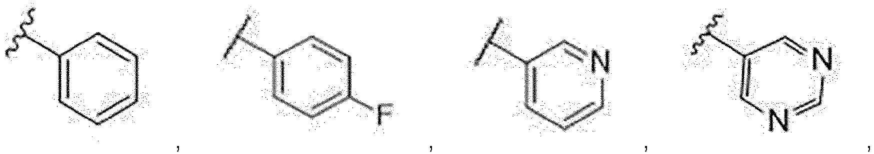 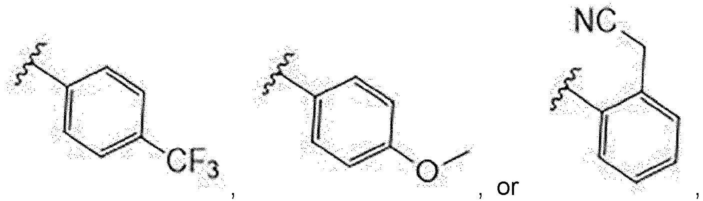 but the present disclosure is not limited thereto.

In an embodiment, in the compound of Formula 1, R₂ is furanyl, thiophenyl, thiazolyl, or pyrazolyl, wherein the thiazolyl, the furanyl, the thiophenyl, and the pyrazolyl may each independently be unsubstituted or substituted with 1 to 4 halogens, -CF₃, or C₁₋₆ alkyl.

Specifically, R₂ may be, for example, 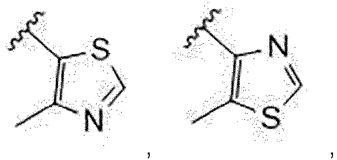 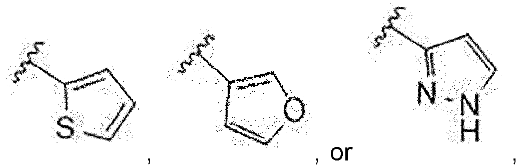 but the present disclosure is not limited thereto.

In an embodiment, in the compound of Formula 1, R₃ is phenyl, pyridinyl, morpholinyl, or benzodioxolyl, wherein the phenyl, the pyridinyl, the morpholinyl, and the benzodioxolyl may each independently be unsubstituted or substituted with 1 to 4 halogens, hydroxy, -NH₂, -CF₃, C₁₋₆ alkyl, -O-C₁₋₆ alkyl, -NH-C₁₋₆ alkyl, or -N(C₁₋₆ alkyl)₂.

**Specifically,** R₃ may be, for example, 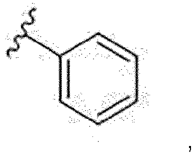 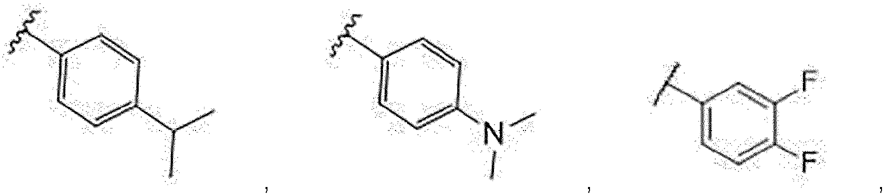 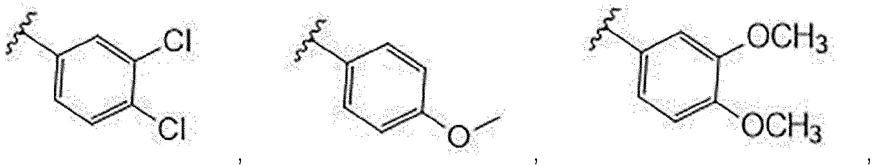 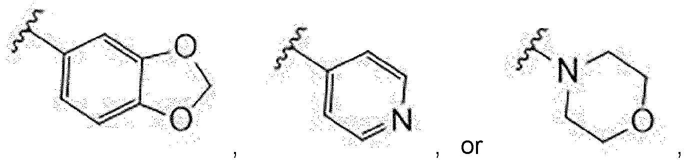 but the present disclosure is not limited thereto.

In an embodiment, in the compound of Formula 1, R₁ is halogen, phenyl, pyridinyl, pyrimidinyl, or thiophenyl, wherein the phenyl, the pyridinyl, the pyrimidinyl, and the thiophenyl may each independently be unsubstituted or substituted with 1 to 4 halogens, hydroxy, CN, -CF₃, C₁₋₆ alkyl, -C₁₋₆ alkyl-CN, or -O-C₁₋₆ alkyl,
R₂ is furanyl, thiophenyl, thiazolyl, or pyrazolyl, wherein the thiazolyl, the furanyl, the thiophenyl, and the pyrazolyl may each independently be unsubstituted or substituted with 1 to 4 halogens, -CF₃, or C₁₋₆ alkyl, and
R₃ is phenyl, pyridinyl, morpholinyl, or benzodioxolyl, wherein the phenyl, the pyridinyl, the morpholinyl, and the benzodioxolyl may each independently be unsubstituted or substituted with 1 to 4 halogens, hydroxy, -NH₂, -CF₃, C₁₋₆ alkyl, -O-C₁₋₆ alkyl, -NH-C₁₋₆ alkyl, or -N(C₁₋₆ alkyl)₂.

Specific examples of the compound of Formula 1 according to the present disclosure are, but are not limited to, the following compounds:
(1) 3-(4-methylthiazol-5-yl)-6-(3-phenylpropoxy)-2-(pyridin-3-yl)-1H-inden-1-one;
(2) 2-(2-(3-(4-methylthiazol-5-yl)-1-oxo-6-(3-phenylpropoxy)-1H-inden-2-yl)phenyl)acetonitrile;
(3) 2-bromo-3-(4-methylthiazol-5-yl)-6-(3-phenylpropoxy)-1H-inden-1-one;
(4) 3-(5-methylthiazol-4-yl)-6-(3-morpholinopropoxy)-2-(pyridin-3-yl)-1H-inden-1-one;
(5) 2-bromo-3-(5-methylthiazol-4-yl)-6-(3-morpholinopropoxy)-1H-inden-1-one;
(6) 6-(3-phenylpropoxy)-2-(pyridin-3-yl)-3-(thiophen-2-yl)-1H-inden-1-one;
(7) 6-(3-phenylpropoxy)-3-(1H-pyrazol-3-yl)-2-(pyridin-3-yl)-1H-inden-1-one;
(8) 3-(5-methylthiazol-4-yl)-6-(3-phenylpropoxy)-2-(pyridin-3-yl)-1H-inden-1-one;
(9) 3-(furan-3-yl)-6-(3-phenylpropoxy)-2-(pyridin-3-yl)-1H-inden-1-one;
(10) 3-(4-methylthiazol-5-yl)-2-phenyl-6-(3-phenylpropoxy)-1H-inden-1-one;
(11) 3-(4-methylthiazol-5-yl)-6-(3-phenylpropoxy)-2-(pyrimidin-5-yl)-1H-inden-1-one;
(12) 3-(4-methylthiazol-5-yl)-6-(3-phenylpropoxy)-2-(thiophen-2-yl)-1H-inden-1-one;
(13) 3-(4-methylthiazol-5-yl)-2-(pyridin-3-yl)-6-(3-(pyridin-4-yl)propoxy)-1H-inden-1-one;
(14) 3-(4-methylthiazol-5-yl)-6-phenethoxy-2-(pyridin-3-yl)-1H-inden-1-one;
(15) 3-(4-methylthiazol-5-yl)-6-(4-phenylbutoxy)-2-(pyridin-3-yl)-1H-inden-1-one;
(16) 6-((benzyloxy)methoxy)-3-(4-methylthiazol-5-yl)-2-(pyridin-3-yl)-1H-inden-1-one;
(17) 3-(4-methylthiazol-5-yl)-6-(2-phenoxyethoxy)-2-(pyridin-3-yl)-1H-inden-1-one;
(18) 2-(4-methoxyphenyl)-3-(5-methylthiazol-4-yl)-6-phenethoxy-1H-inden-1-one;
(19) 2-(4-methoxyphenyl)-3-(5-methylthiazol-4-yl)-6-(4-phenylbutoxy)-1H-inden-1-one;
(20) 6-((benzyloxy)methoxy)-2-(4-methoxyphenyl)-3-(5-methylthiazol-4-yl)-1H-inden-1-one;
(21) 2-(4-methoxyphenyl)-3-(5-methylthiazol-4-yl)-6-(2-phenoxyethoxy)-1H-inden-1-one;
(22) 2-(4-fluorophenyl)-6-(2-(4-methoxyphenoxy)ethoxy)-3-(5-methylthiazol-4-yl)-1H-inden-1-one;
(23) 6-(2-(3,4-dimethoxyphenoxy)ethoxy)-2-(4-fluorophenyl)-3-(5-methylthiazol-4-yl)-1H-inden-1-one;
(24) 6-(2-(benzo[d][1,3]dioxol-5-yloxy)ethoxy)-2-(4-fluorophenyl)-3-(5-methylthiazol-4-yl)-1H-inden-1-one;
(25) 2-(4-fluorophenyl)-3-(5-methylthiazol-4-yl)-6-(2-(pyridin-4-yloxy)ethoxy)-1H-inden-1-one;
(26) 6-(2-(3,4-dichlorophenoxy)ethoxy)-3-(5-methylthiazol-4-yl)-2-(pyrimidin-5-yl)-1H-inden-1-one;
(27) 6-(2-(3,4-difluorophenoxy)ethoxy)-3-(5-methylthiazol-4-yl)-2-(pyrimidin-5-yl)-1H-inden-1-one;
(28) 3-(5-methylthiazol-4-yl)-6-phenethoxy-2-(thiophen-2-yl)-1H-inden-1-one;
(29) 3-(5-methylthiazol-4-yl)-6-(4-phenylbutoxy)-2-(thiophen-2-yl)-1H-inden-1-one;
(30) 6-((benzyloxy)methoxy)-3-(5-methylthiazol-4-yl)-2-(thiophen-2-yl)-1H-inden-1-one;
(31) 3-(5-methylthiazol-4-yl)-6-(2-phenoxyethoxy)-2-(thiophen-2-yl)-1H-inden-1-one;
(32) 6-(2-(4-methoxyphenoxy)ethoxy)-3-(5-methylthiazol-4-yl)-2-(4-(trifluoromethyl)phenyl)-1H-inden-1-one;
(33) 6-(2-(3,4-dimethoxyphenoxy)ethoxy)-3-(5-methylthiazol-4-yl)-2-(4-(trifluoromethyl)phenyl)-1H-inden-1-one;
(34) 6-(2-(benzo[d][1,3]dioxol-5-yloxy)ethoxy)-3-(5-methylthiazol-4-yl)-2-(4-(trifluoromethyl)phenyl)-1H-inden-1-one;
(35) 3-(5-methylthiazol-4-yl)-6-(2-(pyridin-4-yloxy)ethoxy)-2-(4-(trifluoromethyl)phenyl)-1H-inden-1-one;
(36) 6-(2-(3,4-dichlorophenoxy)ethoxy)-3-(5-methylthiazol-4-yl)-2-(thiophen-2-yl)-1H-inden-1-one;
(37) 6-(2-(3,4-difluorophenoxy)ethoxy)-3-(5-methylthiazol-4-yl)-2-(thiophen-2-yl)-1H-inden-1-one;
(38) 3-(furan-3-yl)-6-phenethoxy-2-(pyridin-3-yl)-1H-inden-1-one;
(39) 3-(furan-3-yl)-6-(4-phenylbutoxy)-2-(pyridin-3-yl)-1H-inden-1-one;
(40) 6-((benzyloxy)methoxy)-3-(furan-3-yl)-2-(pyridin-3-yl)-1H-inden-1-one;
(41) 3-(furan-3-yl)-6-(2-phenoxyethoxy)-2-(pyridin-3-yl)-1H-inden-1-one;
(42) 3-(furan-3-yl)-6-(2-(4-methoxyphenoxy)ethoxy)-2-(pyridin-3-yl)-1H-inden-1-one;
(43) 6-(2-(3,4-dimethoxyphenoxy)ethoxy)-3-(furan-3-yl)-2-(pyridin-3-yl)-1H-inden-1-one;
(44) 6-(2-(benzo[d][1,3]dioxol-5-yloxy)ethoxy)-3-(furan-3-yl)-2-(pyridin-3-yl)-1H-inden-1-one;
(45) 3-(furan-3-yl)-2-(pyridin-3-yl)-6-(2-(pyridin-4-yloxy)ethoxy)-1H-inden-1-one;
(46) 6-(2-(3,4-dichlorophenoxy)ethoxy)-3-(furan-3-yl)-2-(pyridin-3-yl)-1H-inden-1-one;
(47) 6-(2-(3,4-difluorophenoxy)ethoxy)-3-(furan-3-yl)-2-(pyridin-3-yl)-1H-inden-1-one;
(48) 6-(2-(4-(dimethylamino)phenoxy)ethoxy)-3-(furan-3-yl)-2-(pyridin-3-yl)-1H-inden-1-one;
(49) 3-(furan-3-yl)-6-(2-(4-isopropylphenoxy)ethoxy)-2-(pyridin-3-yl)-1H-inden-1-one;
(50) 3-(furan-3-yl)-6-(((4-methoxybenzyl)oxy)methoxy)-2-(pyridin-3-yl)-1H-inden-1-one; or
a pharmaceutically acceptable salt thereof.

The present disclosure includes a pharmaceutically acceptable salt of the compound of Formula 1.

The pharmaceutically acceptable salt should have low toxicity to humans and should not have any negative effect on the biological activity and physicochemical properties of a parent compound.

For example, the pharmaceutically acceptable salt may be an acid addition salt formed by pharmaceutically acceptable free acid.

The free acid may be an inorganic acid or an organic acid. In this regard, the inorganic acid may be hydrochloric acid, sulfuric acid, nitric acid, phosphoric acid, perchloric acid, bromic acid, or the like, and the organic acid may be acetic acid, methanesulfonic acid, ethanesulfonic acid, p-toluene sulfuric acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, or the like.

The acid addition salt may be prepared by a conventional method, for example, by dissolving the compound of Formula 1 in an excess amount of an aqueous acid solution and precipitating the salt using a water-miscible organic solvent, such as methanol, ethanol, acetone, or acetonitrile.

In addition, the pharmaceutically acceptable salt may be an alkali metal salt (sodium salt or the like) or an alkaline earth metal salt (potassium salt or the like).

The alkali metal salt or alkaline earth metal salt may be obtained, for example, by dissolving the compound of Formula 1 in an excess amount of an alkali metal hydroxide or alkaline earth metal hydroxide solution, filtering the undissolved compound salt, and then evaporating and drying the filtrate.

In addition, the compound of the present disclosure may have a chiral carbon center and therefore, may exist in the form of an R or S isomer, a racemic compound, an individual enantiomer or mixture, or an individual diastereomer or mixture, and all of these stereoisomers and mixtures thereof may fall within the scope of the present disclosure.

In addition, the compound of the preset disclosure may include a hydrate and solvate of the compound of Formula 1. The hydrate and the solvate may be prepared using a known method, and are preferably non-toxic and water-soluble. Particularly, the hydrate and the solvate may preferably be combined with 1 to 5 molecules of water and an alcoholic solvent (particularly, ethanol or the like), respectively.

The present disclosure also provides a method of preparing the compound of Formula 1.

Specifically, the compound of Formula 1 may be prepared by methods shown in Reaction Schemes 1 to 5 below, but the preparation methods are not limited thereto. In particular, it will be fully understood by those of ordinary skill in the art that the compound of Formula 1 of the present disclosure may be prepared by various methods using techniques well known in the art.

Reaction schemes 1 to 5 below illustrate steps of a method of preparing representative compounds according to the present disclosure, and several compounds of the present disclosure may be prepared by changing reagents and solvents used in the following preparation steps or changing the reaction order.

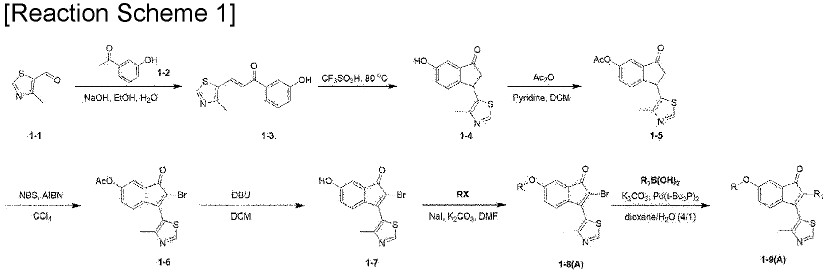

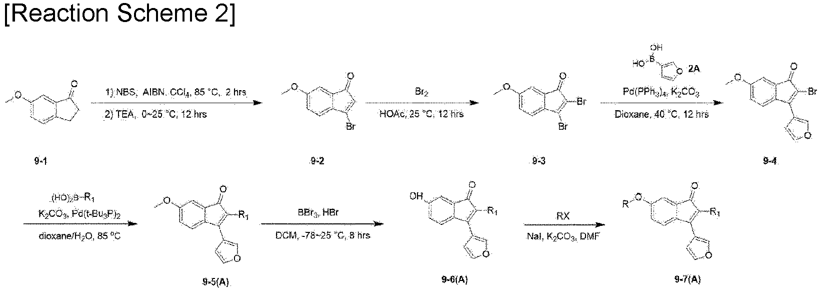

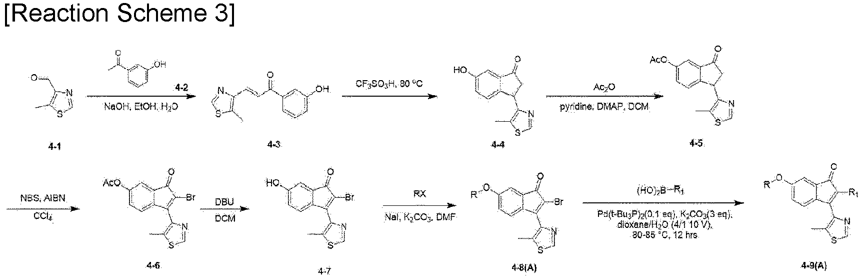

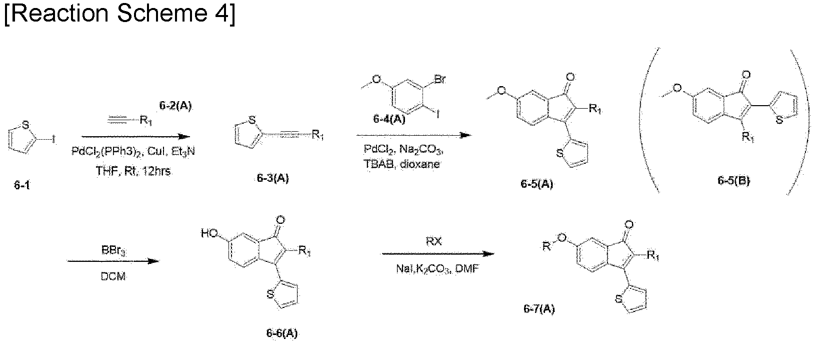

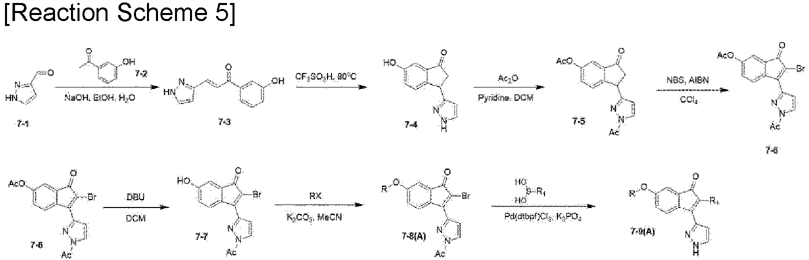

Target compounds produced according to the above reaction schemes may be separated and purified by a conventional method, for example, column chromatography, recrystallization, or the like.

The present disclosure provides a composition including the compound of Formula 1 or the isomer or acceptable salt thereof.

The present disclosure provides a composition including the compound of Formula 1 or the isomer or acceptable salt thereof as an active ingredient.

The present disclosure also provides a pharmaceutical composition for preventing or treating a neurodegenerative brain disease, including the compound of Formula 1 or the isomer or pharmaceutically acceptable salt thereof as an active ingredient.

Since the compound of Formula 1 or the isomer or pharmaceutically acceptable salt thereof has the effects of inhibiting amyloid-beta aggregation or disaggregating amyloid-beta aggregates, inhibiting tau protein aggregation or disaggregating tau protein aggregates, and inhibiting the phosphorylation of tau protein, the compound or a pharmaceutical composition including the same may be effectively used in the prevention or treatment of neurodegenerative brain diseases, e.g., diseases associated with the inhibition of amyloid-beta aggregation and/or the disaggregation of amyloid-beta aggregates, the inhibition of tau protein aggregation and/or the disaggregation of tau protein aggregates, and/or the inhibition of phosphorylation of tau protein.

The term "prevention" as used herein refers to any action that inhibits or delays the occurrence, progression and recurrence of the above diseases via administration of the compound or pharmaceutical composition according to the present disclosure, and the term "treatment" as used herein refers to any action that improves or beneficially alters the symptoms of the above diseases via administration of the compound or pharmaceutical composition according to the present disclosure.

The term "neurodegenerative brain disease" as used herein refers to all diseases associated with degenerative changes in the brain, and in particular, comprehensively refers to all diseases (brain diseases) that may be caused by one or more factor selected from amyloid-beta aggregation, tau protein aggregation, and the hyperphosphorylation of tau protein, in the brain and/or brain nerve cells.

In an embodiment, the neurodegenerative brain disease that may be prevented or treated by using the compound or pharmaceutical composition according to the present disclosure may be, but is not limited to, any one selected from the group consisting of dementia, Alzheimer's disease, preclinical Alzheimer's disease, Parkinson's disease, Huntington's disease, mild cognitive impairment, cerebral amyloid angiopathy, Down's syndrome, amyloid stroke, systemic amyloid disease, Dutch amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia, and may be any disease caused by amyloid-beta aggregation, tau protein aggregation, and/or the phosphorylation of tau protein.

In an embodiment, the present disclosure provides a pharmaceutical composition for inhibiting amyloid-beta aggregation and/or disaggregating amyloid-beta aggregates, the pharmaceutical composition including the compound of Formula 1 or the isomer or pharmaceutically acceptable salt thereof as an active ingredient.

In an embodiment, the present disclosure provides a pharmaceutical composition for inhibiting tau protein aggregation or disaggregating tau protein aggregates and/or inhibiting the phosphorylation of tau protein, the pharmaceutical composition including the compound of Formula 1 or the isomer or pharmaceutically acceptable salt thereof as an active ingredient.

The present disclosure provides use of the compound of Formula 1 or the isomer or pharmaceutically acceptable salt thereof for the prevention or treatment of a disease associated with the inhibition of amyloid-beta aggregation and/or the disaggregation of amyloid-beta aggregates, e.g., a neurodegenerative brain disease.

The present disclosure provides use of the compound of Formula 1 or the isomer or pharmaceutically acceptable salt thereof for inhibiting amyloid-beta aggregation and/or disaggregating amyloid-beta aggregates.

The present disclosure provides use of the compound of Formula 1 or the isomer or pharmaceutically acceptable salt thereof for inhibiting tau protein aggregation or disaggregating tau protein aggregates and/or inhibiting the phosphorylation of tau protein.

The present disclosure provides use of the compound of Formula 1 or the isomer or pharmaceutically acceptable salt thereof for preparing a drug for the prevention or treatment of a disease associated with the inhibition of amyloid-beta aggregation and/or the disaggregation of amyloid-beta aggregates, e.g., a neurodegenerative brain disease.

The present disclosure provides use of the compound of Formula 1 or the isomer or pharmaceutically acceptable salt thereof for preparing a drug for inhibiting amyloid-beta aggregation and/or disaggregating amyloid-beta aggregates.

The present disclosure provides use of the compound of Formula 1 or the isomer or pharmaceutically acceptable salt thereof for preparing a drug for inhibiting tau protein aggregation or disaggregating tau protein aggregates and/or inhibiting the phosphorylation of tau protein.

The present disclosure also provides a method of preventing or treating a disease associated with the inhibition of amyloid-beta aggregation and/or the disaggregation of amyloid-beta aggregates, the method including a step of administering the compound of Formula 1 or the isomer or pharmaceutically acceptable salt thereof to a subject in need of the prevention or treatment of a neurodegenerative brain disease. The method may further include, before the administration step, a step of identifying a subject in need of the prevention and/or treatment of a neurodegenerative brain disease.

The present disclosure also provides a method of inhibiting amyloid-beta aggregation and/or disaggregating amyloid-beta aggregates, the method including a step of administering the compound of Formula 1 or the isomer or pharmaceutically acceptable salt thereof to a subject in need of the inhibition of amyloid-beta aggregation and/or the disaggregation of amyloid-beta aggregates. The method may further include, before the administration step, a step of identifying a subject in need of the inhibition of amyloid-beta aggregation and/or the disaggregation of amyloid-beta aggregates.

The present disclosure provides a method of inhibiting tau protein aggregation or disaggregating tau protein aggregates and/or inhibiting the phosphorylation of tau protein, the method including a step of administering the compound of Formula 1 or the isomer or pharmaceutically acceptable salt thereof to a subject in need of the inhibition of tau protein aggregation and/or the disaggregation of tau protein aggregates, and/or the inhibition of phosphorylation of tau protein. The method may further include, before the administration step, a step of identifying a subject in need of the inhibition of tau protein aggregation, the disaggregation of tau protein aggregates, and/or the inhibition of phosphorylation of tau protein.

In an embodiment, the pharmaceutical composition according to the present disclosure may be administered to a subject (patient) selected from the group consisting of:
(1) subjects (patients) who have a higher level or a higher risk of amyloid-beta aggregation than normal individuals;
(2) subjects (patients) who have a higher level or a higher risk of tau protein aggregation than normal individuals;
(3) subjects (patients) who have a higher level or a higher risk of phosphorylation of tau protein than normal individuals; and
(4) subjects (patients) corresponding to two or more of (1) to (3).

The aggregation level of the amyloid-beta or tau protein may refer to the amount (concentration) of amyloid-beta aggregates or tau protein aggregates, or a ratio of the amyloid-beta aggregate or the tau protein aggregate to the total amyloid-beta or the total tau protein.

The phosphorylation level of the tau protein may refer to the amount (concentration) of phosphorylated tau protein or the ratio of phosphorylated tau protein to the total tau protein.

The term "normal" as used herein refers to a subject not having the above-defined "neurodegenerative brain disease" among individuals to which the pharmaceutical composition is applied and individuals of the same species, or a brain tissue or cell (brain neurons) separated and/or cultured from the individuals.

The term "inhibition" as used herein refers to inhibition of any step among gene transcription, mRNA processing, translation, translocation, and maturation, or inhibition of binding between proteins, activation of proteins, or signal transduction through these.

The pharmaceutical composition of the present disclosure may include a pharmaceutically acceptable carrier, in addition to the active ingredient. In this regard, the pharmaceutically acceptable carrier, which is commonly used in formulation, may be, but is not limited to, lactose, dextrose, sucrose, sorbitol, mannitol, starch, gum acacia, calcium phosphate, alginate, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil. In addition, the pharmaceutically acceptable carrier may further include, in addition to the above ingredients, a lubricant, a wetting agent, a sweetener, a flavoring agent, an emulsifying agent, a suspension agent, a preservative, and the like.

The pharmaceutical composition of the present disclosure may be administered orally or parenterally (e.g., intravenously, subcutaneously, intraperitoneally, or topically) depending on the desired method, and the dosage thereof may vary depending on the patient's condition and body weight, the severity of disease, a drug form, administration routes, and administration time, and may be appropriately selected by those of ordinary skill in the art.

The pharmaceutical composition of the present disclosure may be administered in a pharmaceutically effective amount. The term "pharmaceutically effective amount" as used herein refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration routes, excretion rate, treatment periods, and simultaneously used drugs, and other factors well known in the medicine field.

The pharmaceutical composition according to the present disclosure may be administered as an individual therapeutic agent or in combination with other therapeutic agents, and may be administered sequentially or simultaneously with conventional therapeutic agents, and may be administered in single or multiple doses. It is important to administer the pharmaceutical composition in the minimum amount that enables achievement of the maximum effects without side effects in consideration of all the above-described factors, and this may be easily determined by those of ordinary skill in the art.

Specifically, an effective amount of the pharmaceutical composition of the present disclosure may vary according to the age, gender, condition and body weight of a patient, the absorption, inactivity and excretion rate of active ingredients in the body, the type of disease, and simultaneously used drugs. Generally, the pharmaceutical composition may be administered daily or every other day at a dose of 0.001 mg to 150 mg, preferably, 0.01 mg to 100 mg, per body weight (1 kg), or may be administered one to three times a day. However, the dosage may be increased or decreased according to administration route, the severity of obesity, gender, body weight, age, and the like, and thus, the dosage is not intended to limit the scope of the present disclosure in any way.

The present disclosure also provides a method of preventing, controlling or treating a neurodegenerative brain disease, the method including a step of administering the pharmaceutical composition to a subject. The term "subject" as used herein refers to a subject in need of the treatment of a disease and, more specifically, to a mammal, such as a human or non-human primate, mouse, dog, cat, horse, and cow.

The present disclosure also provides a health functional food for preventing and/or ameliorating a neurodegenerative brain disease, including the compound of Formula 1 or the isomer or sitologically acceptable salt thereof as an active ingredient.

The present disclosure provides a health functional food for inhibiting amyloid-beta aggregation and/or disaggregating amyloid-beta aggregates, the health functional food including the compound of Formula 1 or the isomer or sitologically acceptable salt thereof as an active ingredient.

The present disclosure also provides a health functional food for inhibiting tau protein aggregation, disaggregating tau protein aggregates, and/or inhibiting the phosphorylation of tau protein, the health functional food including the compound of Formula 1 or the isomer or sitologically acceptable salt thereof as an active ingredient.

The health functional food may refer to a food prepared using a raw material or ingredient having a nutrient that is easily deficient in daily meals or having a useful function for the human body, and may also refer to any food that helps to maintain health or to prevent and/or ameliorate a certain disease or symptom, and a final product form thereof is not particularly limited. For example, the health functional food may be selected from the group consisting of various foods, beverage compositions, and food additives, but the present disclosure is not limited thereto.

An amount of the active ingredient (i.e., the compound of Formula 1 or the isomer or sitologically acceptable salt thereof) included in the health functional food may be appropriately adjusted depending on the type of food, intended use, or the like and is not particularly limited.

The health functional food may further include one or more selected from the group consisting of various nutrients, vitamins, minerals (electrolytes), flavoring agents, such as synthetic or natural flavoring agents, coloring agents, thickeners (cheese, chocolate, and the like), pectic acid or salts thereof, alginic acid or salts thereof, organic acids, protective colloidal thickeners, pH adjusters, stabilizers, preservatives, glycerin, alcohol, carbonation agents used in carbonated beverages, and the like. A ratio of these additive is generally selected from 0.001 parts by weight to about 20 parts by weight with respect to 100 parts by weight of the total health functional food, but the present disclosure is not limited thereto.

### Advantageous Effects of Disclosure

An indenone derivative according to the present disclosure can be effectively used for the prevention or treatment of a neurodegenerative brain disease by (i) inhibiting amyloid-beta aggregation and/or disaggregating amyloid-beta aggregates, (ii) inhibiting tau protein aggregation and/or disaggregating tau protein aggregates, and/or (iii) inhibiting the hyperphosphorylation of tau protein.

### Brief Description of Drawings

FIG. 1 illustrates graphs showing the results of confirming amyloid-beta (Aβ) disaggregation of indenone derivative compounds 1 to 9 according to the present disclosure through thioflavin-T assay.
FIG. 2 illustrates graphs showing the results of confirming amyloid-beta (Aβ) disaggregation of indenone derivative compounds 10 and 12 to 19 according to the present disclosure through thioflavin-T assay.
FIG. 3 illustrates graphs showing the results of confirming amyloid-beta (Aβ) disaggregation of indenone derivative compounds 20 to 28 according to the present disclosure through thioflavin-T assay.
FIG. 4 illustrates graphs showing the results of confirming amyloid-beta (Aβ) disaggregation of indenone derivative compounds 29 to 37 according to the present disclosure through thioflavin-T assay.
FIG. 5 illustrates graphs showing the results of confirming amyloid-beta (Aβ) disaggregation of indenone derivative compounds 38 to 46 according to the present disclosure through thioflavin-T assay.
FIG. 6 illustrates graphs showing the results of confirming amyloid-beta (Aβ) disaggregation of indenone derivative compounds 47 to 50 according to the present disclosure through thioflavin-T assay.
FIG. 7 illustrates graphs showing the results of confirming tau disaggregation of indenone derivative compounds 1 to 9 according to the present disclosure through thioflavin-T assay.
FIG. 8 illustrates graphs showing the results of confirming tau disaggregation of indenone derivative compounds 10 and 12 to 19 according to the present disclosure through thioflavin-T assay.
FIG. 9 illustrates graphs showing the results of confirming tau disaggregation of indenone derivative compounds 20 to 28 according to the present disclosure through thioflavin-T assay.
FIG. 10 illustrates graphs showing the results of confirming tau disaggregation of indenone derivative compounds 29 to 37 according to the present disclosure through thioflavin-T assay.
FIG. 11 illustrates graphs showing the results of confirming tau disaggregation of indenone derivative compounds 38 to 46 according to the present disclosure through thioflavin-T assay.
FIG. 12 illustrates graphs showing the results of confirming tau disaggregation of indenone derivative compounds 47 to 50 according to the present disclosure through thioflavin-T assay.
FIG. 13 illustrates graphs showing the effect of reducing amyloid plaques according to treatment with indenone derivative compounds in a 5xFAD TG mouse Alzheimer model according to an aspect.
FIG. 14 illustrates graphs showing the effect of reducing tau aggregates according to treatment with indenone derivative compounds in a PS19 TG mouse Alzheimer model according to an aspect.

### Mode of Disclosure

Hereinafter, exemplary embodiments will be described to aid in understanding of the present disclosure. However, the following examples will be provided to facilitate understanding of the present disclosure and are not intended to limit the scope of the present disclosure. As embodiments may allow for various changes, these embodiments are not limited by the following examples and may be embodied in various forms.

Abbreviations used in the present embodiments are as follows:
LCMS liquid chromatography mass spectrometry
HPLC high-performance liquid chromatography
TLC thin layer chromatography
NMR nuclear magnetic resonance
M+ parent molecular ion
Et ethyl
W watt
T temperature
eq equvalent
N normal (equivalents per liter)
N₂ nitrogen
Psi Pounds per Square inch
PdCL₂ Palladium(II) chloride
Pd₂(dba)₃ Tris(debenzylideneacetone)dipalladium(0)
Pd(dtbpf)Cl₂ [1,1'-bis(di-tert-butylphosphino)ferrocene]dichloropalladium(II)
Pd(dppf)Cl₂ [1,1'-bis(diphenylphosphino)ferrocene]dichloropalladium(II)
PdCl₂(PPh₃)₂ dichloropalladium(triphenylphosphine)
[(t-Bu)₃PH]BF₄ [tri(tert-butyl)phosphonium]tetrafluoroborate)
ACN acetonitrile
Ac₂O acetic anhydride
AIBN 2,2-azobis(2-methylpropionitrile)
DCM dichloromethane
DIAD diisopropyl azodicarboxylate
DMAP 4-dimethylaminopyridine
DMF dimethylformamide
DMSO dimethyl sulfoxide
HOAc acetic acid
TBAB tetrabutylammonium bromide
MTBE methyl tertiary butyl ether
TFA trifluoroacetic acid
THF tetrahydrofuran
PE petroleum ether
Py pyridine
EA ethyl acetate= EtOAc

### General Experimental Method

1H NMR spectra were recorded at a Varian Mercury 400 MHz, and trimethylsilyl (TMS) was used as an internal standard.

LCMS measurements were performed using an Agilent LC/MSD 1200 series quadrupole mass spectrometer (Column: Ultimate XB-C18 (50 X 4.6 mm, 5 µm) that operates in an ES (+) or (-) ionization mode: T = 30oC; flow rate = 1.5 mL/min; detected wavelength: 214 nm and 254 nm.

### Preparation of Compounds

### Example 1. 3-(4-methylthiazol-5-yl)-6-(3-phenylpropoxy)-2-(pyridin-3-yl)-1H-inden-1-one (Compound 1)

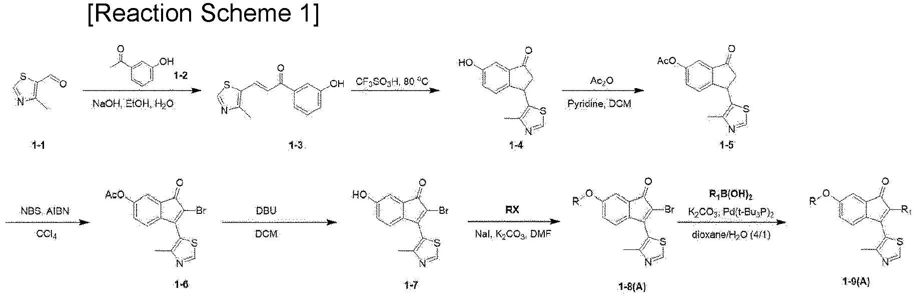

### Step 1: Synthesis of (E)-1-(3-hydroxyphenyl)-3-(4-methylthiazol-5-yl)prop-2-en-1-one (1-3)

Compound 1-1 (5.00 g, 39.3 mmol) and Compound 1-2 (5.35 g, 39.3 mmol, 4.87 mL) were mixed with ethanol (50.0 mL), and then NaOH (2.36 g, 58.9 mmol) in H₂O (20.0 mL) was added thereto at 0 °C in atmosphere including nitrogen. The mixture was stirred at 20 °C for 12 hours. It was confirmed by TLC (DCM: methanol = 10/1) that the reactants were fully consumed. The mixture was adjusted to pH 7 with 1N HCl, and then filtered and concentrated in a vacuum to thereby obtain Compound 1-3 (6.00 g, 24.46 mmol, yield: 62.21 %) as a yellow solid.

### Step 2: Synthesis of 6-hydroxy-3-(4-methylthiazol-5-yl)-2,3-dihydro-1H-inden-1-one (1-4)

Compound 1-3 (6.00 g, 24.5 mmol) was dissolved in triflic acid (30.0 mL), and then the mixture was stirred at 80 °C for 16 hours. It was confirmed by TLC (PE: EA= 1/1) that the reactants were fully consumed. The mixture was adjusted to pH 8 with an aqueous NaHCO₃ solution, and then filtered and concentrated in a vacuum to thereby obtain Compound 1-4 (5.1 g, 20.8 mmol, yield: 85.00 %) as a brown solid.

### Step 3: Synthesis of 1-(4-methylthiazol-5-yl)-3-oxo-2,3-dihydro-1H-inden-5-yl acetate (1-5)

Compound 1-4 (2.00 g, 8.15 mmol) and Ac₂O (4.16 g, 40.77 mmol, 3.82 mL) were mixed with DCM (20 mL), and then pyridine (3.22 g, 40.77 mmol, 3.29 mL) were added thereto at 0 °C in atmosphere including nitrogen. The mixture was stirred at 20 °C for 12 hours. It was confirmed by TLC (PE: EA= 1/1) that the reactants were fully consumed. The mixture was diluted with DCM (100 mL), and extracted with brine (30 mL x 3). The resulting extract was dried with anhydrous Na₂SO₄, and then concentrated in a vacuum to thereby obtain a residue. The residue was purified by flash silica gel chromatography to thereby obtain Compound 1-5 (850 mg, 2.96 mmol, yield: 36.28 %) as a yellow solid.

### Step 4: Synthesis of 2-bromo-3-(4-methylthiazol-5-yl)-1-oxo-1H-inden-6-yl acetate (1-6)

Compound 1-5 (650 mg, 2.26 mmol) and NBS (886 mg, 4.98 mmol) were mixed with carbon tetrachloride (5.00 mL), and then AIBN (37.1 mg, 226 µmol) was added thereto in atmosphere including nitrogen. The mixture was stirred at 80 °C for 1 hour. Thereafter, the reaction mixture was stirred at 400 W and 80 °C for 1.5 hours. It was confirmed by LCMS that the reactants were fully consumed, and Compound 1-6 was synthesized. The mixture was diluted with DCM (100 mL), and extracted with brine (30 mL x 3). The resulting extract was dried with anhydrous Na₂SO₄ and filtered, and then concentrated in a vacuum to thereby obtain Compound 1-6 (890 mg) as brown oil.

### Step 5: Synthesis of 2-bromo-6-hydroxy-3-(4-methylthiazol-5-yl)inden-1-one (1-7)

Compound 1-6 (780 mg, 2.14 mmol) was mixed with DCM (4.00 mL), and then DBU (326 mg, 2.14 mmol, 322 µL) was added thereto in atmosphere including nitrogen. The mixture was stirred at 20 °C for 12 hours. It was confirmed by TLC (PE: EA= 1/1) that the reactants were fully consumed. The mixture was diluted with DCM (100 mL), and extracted with brine (30 mL x 3). The resulting extract was dried with anhydrous Na₂SO₄, and then concentrated in a vacuum to thereby obtain a residue. The residue was purified by flash silica gel chromatography to thereby obtain Compound 1-7 (110 mg, 341 µmol, yield: 15.9 %) as red oil.

### Step 6: Synthesis of 2-bromo-3-(4-methylthiazol-5-yl)-6-(3-phenylpropoxy)-1H-inden-1-one (Compound 3)

Compound 1-7 (110 mg, 341 µmol) and (3-bromopropyl)benzene (81.5 mg, 409 µmol, 61.8 µL) as RX in Reaction Scheme 1 below were mixed with acetonitrile (2.00 mL), and then K₂CO₃(141 mg, 1.02 mmol) was added thereto. The mixture was stirred at 40 °C for 12 hours. It was confirmed by LCMS that the reactants were fully consumed, and Compound 3 was synthesized. The mixture was diluted with EA (50.0 mL) and extracted with brine (10 mL x 3). The resulting extract was dried with anhydrous Na₂SO₄ and filtered, and then concentrated in a vacuum to thereby obtain a residue. The residue was purified by reversed-phase HPLC to thereby obtain Compound 3 (10.0 mg, 22.7 µmol, yield: 6.65%) as red oil.

### Step 7: 3-(4-methylthiazol-5-yl)-6-(3-phenylpropoxy)-2-(pyridin-3-yl)-1H-inden-1-one (Compound 1)

Compound 3 (40.0 mg, 90.8 µmol) and pyridin-3-ylboronic acid (13.4 mg, 109 µmol)as R₁-B(OH)₂ in Reaction Scheme 1 were mixed with dioxane (2.00 mL) and H₂O (0.5 mL), and then K₃PO₄ (57.8 mg, 272 µmol) and Pd(dppf)Cl₂ (5.92 mg, 9.08 µmol) were added thereto in atmosphere including nitrogen. The mixture was stirred with microwaves at 100 °C for 2 hours. It was confirmed by LCMS that the reactants were fully consumed, and 36.1 % of Compound 1 was detected. The mixture was filtered and concentrated in a vacuum to thereby obtain a residue. The residue was purified by reversed-phase HPLC to thereby obtain Compound 1 (6.00 mg, 13.68 µmol, yield: 15.06 %) as a red solid.
¹H NMR(400 MHz, CD₃OD): *δ* 9.19 (s, 1H), 8.4-8.5 (m, 2H), 7.70-7.78 (m, 1H), 7.37-7.45 (m, 1H), 7.13-7.30 (m, 6H), 7.11 (d, *J* = 8.4 Hz, 1H), 6.94 (dd, *J =* 2.4, 8.0 Hz, 1H), 4.03 (t, *J* = 6.4 Hz, 2H), 2.81 (t, J=7.6 Hz, 2H,), 2.07-2.15 (m, 2H), 2.05 (s, 3H)
MS found value: 439.1 [M+H]⁺

### Example 2. 2-(2-(3-(4-methylthiazol-5-yl)-1-oxo-6-(3-phenylpropoxy)-1H-inden-2-yl)phenyl)acetonitrile (Compound 2)

Compound 3 (20.0 mg, 45.4 µmol) synthesized according to Example 1, 2-(2-(4,4,5,5-tetramethyl-1,3,2-dioxaborolan-2yl)phenyl)acetonitrile (13.2 mg, 54.5 µmol) as R₁-B(OH)₂ in Reaction Scheme 1 above, and Pd₂(dba)₃ (4.16 mg, 4.54 µmol) were mixed with THF (2.00 mL) and H₂O(0.50 mL), and then KF(7.92 mg, 136 µmol) and [(t-Bu)₃PH]BF₄ (1.32 mg, 4.54 µmol) were added thereto in atmosphere including nitrogen. The mixture was stirred at 20 °C for 4 hours. It was confirmed by LCMS that the reactants were fully consumed, and it was confirmed by MS that Compound 2 was synthesized. The mixture was diluted with H₂O (20.0 mL) and extracted with EA (20.0 mL x 3). The combined organic phase was dried with anhydrous Na₂SO₄ and filtered, and then concentrated in a vacuum to thereby obtain a residue. The residue was purified by reversed-phase HPLC to thereby obtain Compound 2 (3.80 mg, 7.97 µmol, yield: 17.5%) as red oil.
¹H NMR (400 MHz, CD₃OD): *δ* 9.09 (s, 1H), 7.5-7.5 (m, 1H), 7.40 (t, *J* = 7.6 Hz, 1H,), 7.21-7.32 (m, 5H), 7.15-7.20 (m, 3H), 7.05 (d, *J* = 7.2 Hz, 1H), 6.93-6.97 (m, 1H), 4.05 (t, J=6.4 Hz, 2H), 3.75 (s, 2H), 2.82 (t, *J* = 7.6 Hz, 2H), 2.08-2.18 (m, 2H), 2.07 (s, 3H)
MS found value: 477.0 [M+H]⁺

### Example 3. 2-bromo-3-(4-methylthiazol-5-yl)-6-(3-phenylpropoxy)-1H-inden-1-one (Compound 3)

Compound 3 was synthesized in Example 1.
¹H NMR (400 MHz, CD₃OD): *δ* 9.20 (s, 1H), 7.24-7.30 (m, 2H), 7.13-7.22 (m, 4H), 7.00 (d, *J* = 8.0 Hz, 1H), 6.88 (dd, *J =* 2.4, 8.4 Hz, 1H), 4.01 (t, *J* = 6.4 Hz, 2H), 2.80 (t, J=7.6 Hz, 2H), 2.49 (s, 3H), 2.05-2.14 (m, 2H)
MS found value: 439.9 [M+H]⁺

### Example 4. 3-(4-methylthiazol-5-yl)-2-phenyl-6-(3-phenylpropoxy)-1H-inden-1-one (Compound 10)

### Step 1: Synthesis of 2-bromo-3-(4-methylthiazol-5-yl)-6-(3-phenylpropoxy)-1H-inden-1-one (10-1)

Compound 1-7 (30.0 g, 1.0 eq) synthesized according to Example 1 and (3-bromopropyl)benzene (22.3 g, 1.2 eq) as RX in Reaction Scheme 1 above were mixed with DMF (300 mL), and then K₂CO₃ (23.2 g, 1.8 eq) and Nal(1.40 g, 0.1 eq) were added thereto at 20 °C to 25 °C in atmosphere including nitrogen. The mixture was stirred for 2 hours. It was confirmed by TLC (PE: EA= 1/1) that the reactants were fully consumed. Water (300 mL) was added to the mixture, and the mixture was adjusted to pH 3 with 1 M HCl, and then extracted with EtOAc (100 mL X 2). The organic layer was dried with Na₂SO₄, and then obtained as a concentrated organic phase residue at 40 °C to 45 °C under reduced pressure. The residue was purified by flash silica gel chromatography (PE/EA = 20/1, 1/1) to thereby obtain Compound 10-1 (25.0 g) as red oil.

### Step 2: Synthesis of 3-(4-methylthiazol-5-yl)-2-phenyl-6-(3-phenylpropoxy)-1H-inden-1-one (Compound 10)

Compound 10-1 (100 mg, 220 µmol, 1 eq) and phenylboronic acid (41.5 mg, 340 µmol, 1.5 eq) as R₁-B(OH)₂ in Reaction Scheme 1 above were mixed, and then the mixture was added to Pd(t-Bu₃P)₂ (11.0 mg, 21 µmol, 0.1 eq) and dioxane (0.8 mL) and water (0.2 mL) at 20 °C to 25 °C in atmosphere including nitrogen. The mixture was stirred at 80 °C to 85 °C for 3 hours. It was confirmed by TLC (PE: EA= 1/1) that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated organic phase residue at 40 °C to 45 °C under reduced pressure. The residue was purified by column chromatography (SiO₂, PE/EA = 10/1 to 1/1) to thereby obtain Compound 10 (82.0 mg, 179 µmol, yield: 79.1 %) as a red solid.
¹H NMR (400 MHz, DMSO-d6): *δ* 9.28 (s, 1H), 7.37 - 7.26 (m, 5H), 7.25 - 7.16 (m, 5H), 7.15 - 7.06 (m, 2H), 6.99 (dd, *J* = 2.4, 8.0 Hz, 1H), 4.04 (t, *J* = 6.4 Hz, 2H), 2.74 (t, *J* = 7.6 Hz, 2H), 2.10 - 1.98 (m, 2H), 1.91 (s, 3H)
MS found value: 438.1 [M+H]⁺

### Example 5. 3-(4-methylthiazol-5-yl)-6-(3-phenylpropoxy)-2-(pyrimidin-5-yl)-1H-inden-1-one (Compound 11)

Compound 10-1 (100 mg, 220 µmol, 1 eq) synthesized according to Example 4 and pyrimidin-5-ylboronic acid (42.2 mg, 340 µmol, 1.5 eq) as R₁-B(OH)₂ in Reaction Scheme 1 above were mixed, and then added to K₂CO₃ (94.0 mg, 681 µmol, 3.0 eq), Pd(t-Bu₃P)₂(11.0 mg, 21 µmol, 0.1 eq), and dioxane (0.8 mL) and water (0.2 mL) at 20 °C to 25 °C in atmosphere including nitrogen. The mixture was stirred at 80 °C to 85 °C for 3 hours. It was confirmed by TLC (PE: EA= 1/1) that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated organic phase residue at 40 °C to 45 °C under reduced pressure. The residue was purified by column chromatography (SiO₂, PE/EA = 10/1 to 1/1) to thereby obtain Compound 11 (23.0 mg, 179 µmol, yield: 22.1 %) as a red solid.
¹H NMR (400 MHz, DMSO-d6): *δ* 9.29 (s, 1H), 9.08 (s, 1H), 8.62 (s, 2H), 7.34 - 7.11 (m, 7H), 7.03 (m, 1H), 4.11 (brt, *J* = 6.4 Hz, 2H), 2.77 (brt, *J* = 7.6 Hz, 2H), 2.08 (s, 3H), 2.06 (brd, *J* = 7.2 Hz, 2H)
MS found value: 440 [M+H]⁺

### Example 6. 3-(4-methylthiazol-5-yl)-6-(3-phenylpropoxy)-2-(thiophen-2-yl)-1H-inden-1-one (Compound 12)

Compound 10-1 (100 mg, 220 µmol, 1 eq) synthesized according to Example 4 and thiophen-2-ylboronic acid (43.6 mg, 340 µmol, 1.5 eq) as R₁-B(OH)₂ in Reaction Scheme 1 above were mixed, and then added to K₂CO₃ (94.0 mg, 681 µmol, 3.0 eq), Pd(t-Bu₃P)₂ (11.0 mg, 21 µmol, 0.1 eq), and dioxane (0.8 mL) and water (0.2 mL) at 20 °C to 25 °C in atmosphere including nitrogen. The mixture was stirred at 80 °C to 85 °C for 3 hours. It was confirmed by TLC (PE: EA= 1/1) that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated organic phase residue at 40 °C to 45 °C under reduced pressure. The residue was purified by column chromatography (SiO₂, PE/EA = 10/1 to 1/1) to thereby obtain Compound 12 (23.0 mg, 179 µmol, yield: 22.08 %) as a red solid.
¹H NMR (400 MHz, DMSO-d6): *δ* 9.38 (s, 1H), 7.59 (d, *J* = 5.2 Hz, 1H), 7.40 (d, *J* = 3.6 Hz, 1H), 7.31 - 7.25 (m, 2H), 7.25 - 7.17 (m, 3H), 7.12 - 7.05 (m, 2H), 6.97 - 6.91 (m, 1H), 6.85 (d, *J* = 8.0 Hz, 1H), 4.02 (brt, *J* = 6.4 Hz, 2H), 2.74 (brt, *J* = 7.6 Hz, 2H), 2.18 (s, 3H), 2.06 - 1.97 (m, 2H)
MS found value: 444.1 [M+H]⁺

### Example 7. 3-(4-methylthiazol-5-yl)-2-(pyridin-3-yl)-6-(3-(pyridin-4-yl)propoxy)-1H-inden-1-one (Compound 13)

### Step 1: Synthesis of 2-bromo-3-(4-methylthiazol-5-yl)-6-(3-(pyridin-4-yl)propoxy)-1H-inden-1-one (13-1)

Compound 1-7 (300 mg, 745 µmol, 1.00 eq) synthesized according to Example 1 and 4-(3-bromopropyl)pyridine (557 mg, 2.24 mmol, 3.00 eq) as RX in Reaction Scheme 1 above were mixed with DMF (3.00 mL), and then K₂CO₃ (190 mg, 1.35 mmol, 1.80 eq) and Nal (13.0 mg, 85.8 µmol, 0.10 eq) were added thereto at 20 °C to 25 °C in atmosphere including nitrogen. The mixture was stirred at 40 °C to 45 °C for 18 hours. It was confirmed by TLC (PE: EA= 1/1) that the reactants were fully consumed. Water (10.0 mL) was added to the mixture, and the mixture was adjusted to pH 3 with 1 M HCl, and then extracted with EtOAc (10.0 mL X 3). The residue was purified by reverse-phase HPLC under HCI conditions to thereby obtain Compound 13-1 (47.0 mg, 97.9 µmol, yield: 13.1%) as a red solid.

### Step 2: Synthesis of 3-(4-methylthiazol-5-yl)-2-(pyridin-3-yl)-6-(3-(pyridin-4-yl)propoxy)-1H-inden-1-one (Compound 13)

Compound 13-1 (45.0 mg, 93.8 µmol, 1.00 eq) and pyridin-3-ylboronic acid (18.0 mg, 145 µmol, 1.45 eq) as R₁-B(OH)₂ in Reaction Scheme 1 above were mixed, and then added to K₂CO₃ (40.0 mg, 283 µmol, 3.00 eq), Pd(t-Bu₃P)₂ (6.00 mg, 11.1 µmol, 0.10 eq), and dioxane (0.80 mL) and water (0.20 mL) at 20 °C to 25 °C in atmosphere including nitrogen. The mixture was stirred at 80 °C to 85 °C for 12 hours. It was confirmed by LCMS that the reactants were fully consumed. Water (10 mL) was added to the mixture, and the mixture was adjusted to pH 7 with 1 M HCl, and then extracted with EtOAc (10 mL X 3). The organic layer was dried with Na₂SO₄, and then was obtained as a concentrated residue at 40 °C to 45 °C under reduced pressure. The residue was purified by prep-HPLC to thereby obtain Compound 13 (36.0 mg, 79.4 µmol, yield: 84.7%) as a red solid.
¹H NMR (400 MHz, DMSO-d6): *δ* 9.31 (s, 1H), 8.44 - 8.50 (m, 3H), 8.35 (d, *J* =1.2 Hz, 1H), 7.61 (m, 1H), 7.40 (m, 1H), 7.28 (d, *J* =5.6 Hz, 2H), 7.11 - 7.18 (m, 2H), 7.02 (m, 1H), 4.07 (t, *J* =6.4 Hz, 2H), 2.77 (br t, *J* =7.6 Hz, 2H), 2.02 - 2.11 (m, 2H), 1.96 (s, 3H)
MS found value: 440.0 [M+H]⁺

### Example 8. 3-(4-methylthiazol-5-yl)-6-phenethoxy-2-(pyridin-3-yl)-1H-inden-1-one (Compound 14)

### Step 1: Synthesis of 2-bromo-3-(4-methylthiazol-5-yl)-6-phenethoxy-1H-inden-1-one (14-1)

Compound 1-7 (3.00 g, 9.31 mmol,1.00 eq) synthesized according to Example 1 and (2-bromoethyl)benzene (2.58 g, 13.97 mmol, 1.5 eq) as RX in Reaction Scheme 1 above were mixed with DMF (30 mL), and then K₂CO₃ (2.32 g, 16.76 mmol, 1.5 eq) and Nal (139 mg, 931.17 µmol, 0.2 eq) were added thereto at 20 °C to 25 °C in atmosphere including nitrogen. The mixture was stirred at 20 °C to 25 °C for 12 hours. It was confirmed by TLC (PE: EA= 1/1) that the reactants were fully consumed. Water (20 mL) was added to the mixture, and the mixture was adjusted to pH 3 with 1 M HCl, and then extracted with EtOAc (5 mL). The residue was purified by column chromatography (SiO₂, PE/EA = 10/1 to 1/1) to thereby obtain Compound 14-1 (2.3 mg, 508 µmol, yield: 32.76%) as a red solid.

### Step 2: Synthesis of 3-(4-methylthiazol-5-yl)-6-phenethoxy-2-(pyridin-3-yl)-1H-inden-1-one (Compound 14)

Compound 14-1 (500 mg, 220 µmol, 1 eq) and pyridin-3-ylboronic acid (209 mg, 1.70 mmol, 1.5 eq) as R₁-B(OH)₂ in Reaction Scheme 1 above were mixed, and then added to K₂CO₃ (470 mg, 681 µmol, 3.0 eq), Pd(t-Bu₃P)₂ (57.9 mg, 113 µmol, 0.1 eq), and dioxane (8 mL) and water (2 mL) at 20 °C to 25 °C in atmosphere including nitrogen. The mixture was stirred at 80 °C to 85 °C for 3 hours. It was confirmed by TLC (PE: EA= 1/1) that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated residue at 40 °C to 45 °C under reduced pressure. The residue was purified by column chromatography (SiO₂, PE/EA = 10/1 to 1/1) to thereby obtain Compound 14 (300 mg, 692 µmol, yield: 61.07 %) as a red solid.
¹H NMR (400 MHz, DMSO-d6): *δ* 9.36 (s, 1H), 8.53 (dd, *J* = 1.2, 4.8 Hz, 1H), 8.40 (d, *J* = 1.6 Hz, 1H), 7.65 (m, 1H), 7.44 (dd, *J* = 4.8, 7.8 Hz, 1H), 7.41 - 7.34 (m, 4H), 7.31 - 7.26 (m, 1H), 7.21 (d, *J* = 2.4 Hz, 1H), 7.17 (d, *J* = 8.0 Hz, 1H), 7.10 - 7.05 (m, 1H), 4.34 (t, *J* = 6.8 Hz, 2H), 3.10 (t, *J* = 6.8 Hz, 2H), 2.00 (s, 3H)
MS found value: 425 [M+H]⁺

### Example 9. 3-(4-methylthiazol-5-yl)-6-(4-phenylbutoxy)-2-(pyridin-3-yl)-1H-inden-1-one (Compound 15)

### Step 1: Synthesis of 2-bromo-3-(4-methylthiazol-5-yl)-6-(4-phenylbutoxy)-1H-inden-1-one (15-1)

Compound 1-7 (500 mg, 1.55 mmol,1 eq) synthesized according to Example 1 and (4-bromobutyl)benzene (396 mg, 1.86 mmol, 1.2 eq) as RX in Reaction Scheme 1 above were mixed with DMF (5 mL), and then K₂CO₃ (321 mg, 2.33 mmol, 1.5 eq) and Nal (46.0 mg, 310 µmol, 0.2 eq) were added thereto at 20 °C to 25 °C in atmosphere including nitrogen. The mixture was stirred at 20 °C to 25 °C for 12 hours. It was confirmed by TLC (PE: EA= 1/1) that the reactants were fully consumed. Water (20 mL) was added to the mixture, and the mixture was adjusted to pH 3 with 1 M HCl, and then extracted with EtOAc (5 mL). The residue was purified by column chromatography (SiO₂, PE/EA = 10/1 to 1/1) to thereby obtain Compound 15-1 (231 mg, 508 µmol, yield: 32.7 %) as a red solid.

### Step 2: Synthesis of 3-(4-methylthiazol-5-yl)-6-(4-phenylbutoxy)-2-(pyridin-3-yl)-1H-inden-1-one (Compound 15)

Compound 15-1 (100 mg, 220 µmol, 1 eq) and pyridin-3-ylboronic acid (40.0 mg, 325 µmol, 1.48 eq) as R₁-B(OH)₂ in Reaction Scheme 1 above were mixed, and then added to K₂CO₃ (91.0 mg, 658 µmol, 2.99 eq), Pd(t-Bu₃P)₂(11.00 mg, 21 µmol, 0.10 eq), and dioxane (0.8 mL) and water (0.2 mL) at 20 °C to 25 °C in atmosphere including nitrogen. The mixture was stirred at 80 °C to 85 °C for 3 hours. It was confirmed by TLC (PE: EA= 1/1) that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated residue at 40 °C to 45 °C under reduced pressure. The residue was purified by column chromatography (SiO₂, PE/EA = 10/1 to 1/1) to thereby obtain Compound 15 (14.9 mg, 26 µmol, yield: 12.1 %) as a red solid.
¹H NMR (400 MHz, DMSO-d6): *δ* 9.30 (s, 1H), 8.52 - 8.39 (m, 2H), 7.61 - 7.59 (d, *J* = 8.0 Hz, 1H), 7.30 (s, 1H), 7.28 - 7.11 (m, 7H), 7.02 (s, 1H), 4.09 - 4.07 (t, *J* = 10.8, 2H), 2.66 - 2.62 (t, *J* = 14 , 2H), 1.95 (s, 3H), 1.74 -1.73 (d, *J* = 3.6, 4H)
MS found value: 453.3 [M+H]⁺

### Example 10. 6-((benzyloxy)methoxy)-3-(4-methylthiazol-5-yl)-2-(pyridin-3-yl)-1H-inden-1-one (Compound 16)

### Step 1: Synthesis of 6-((benzyloxy)methoxy)-2-bromo-3-(4-methylthiazol-5-yl)-1H-inden-1-one (16-1)

Compound 1-7 (1.00 g, 3.10 mmol,1.00 eq) synthesized according to Example 1 and ((chloromethoxy)methyl)benzene (614 mg, 3.72 mmol, 1.20 eq) as RX in Reaction Scheme 1 above were mixed with DMF (10.0 mL), and then K₂CO₃ (657 mg, 4.66 mmol, 1.50 eq) and Nal (94 mg, 620 µmol, 0.20 eq) were added thereto at 20 °C to 25 °C in atmosphere including nitrogen. The mixture was stirred at 40 °C to 45 °C for 18 hours. It was confirmed by LCMS that the reactants were fully consumed. Water (20 mL) was added to the mixture, and the mixture was adjusted to pH 3 with 1 M HCl, and then extracted with EtOAc (30.0 mL X 3). The residue was purified by column chromatography (SiO₂, PE/EA = 20/1 to 5/1) to thereby obtain Compound 16-1 (270 mg, 568 µmol, yield: 18.3 %) as a red solid.

### Step 2: Synthesis of 6-((benzyloxy)methoxy)-3-(4-methylthiazol-5-yl)-2-(pyridin-3-yl)-1H-inden-1-one (Compound 16)

Compound 16-1 (250 mg, 525 µmol, 1.00 eq) and pyridin-3-ylboronic acid (98.0 mg, 789 µmol, 1.50 eq) as R₁-B(OH)₂ in Reaction Scheme 1 above were mixed, and then added to K₂CO₃ (224 mg, 4.59 mmol, 3.02 eq), Pd(t-Bu₃P)₂ (29.0 mg, 53.9 µmol, 0.10 eq), and dioxane (2.00 mL) and water (0.50 mL) at 20 °C to 25 °C in atmosphere including nitrogen. The mixture was stirred at 80 °C to 85 °C for 12 hours. It was confirmed by LCMS that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated residue at 40 °C to 45 °C under reduced pressure. The residue was purified by column chromatography (SiO₂, PE/EA = 20/1 to 1/1) to thereby obtain Compound 16 (67.4 mg, 26 µmol, yield: 12.1 %) as a red solid.
¹H NMR (400 MHz, DMSO-d6): *δ* 8.86 (s, 1H), 8.37 - 8.50 (m, 2H), 7.55 - 7.64 (m, 1H), 7.30 (br d, *J* = 1.2 Hz, 3H), 7.18 - 7.27 (m, 4H), 6.96 - 7.05 (m, 2H), 5.26 (s, 2H), 4.66 (s, 2H), 2.03 (s, 3H)
MS found value: 441.0 [M+H]⁺

### Example 11. 3-(4-methylthiazol-5-yl)-6-(2-phenoxyethoxy)-2-(pyridin-3-yl)-1H-inden-1-one (Compound 17)

### Step 1: Synthesis of 2-bromo-3-(4-methylthiazol-5-yl)-6-(2-phenoxyethoxy)-1H-inden-1-one (17-1)

Compound 1-7 (500 mg, 1.55 mmol,1 eq) synthesized according to Example 1 and (2-bromoethoxy)benzene (374 mg, 1.86 mmol, 1.2 eq) as RX in Reaction Scheme 1 above were mixed with DMF (5 mL), and then K₂CO₃ (321 mg, 2.33 mmol, 1.5 eq) and Nal (46.0 mg, 310 µmol, 0.2 eq) were added thereto at 20 °C to 25 °C in atmosphere including nitrogen. The mixture was stirred at 20 °C to 25 °C for 12 hours. It was confirmed by LCMS that the reactants were fully consumed. Water (20 mL) was added to the mixture, and the mixture was adjusted to pH 3 with 1 M HCl, and then extracted with EtOAc (5 mL). The residue was purified by column chromatography (SiO₂, PE/EA = 10/1 to 1/1) to thereby obtain Compound 16-1 (179 mg, 404 µmol, yield: 26.1%) as a red solid.

### Step 2: Synthesis of 3-(4-methylthiazol-5-yl)-6-(2-phenoxyethoxy)-2-(pyridin-3-yl)-1H-inden-1-one (Compound 17)

Compound 17-1 (100 mg, 220 µmol, 1 eq) and pyridin-3-ylboronic acid (40.0 mg, 325 µmol, 1.48 eq) as R₁-B(OH)₂ in Reaction Scheme 1 above were mixed, and then added to K₂CO₃ (91.0 mg, 658 µmol, 2.99 eq), Pd(t-Bu₃P)₂(11.00 mg, 21 µmol, 9.78e-2 eq), and dioxane (0.8 mL) and water (0.2 mL) at 20 °C to 25 °C in atmosphere including nitrogen. The mixture was stirred at 80 °C to 85 °C for 3 hours. It was confirmed by TLC (PE: EA= 1/1) that the reactants were fully consumed. Water (10 mL) was added to the mixture, and the mixture was adjusted to pH 3 with 1 M HCl, and then extracted with EtOAc (10 mL X 3). The organic layer was dried with Na₂SO₄, and then was obtained as a concentrated residue at 40 °C to 45 °C under reduced pressure. The residue was purified by column chromatography (SiO₂, PE/EA = 10/1 to 1/1) to thereby obtain Compound 17 (22.86 mg, 37 µmol, yield: 16.44 %) as a red solid.
¹H NMR (400 MHz, DMSO-d6): *δ* 9.31 (s, 1H), 8.49 - 8.49 (d, *J* = 1.2 Hz, 1H), 8.48 - 8.47 (d, *J* = 1.2 Hz, 1H), 8.36-8.35 (d, *J* = 1.6 Hz, 1H), 7.62 - 7.60 (d, *J* = 8 Hz, 1H), 7.39 - 6.95 (m, 9H), 4.44 - 4.42 (m, 2H), 4.34 - 4.32 (m, 2H), 1.96 (s , 1H)
MS found value: 441.3 [M+H]⁺

### Example 12. 3-(furan-3-yl)-6-(3-phenylpropoxy)-2-(pyridin-3-yl)-1H-inden-1-one (Compound 9)

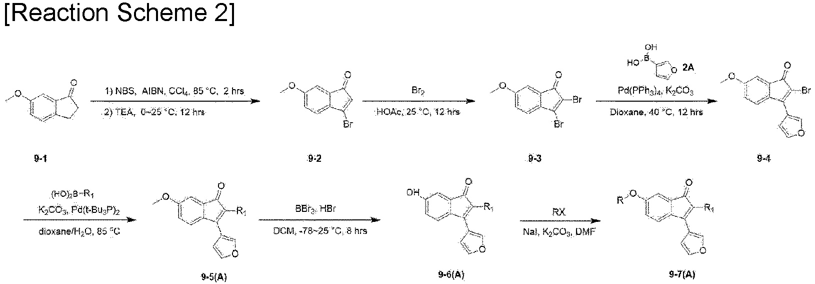

### Step 1: Synthesis of 3-bromo-6-methoxy-1H-inden-1-one (9-2)

Compound 9-1 (50.0 g, 308 mmol, 1.00 eq), NBS (109 g, 616 mmol, 2.00 eq) and AIBN (1.02 g, 6.20 mmol, 2.01 eq) according to Reaction Scheme 2 above were mixed with CCl₄ (1.00 L), and then AIBN (1.02 g, 6.20 mmol, 2.01 eq) was added thereto at 25 °C in atmosphere including nitrogen. The mixture was stirred at 85 °C for 2 hours. It was confirmed by TLC (PE: EA= 1:1) that the reactants were fully consumed. TEtOAc (93.5 g, 924 mmol, 128 mL, 3.00 eq) was added to the mixture, followed by stirring at 25 °C for 12 hours. It was confirmed by LCMS that a desired compound was synthesized from the reactants. The mixture was cooled with water (1000 mL) and extracted with DCM (800 mL X 2). The organic layer was washed with NaCl (500 mL X 3), and then dried with Na₂SO₄. The dried organic layer was filtered, and then concentrated under reduced pressure, and the residue was obtained as Compound 9-2 (70.0 g, 201 mmol, yield: 65.4 %, purity: 68.9 %) in the form of a red solid.

### Step 2: Synthesis of 2,3-dibromo-6-methoxy-1H-inden-1-one (9-3)

Compound 9-2 (65.0 g, 271.8 mmol, 1.00 eq) was dissolved in HOAc (850 mL), and then the mixture was stirred at 22-25 °C. Br₂ (130 g, 815 mmol, 42.0 mL, 3.00 eq) was added dropwise thereto, followed by stirring at 22-25 °C for 12 hours. It was confirmed by TLC (PE: EA= 3:1) that the reactants were fully consumed. The mixture was extracted with a 10% aqueous NaS₂O₃ solution (1000 mL), and the water phase was extracted with MTBE (500 mL X 2). The organic phase was washed with NaHCO₃ (1000 mL X 2), and then washed with brine (500 mL X 2) and dried with anhydrous Na₂SO₄. The dried organic layer was concentrated in a vacuum to thereby obtain a residue. The residue was purified by column chromatography (SiO₂, PE/EA = 3:1, P₁=0.49) to thereby obtain Compound 9-3 (15.0 g, 39.8 nmol, yield: 14.6 %, purity: 84.5 %) as a red solid.

### Step 3: Synthesis of 2-bromo-3-(furan-3-yl)-6-methoxy-1H-inden-1-one (9-4)

Compound 9-3 (11.9 g, 37.4 mmol, 1.00 eq) and 2A (5.03 g, 44.9 mmol, 1.20 eq) in Reaction Scheme 2 above were mixed with dioxane (120 mL) and H₂O (30.0 mL), and then K₂CO₃ (15.5 g, 112 mmol, 3.00 eq) and Pd(PPh₃)₄ (4.32 g, 3.74 mmol, 0.10 eq) were added thereto at 25 °C in atmosphere including nitrogen. The mixture was stirred at 40 °C for 12 hours in atmosphere including nitrogen. While adding dropwise ACN (1 mL), it was confirmed by LCMS that the reactants were fully consumed, and the reaction mixture was cooled to 22-25 °C. Water (200 mL) was added to the mixture, and the mixture was extracted with EtOAc (100 mL X 2). The organic layer was washed with NaCl (100 mL X 2), and then dried with Na₂SO₄. The mixture was filtered and concentrated in a vacuum to thereby obtain a residue. The residue was filtered, and then concentrated under reduced pressure, to thereby obtain Compound 9-4 (5.20 g, 4.67 mmol, yield: 12.4 %, purity: 27.4 %) as a red solid.

### Step 4: Synthesis of 3-(furan-3-yl)-6-methoxy-2-(pyridin-3-yl)-1H-inden-1-one (9-5)

Compound 9.4 (5.00 g, 16.3 mmol, 1.00 eq) and pyridin-3-ylboronic acid (2.42 g, 19.7 mmol, 1.20 eq) as R₁-B(OH)₂ in Reaction Scheme 2 above were mixed with dioxane (120 mL) and H₂O (30.0 mL), and then K₂CO₃ (6.78 g, 49.0 mmol, 2.99 eq) and Pd(t-Bu₃P)₂ (875 mg, 1.71 mmol, 1.04 eq) were added thereto in atmosphere including nitrogen. The mixture was stirred at 85 °C for 12 hours. The reaction mixture was cooled to 22-25 °C, and then, while adding dropwise ACN (1 mL), it was confirmed by LCMS that the reactants were fully consumed. Water (30.0 mL) was added to the mixture, and the mixture was extracted with EtOAc (30.0 mL X 2). The organic layer was washed with NaCl (20 mL X 2), and then dried with Na₂SO₄. The mixture was filtered and concentrated in a vacuum to thereby obtain a residue. The residue was purified by column chromatography (SiO₂, PE: EA = 3:1, P₁=0.20) to thereby obtain Compound 9-5 (2.50 g, 5.17 mmol, yield: 31.5 %, purity: 62.7 %) as a red solid.

### Step 5: Synthesis of 3-(furan-3-yl)-6-hydroxy-2-(pyridin-3-yl)-1H-inden-1-one (9-6)

Compound 9-5 (3.00 g, 9.89 mmol, 1.00 eq) was mixed with DCM (90.0 mL) at 22-25 °C in atmosphere including nitrogen, followed by mixing with BBr₃ (7.44 g, 29.7 mmol, 2.86 mL, 3.00 eq) at -78 °C in atmosphere including nitrogen. The mixture was stirred at -78 °C for 2 hours, and then stirred at 22-25 °C for 6 hours in atmosphere including nitrogen. While adding dropwise ACN (1 mL), it was confirmed by LCMS that the reactants were fully consumed. Water (100 mL) was added to the mixture at 0 °C, and then the mixture was stirred for 10 minutes. The mixture was adjusted to pH 7 with 5% NaHCO₃/H₂O, and then extracted with EtOAc (50.0 mL X 3). The organic layer was washed with brine (100 mL), and then dried with Na₂SO₄, and the mixture was filtered and concentrated. THF (20.0 mL) and PE (80.0 mL) were added thereto, followed by stirring at 25 °C for 30 minutes. The resultant was filtered, washed with PE (50.0 mL), and then dried in a vacuum to thereby obtain a pale red solid compound. The solution was removed therefrom to thereby obtain Compound 9-6 (3.30 g, 7.41 mmol, yield: 74.9%, purity: 65.0%) as a red solid.

### Step 6: Synthesis of 3-(furan-3-yl)-6-(3-phenylpropoxy)-2-(pyridin-3-yl)-1H-inden-1-one (Compound 9)

Compound 9-6 (50.0 mg, 172.84 µmol, 1 eq) and (3-bromopropyl)benzene (1 eq) as RX in Reaction Scheme 2 above were mixed with DMF (1 mL), and then K₂CO₃ (35.8 mg, 259.26 µmol, 1.5 eq) and Nal (5.18 mg, 34.57 µmol, 0.2 eq) were added thereto at 25 °C. The mixture was stirred at 45 °C for 12 hours. It was confirmed by LCMS that the reactants were fully consumed. The mixture was diluted with water (5 mL) and extracted with EtOAc (5 mL X 3). The organic layer was washed with brine (5 mL X 2), and then dried with Na₂SO₄, and the mixture was filtered and concentrated under reduced pressure. The residue was purified by reversed-phase HPLC to thereby obtain Compound 9 (12.18 mg, 28.93 µmol, yield: 16.74 %, purity: 96.78 %) as an orange solid.
¹H NMR (400 MHz, DMSO-d6): *δ* 8.56 (dd, *J* = 1.6, 4.8 Hz, 1H), 8.50 - 8.41 (m, 2H), 7.83 (t, *J* = 1.6 Hz, 1H), 7.72 (td, *J* = 2.0, 8.0 Hz, 1H), 7.55 - 7.44 (m, 2H), 7.36 - 7.13 (m, 6H), 7.04 (dd, *J* = 2.4, 8.0 Hz, 1H), 6.23 (d, *J* = 1.2 Hz, 1H), 4.09 (t, *J* = 6.4 Hz, 2H), 2.78 (t, *J* = 8.0 Hz, 2H), 2.14 - 2.02 (m, 2H)
MS found value: 408.2 [M+H]⁺

### Example 13. 3-(furan-3-yl)-6-phenethoxy-2-(pyridin-3-yl)-1H-inden-1-one (Compound 38)

Compound 9-6 (50.0 mg, 172 µmol, 1 eq) synthesized according to Example 12 and (2-bromoethyl)benzene (31.9 mg, 172 µmol, 23.38 µL, 1 eq) as RX in Reaction Scheme 2 above were mixed with DMF (1 mL), and then K₂CO₃ (35.8 mg, 259 µmol, 1.5 eq) and Nal (5.18 mg, 34.5 µmol, 0.2 eq) were added thereto at 25 °C. The mixture was stirred at 50 °C for 14 hours. While adding dropwise DCM (1.00 mL), it was confirmed by TLC (PE: EA = 1/1) that the reactants were fully consumed. The mixture was diluted with water (5 mL) and extracted with EtOAc (5 mL X 3). The organic layer was washed with brine (5 mL X 2), and then dried with Na₂SO₄, and the mixture was filtered and concentrated under reduced pressure. The residue was purified by reversed-phase HPLC to thereby obtain Compound 38 (12 mg, 30.50 µmol, yield: 17.65 %) as a red solid.
¹H NMR (400 MHz, DMSO-d6): *δ* 8.54 (d, *J* = 4.8 Hz, 1H), 8.45 (s, 2H), 7.81 (s, 1H), 7.70 (br d, *J* = 8.0 Hz, 1H), 7.52 - 7.40 (m, 2H), 7.37 - 7.30 (m, 4H), 7.28 - 7.20 (m, 1H), 7.13 (d, *J* = 2.0 Hz, 1H), 7.04 (dd, *J* = 2.0, 8.0 Hz, 1H), 6.20 (s, 1H), 4.31 (t, *J* = 6.8 Hz, 2H), 3.06 (t, *J* = 6.8 Hz, 2H)
MS found value: 394.1 [M+H]⁺

### Example 14. 3-(furan-3-yl)-6-(4-phenylbutoxy)-2-(pyridin-3-yl)-1H-inden-1-one (Compound 39)

Compound 9-6 (54.3 mg, 187.69 µmol, 1 eq) synthesized according to Example 12 and (4-bromobutyl)benzene (40.0 mg, 187.69 µmol, 1 eq) as RX in Reaction Scheme 2 above were mixed with DMF (1 mL), and then K₂CO₃ (38.9 mg, 281 µmol, 1.5 eq) and Nal (5.63 mg, 37.5 µmol, 0.2 eq) were added thereto at 25 °C. The mixture was stirred at 50 °C for 14 hours. It was confirmed by LCMS that a desired compound was synthesized. The mixture was diluted with water (5 mL) and extracted with EtOAc (5 mL X 3). The organic layer was washed with brine (5 mL X 2), and then dried with Na₂SO₄, and the mixture was filtered and concentrated under reduced pressure. The residue was purified by reversed-phase HPLC to thereby obtain Compound 39 (15 mg, 34.95 µmol, yield: 62 %, purity: 98.2 %) as a red solid.
¹H NMR (400 MHz, DMSO-d6): *δ* 8.55 (dd, *J* = 1.6, 4.8 Hz, 1H), 8.46 (s, 2H), 7.83 - 7.80 (m, 1H), 7.71 (td, *J* = 2.0, 8.0 Hz, 1H), 7.49 (d, *J* = 8.0 Hz, 1H), 7.45 (m, 1H), 7.32 - 7.26 (m, 2H), 7.26 - 7.16 (m, 3H), 7.13 (d, *J* = 2.4 Hz, 1H), 7.02 (dd, *J* = 2.4, 8.0 Hz, 1H), 6.20 (d, *J* = 1.6 Hz, 1H), 4.13 - 4.06 (m, 2H), 2.73 - 2.61 (m, 2H), 1.75 (br d, *J* = 3.6 Hz, 4H)
MS found value: 422.2 [M+H]⁺

### Example 15. 6-((benzyloxy)methoxy)-3-(furan-3-yl)-2-(pyridin-3-yl)-1H-inden-1-one (Compound 40)

Compound 9-6 (80.0 mg, 276.54 µmol, 1 eq) synthesized according to Example 12 and NaH (13.3 mg, 331 µmol, purity: 60 %, 1.2 eq) were mixed with DMF (1 mL), and then ((chloromethoxy)methyl)benzene (86.6 mg, 553 µmol, 76.4 µL, 2 eq) as RX in Reaction Scheme 2 above was dissolved in DMF (0.1 mL) and the resulting solution was mixed therewith while being added dropwise, and then the mixture was stirred at 25 °C for 1 hour. It was confirmed by LCMS that a desired compound was synthesized. The mixture was cooled with NH₄Cl (5 mL), and then diluted with water (5 mL) and extracted with EtOAc (5 mL X 3). The organic layer was washed with brine (5 mL X 2), and then dried with Na₂SO₄, and the mixture was filtered and concentrated under reduced pressure. The residue was purified by prep-HPLC (SiO₂, PE: EA = 1:2) to thereby obtain Compound 40 (9.72 mg, 20.58 µmol, yield: 7.44 %, purity: 86.7 %) as a red gum.
¹H NMR (400 MHz, CHLOROFORM-d): *δ* 8.55 - 8.43 (m, 2H), 7.79 (s, 1H), 7.65 (br d, *J* = 8.0 Hz, 1H), 7.41 (s, 1H), 7.28 (br s, 3H), 7.25 - 7.13 (m, 5H), 7.02 (dd, *J* = 2.4, 8.0 Hz, 1H), 6.19 (s, 1H), 5.26 (s, 2H), 4.72 - 4.61 (s, 2H)
MS found value: 410.1 [M+H]⁺

### Example 16. 3-(furan-3-yl)-6-(2-phenoxyethoxy)-2-(pyridin-3-yl)-1H-inden-1-one (Compound 41)

Compound 9-6 (54.7 mg, 189 µmol, 1 eq) synthesized according to Example 12 and (2-bromoethoxy)benzene (38.0 mg, 189 µmol, 1 eq) as RX in Reaction Scheme 2 above were mixed with DMF (1 mL), and then K₂CO₃ (39.2 mg, 283 µmol, 1.5 eq) and Nal (5.67 mg, 37.8 µmol, 0.2 eq) were added thereto at 25 °C. The mixture was stirred at 45 °C for 12 hours. It was confirmed by LCMS that a desired compound was synthesized. The mixture was diluted with water (5 mL) and extracted with EtOAc (5 mL X 3). The organic layer was washed with brine (5 mL X 2), and then dried with Na₂SO₄, and the mixture was filtered and concentrated under reduced pressure. The residue was purified by reversed-phase HPLC to thereby obtain Compound 41 (20 mg, 48.26 µmol, yield: 25.54 %, purity: 98.8 %) as a red solid.
¹H NMR (400 MHz, METHANOL-d): *δ* 8.53 - 8.47 (m, 2H), 8.18 (s, 1H), 7.85 (td, *J* = 2.0, 8.0 Hz, 1H), 7.66 (t, *J* = 1.6 Hz, 1H), 7.51 - 7.41 (m, 2H), 7.31 (t, *J* = 7.2 Hz, 2H), 7.24 (d, *J* = 2.4 Hz, 1H), 7.08 (dd, *J* = 2.4, 8.0 Hz, 1H), 7.03 - 6.94 (m, 3H), 6.30 (dd, *J* = 0.8, 2.0 Hz, 1H), 4.49 - 4.31 (m, 4H)
MS found value: 410.1 [M+H]⁺

### Example 17. 3-(furan-3-yl)-6-(2-(4-methoxyphenoxy)ethoxy)-2-(pyridin-3-yl)-1H-inden-1-one (Compound 42)

Compound 9-6 (50.0 mg, 172 µmol, 1 eq) synthesized according to Example 12 and 1-(2-bromoethoxy)-4-methoxybenzene (40.7 mg, 176 µmol, 1.02 eq) as RX in Reaction Scheme 2 above were mixed with DMF (3.00 mL), and then K₂CO₃ (36.0 mg, 260 µmol, 1.51 eq) and Nal (5.00 mg, 33.3 µmol, 1.93 eq) were added thereto at 25 °C. The mixture was stirred at 45-50 °C for 12 hours. It was confirmed by TLC (PE: EA= 3:1) that the reactants were fully consumed. The mixture was cooled with water (10.0 mL). The mixture was extracted with EtOAc (10.0 mL X 2). The organic layer was washed with NaCl (10.0 mL X 2), and then dried with Na₂SO₄, and the mixture was filtered and concentrated under reduced pressure. The residue was purified by reversed-phase HPLC to thereby obtain Compound 42 (11.0 mg, 24.6 µmol, yield: 14.2 %, purity: 98.4 %).
¹H NMR (400 MHz, DMSO-d6): *δ* ppm 3.70 (s, 3 H), 4.24-4.32 (m, 2 H), 4.38-4.44 (m, 2 H), 6.20 (d, J=1.2 Hz, 1 H), 6.84-6.89 (m, 2 H), 6.90-6.96 (m, 2 H), 7.06-7.11 (m, 1 H), 7.17-7.22 (m, 1 H), 7.45 (dd, J=8.0, 4.8 Hz, 1 H), 7.51 (d, J=8.0 Hz, 1 H), 7.67-7.74 (m, 1 H), 7.77-7.86 (m, 1 H), 8.44-8.50 (m, 2 H), 8.54 (dd, J=4.8, 1.2 Hz, 1 H)
MS found value: 440.1 (M+1)

### Example 18. 6-(2-(3,4-dimethoxyphenoxy)ethoxy)-3-(furan-3-yl)-2-(pyridin-3-yl)-1H-inden-1-one (Compound 43)

Compound 9-6 (20.0 mg, 69.1 µmol, 1.00 eq) synthesized according to Example 12 and 4-(2-bromoethoxy)-1,2-dimethoxybenzene (18.4 mg, 70.5 µmol, 1.02 eq) as RX in Reaction Scheme 2 above were mixed with DMF (3.00 mL), and then K₂CO₃ (14.4 mg, 104 µmol, 1.51 eq) and Nal (20.0 mg, 133 µmol, 1.93 eq) were added thereto at 25 °C. The mixture was stirred at 40 °C for 12 hours. While adding dropwise EtOAc (1 mL), it was confirmed by TLC (PE: EA = 1:0) that the reactants were fully consumed. The mixture was cooled to 22-25 °C. The mixture was diluted with water (30.0 mL) and extracted with EtOAc (10.0 mL X 3). The organic layer was washed with brine (15.0 mL), and then dried with Na₂SO₄, and the mixture was filtered and concentrated under reduced pressure. Compound 43 (12.1 mg, 25.33 µmol, yield: 36.64 %, purity: 98.3 %) was obtained as a red solid.
¹H NMR (400 MHz, DMSO-d6): *δ* ppm 8.54 (s, 1 H), 8.46 (s, 2 H) 7.81 (s, 1 H), 7.71 (s, 1 H), 7.48 (d, J=21.6 Hz, 2 H), 7.19 (s, 1 H), 7.11 (s, 1 H), 6.86 (d, J=4.8 Hz, 1 H) 6.62 (s, 1 H), 6.50 (s, 1 H), 6.20 (s, 1 H), 4.21 - 4.48 (d, J=52 Hz, 4 H) 3.71 (d, *J*=19.2 Hz, 6 H)
MS found value: 470.2 [M+H]⁺

### Example 19. 6-(2-(benzo[d][1,3]dioxol-5-yloxy)ethoxy)-3-(furan-3-yl)-2-(pyridin-3-yl)-1H-inden-1-one (Compound 44)

Compound 9-6 (50.0 mg, 172 µmol, 1.00 eq) synthesized according to Example 12 and 5-(2-bromoethoxy)benzo[*d*][1,3]dioxole (40.7 mg, 176 µmol, 1.02 eq) as RX in Reaction Scheme 2 above were mixed with DMF (3.0 mL), and then K₂CO₃ (36.0 mg, 260 µmol, 1.51 eq) and Nal (5.00 mg, 33.3 µmol, 1.93 eq) were added thereto at 25 °C. The mixture was stirred at 45-50 °C for 12 hours. It was confirmed by LCMS that a desired compound was synthesized. The mixture was cooled with water (10.0 mL). The mixture was extracted with EtOAc (10.0 mL X 2). The organic layer was washed with NaCl (10.0 mL X 2), and then dried with Na₂SO₄, and the mixture was filtered and concentrated under reduced pressure. The residue was purified by reversed-phase HPLC to thereby obtain Compound 44 (15.9 mg, 31.4 µmol, yield: 18.1 %, purity: 89.6 %) as a red solid.
¹H NMR (400 MHz, DMSO-d6): *δ* ppm 8.54 (dd, J=4.8, 1.6 Hz, 1 H), 8.45-8.49 (m, 2 H), 7.82 (t, *J*=2.0 Hz,1 H), 7.71 (dt, J=8.0, 2.0 Hz, 1 H), 7.51 (d, *J*=8.4 Hz, 1 H), 7.42-7.47 (m, 1 H), 7.19 (d, *J*=2.4 Hz, 1 H), 7.09 (dd, *J*=8.4, 2.4 Hz, 1 H), 6.82 (d, *J*=8.0 Hz, 1 H), 6.69 (d, *J*=2.4 Hz, 1 H), 6.43 (dd, *J*=8.4, 2.8 Hz, 1 H), 6.20 (dd, *J*=2.0, 0.8 Hz, 1 H), 5.96 (s, 2 H), 4.38-4.40 (m, 2 H), 4.21-4.30 (m, 2 H)
MS found value: 454 (M+1)

### Example 20. 3-(furan-3-yl)-2-(pyridin-3-yl)-6-(2-(pyridin-4-yloxy)ethoxy)-1H-inden-1-one (Compound 45)

Compound 9-6 (50.0 mg, 172 µmol, 1.00 eq) synthesized according to Example 12 and 4-(2-bromoethoxy)pyridine (35.6 mg, 176 µmol, 1.02 eq) as RX in Reaction Scheme 2 above were mixed with DMF (3.00 mL), and then K₂CO₃ (60.0 mg, 434 µmol, 2.51 eq) and Nal (5.18 mg, 34.5 µmol, 0.20 eq) were added thereto at 25 °C. The mixture was stirred at 40 °C for 12 hours. While adding dropwise EtOAc (1 mL), it was confirmed by TLC (PE: EA = 1:0) that the reactants were fully consumed. The mixture was cooled to 22-25 °C. The mixture was diluted with water (30.0 mL) and extracted with EtOAc (10.0 mL X 3). The organic layer was washed with brine (15.0 mL), and then dried with Na₂SO₄, and the mixture was filtered and concentrated under reduced pressure. Compound 45 (8 mg, 18.7 µmol, yield: 10.8 %, purity: 96.2 %) were obtained as a red solid.
¹H NMR (400 MHz, CHLOROFORM-d): *δ* ppm 8.58 (s, 2 H), 8.50 (s, 1 H), 7.88 (s, 1 H), 7.74 (d, *J*=8.4 Hz, 1 H), 7.50 (s, 1 H), 7.31 - 7.34 (m, 1 H), 7.30 (s, 1 H), 6.89-6.98 (m, 3 H), 6.27 (s, 1 H), 4.43 (s, 4 H)
MS found value: 411.2 [M+H]⁺

### Example 21. 6-(2-(3,4-dichlorophenoxy)ethoxy)-3-(furan-3-yl)-2-(pyridin-3-yl)-1H-inden-1-one (Compound 46)

Compound 9-6 (50.0 mg, 173 µmol, 1.00 eq) synthesized according to Example 12 and 4-(2-bromoethoxy)-1,2-dichlorobenzene (46.0 mg, 194 µmol, 1.12 eq) as RX in Reaction Scheme 2 above were mixed with DMF (3.0 mL), and then K₂CO₃ (36.0 mg, 260 µmol, 1.51 eq) and Nal (5.00 mg, 33.4 µmol, 0.20 eq) were added thereto at 25 °C. The mixture was stirred at 40 °C for 12 hours. While adding dropwise MeOH (1.00 mL), it was confirmed by LCMS that a desired compound was synthesized. The mixture was cooled to 25 °C. The mixture was diluted with water (30.0 mL) and extracted with EtOAc (10.0 mL X 3). The organic layer was washed with brine (15.0 mL), and then dried with Na₂SO₄, and the mixture was filtered and concentrated in a vacuum. The residue was purified by reversed-phase HPLC to thereby obtain Compound 46 (34.0 mg, purity: 90.1 %) as a red solid.
¹H NMR (400 MHz, DMSO-d6): *δ* ppm 8.54 (dd, J=4.8, 1.6 Hz, 1 H), 8.48 (s, 1 H), 8.46 (d, *J*=2.0 Hz, 1 H), 7.82 (t, *J*=2.0 Hz, 1 H), 7.71 (dt, *J*=8.0, 2.0 Hz, 1 H), 7.53 (dd, *J*=11.6, 9.2 Hz, 2 H), 7.45 (dd, *J*=7.6, 4.8 Hz, 1 H), 7.33 (d, *J*=3.2 Hz, 1 H), 7.19 (d, *J*=2.4 Hz, 1 H), 7.08 (dd, *J*=8.4, 2.4 Hz, 1 H), 7.04 (dd, *J*=9.2, 3.2 Hz, 1 H), 6.20 (d, *J*=1.2 Hz, 1 H), 4.41 (dd, *J*=16.0, 1.2 Hz, 4 H)
MS found value: 478.1 (M)

### Example 22. 6-(2-(3,4-difluorophenoxy)ethoxy)-3-(furan-3-yl)-2-(pyridin-3-yl)-1H-inden-1-one (Compound 47)

Compound 9-6 (50.0 mg, 172 µmol, 1.00 eq) synthesized according to Example 12 and 4-(2-bromoethoxy)-1,2-difluorobenzene (46.0 mg, 194 µmol, 1.12 eq) as RX in Reaction Scheme 2 above were mixed with DMF (3.00 mL), and then K₂CO₃ (36.0 mg, 260 µmol, 1.51 eq) and Nal (5.00 mg, 33.3 µmol, 0.20 eq) were added thereto at 25 °C. The mixture was stirred at 40 °C for 12 hours. While adding dropwise MeOH (1.00 mL), it was confirmed by LCMS that a desired compound was synthesized. The mixture was cooled to 25 °C. The mixture was diluted with water (50.0 mL) and extracted with EtOAc (30.0 mL X 3). The organic layer was washed with brine (15.0 mL), and then dried with Na₂SO₄, and the mixture was filtered and concentrated in a vacuum. The residue was purified by column chromatography (P₁=0.30) to thereby obtain Compound 47 (21.0 mg, purity: 98.5 %) as a red solid.
¹H NMR (400 MHz, METHANOL-d): *δ* ppm 8.54 (d, *J*=4.4 Hz, 1 H), 8.46 (s, 2 H), 7.81 (s, 1 H), 7.71 (d, *J*=8.0 Hz, 1 H), 7.51 (d, *J*=8.0 Hz, 1 H), 7.44 (dd, *J*=8.0, 5.2 Hz, 1 H), 7.36 (q, *J*=9.6 Hz, 1 H), 7.11-7.24 (m, 2 H), 7.08 (dd, *J*=8.0, 2.4 Hz, 1 H), 6.83 (d, *J*=8.8 Hz, 1 H), 6.20 (s, 1 H), 4.39-4.49 (m, 2 H), 4.34 (d, *J*=4.4 Hz, 2 H)
MS found value: 446.1 (M)

### Example 23. 6-(2-(4-(dimethylamino)phenoxy)ethoxy)-3-(furan-3-yl)-2-(pyridin-3-yl)-1H-inden-1-one (Compound 48)

Compound 9-6 (50.0 mg, 172 µmol, 1.00 eq) synthesized according to Example 12 and 4-(2-bromoethoxy)-N,N-dimethylaniline (43.0 mg, 176 µmol, 1.02 eq) as RX in Reaction Scheme 2 above were mixed with DMF (3.00 mL), and then K₂CO₃ (36.0 mg, 260 µmol, and 1.51 eq) and Nal (5.00 mg, 33.3 µmol, 1.93 eq) were added thereto at 25 °C. The mixture was stirred at 45-50 °C for 12 hours. It was confirmed by LCMS that a desired compound was synthesized. The mixture was cooled with water (10.0 mL) and extracted with EtOAc (10.0 mL X 2). The organic layer was washed with NaCl (10.0 mL X 2), and then dried with Na₂SO₄, and the mixture was filtered and concentrated under reduced pressure. The residue was purified by reversed-phase HPLC to thereby obtain Compound 48 (7.00 mg, 13.8 µmol, yield: 8.03 % purity: 89.7 %) as a red gum.
¹H NMR (400 MHz, CHLOROFORM-d): *δ* ppm 8.52-8.61 (m, 2 H), 7.88 (s, 1 H), 7.74 (d, J=8.0 Hz, 1 H), 7.49 (s, 1 H), 7.31-7.42 (m, 2 H), 6.87-7.01 (m, 3 H), 6.76 (d, *J*=9.2 Hz, 2 H), 6.27 (s, 1 H), 4.28-4.42 (m, 4 H), 2.89 (s, 6 H)
MS found value: 453.2 (M+1)

### Example 24. 3-(furan-3-yl)-6-(2-(4-isopropylphenoxy)ethoxy)-2-(pyridin-3-yl)-1H-inden-1-one (Compound 49)

Compound 9-6 (50.0 mg, 172 µmol, 1 eq) synthesized according to Example 12 and 1-(2-bromoethoxy)-4-isopropylbenzene (43.0 mg, 176 µmol, 1.02 eq) as RX in Reaction Scheme 2 above were mixed with DMF (3.00 mL), and then K₂CO₃ (36.0 mg, 260 µmol, 1.51 eq) and Nal (5.00 mg, 33.3 µmol, 1.93 eq) were added thereto at 25 °C. The mixture was stirred at 45-50 °C for 12 hours. While adding dropwise MeOH (1.00 mL), it was confirmed by LCMS that a desired compound was synthesized. The mixture was cooled with water (10.0 mL) and extracted with EtOAc (10.0 mL X 2). The organic layer was washed with NaCl (10.0 mL X 2), and then dried with Na₂SO₄, and the mixture was filtered and concentrated under reduced pressure. The residue was purified by reversed-phase HPLC to thereby obtain Compound 49 (13.00 mg, 26.1 µmol, yield: 15.11 % purity: 90.7 %) as a red gum.
¹H NMR (400 MHz, DMSO-d6): *δ* ppm 8.53-8.60 (m, 2 H), 7.88 (s, 1 H), 7.74 (d, J=8.0 Hz, 1 H), 7.49 (s, 1 H), 7.30-7.39 (m, 2 H), 6.88-7.00 (m, 3 H), 6.76 (d, J=9.2 Hz, 2 H), 6.27 (s, 1 H), 4.25-4.48 (m, 4 H), 2.89 (s, 6 H), 1.23 (br s, 3 H), 1.16 (s, 3 H)
MS found value: 452.2 (M+1)

### Example 25. 3-(furan-3-yl)-6-(((4-methoxybenzyl)oxy)methoxy)-2-(pyridin-3-yl)-1H-inden-1-one (Compound 50)

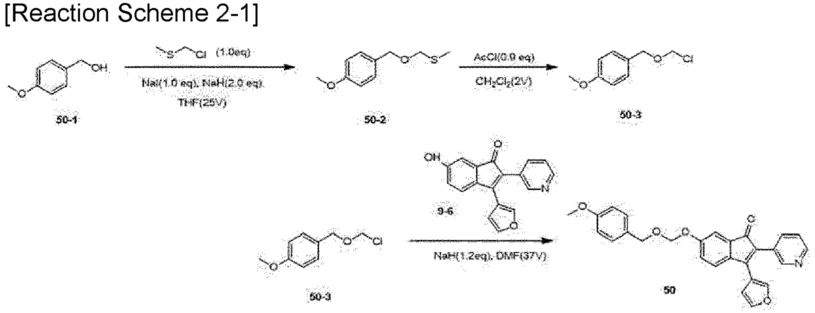

### Step 1: Synthesis of (((4-methoxybenzyl)oxy)methyl)(methyl)sulfane (50-2)

THF (100 mL), Nal (10.8 g, 72.4 mmol, 1 eq), and NaH (5.79 g, 144.76 mmol, purity: 60 %, 2 eq) were mixed at 25 °C. The mixture was cooled to 0 °C, and then mixed with Compound 50-1 (10.0 g, 72.4 mmol, 9.03 mL, 1 eq) of Reaction Scheme 2-1 above, and then a reaction was allowed to occur between the reactants at 25-30 °C. The mixture was stirred at 25-30 °C for 1 hour. Chloro(methylsulfanyl)methane (6.99 g, 72.38 mmol, 6.06 mL, 1 eq) was added thereto, and a reaction was allowed to occur therebetween at 25-30 °C. The reaction mixture was stirred at 25-30 °C for 12 hours in atmosphere including nitrogen. While adding dropwise DCM (0.5 mL), it was confirmed by TLC (PE/EA = 3/1) that a desired compound was synthesized. The mixture was cooled at 0 °C with NH₄Cl (300 mL), and diluted with water (100 mL) and extracted with EtOAc (100 mL X 3). The organic layer was washed with brine (100 mL X 2), and then dried with Na₂SO₄, and the mixture was filtered and concentrated under reduced pressure. The residue was purified by column chromatography (SiO₂, PE: EA = 50:1 to 10:1 P₁=0.20) to thereby obtain Compound 50-2 (9.2 g, 46.40 mmol, yield: 64.11 %) as white oil.

### Step 2: Synthesis of 1-((chloromethoxy)methyl)-4-methoxybenzene (50-3)

Compound 50-2 (500 mg, 2.52 mmol, 1 eq) was dissolved in DMF (1 mL), followed by mixing with acetylchloride (178 mg, 2.27 mmol, 161 µL, 0.9 eq), and the mixture was stirred at 25-30 °C for 12 hours in atmosphere including nitrogen. While adding dropwise DCM (1.00 mL), it was confirmed by TLC (PE: EA = 5:1) that a desired compound was synthesized. The mixture was concentrated in a vacuum and extracted with EA (5 mL). The resulting extract was diluted with water (10 mL) and extracted with EtOAc (10 mL X 2), the organic was washed with water (10 mL X 3), followed by drying with Na₂SO₄, and the mixture was filtered and concentrated under reduced pressure. The residue was obtained as Compound 50-3 (400 mg, 2.14 mmol, yield: 84.99 %) in the form of white oil.

### Step 3: Synthesis of 3-(furan-3-yl)-6-(((4-methoxybenzyl)oxy)methoxy)-2-(pyridin-3-yl)-1H-inden-1-one (Compound 50)

Compound 9-6 (60.0 mg, 207 µmol, 1 eq) synthesized according to Example 12 and NaH (5.97 g, 248 µmol, 1.2 eq) were mixed with DMF (1 mL). Compound 50-3 (77.4 mg, 414 µmol, 2 eq) was added to DMF (0.2 mL), and the resulting solution was added dropwise to the mixture. The mixture was stirred at 25-30 °C for 2 hours in atmosphere including nitrogen. While adding dropwise a mixture of DCM (1.00 mL) and water (1.00 mL), it was confirmed by TLC (PE: EA =1:1) that the reactants were fully consumed. The mixture was cooled at 0 °C with NH₄Cl (30 mL), and diluted with water (10 mL) and extracted with EtOAc (10 mL X 3). The organic layer was washed with brine (5.00 mL X 2), and then dried with Na₂SO₄, and the mixture was filtered and concentrated under reduced pressure. The residue was purified by prep-HPLC (SiO₂, PE: EA = 1:1) to thereby obtain Compound 50 (11.0 mg, 34.9 µmol, yield: 11.1 %, purity: 92.1 %) as a red solid.
¹H NMR (400 MHz, CHLOROFORM-d): *δ* 8.50 (br s, 2H), 7.80 (s, 1H), 7.68 (br d, *J* = 8.0 Hz, 1H), 7.31 - 7.23 (m, 2H), 7.22 - 7.15 (m, 4H), 7.02 (dd, *J =* 8.0, 2.4 Hz, 1H), 6.81 (d, *J* = 8.4 Hz, 2H), 6.19 (d, *J* = 1.2 Hz, 1H), 5.23 (s, 2H), 4.59 (s, 2H), 3.73 (s, 3H)
MS found value: 440.1 [M+H]⁺

### Example 26. 3-(5-methylthiazol-4-yl)-6-(3-morpholinopropoxy)-2-(pyridin-3-yl)-1H-inden-1-one (Compound 4)

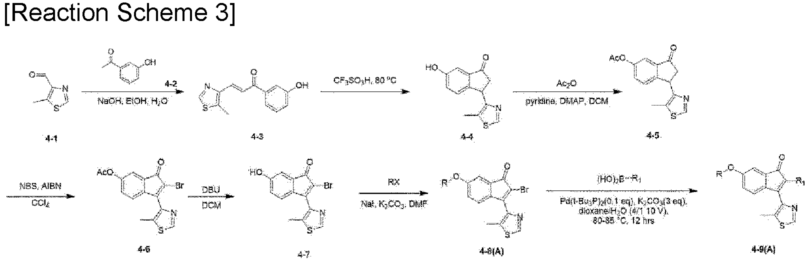

### Step 1: Synthesis of (E)-1-(3-hydroxyphenyl)-3-(5-methylthiazol-4-yl)prop-2-en-1-one (4-3)

Compound 4-1 (500 mg, 3.93 mmol) of Reaction Scheme 3 above and Compound 4-2 (535 mg, 3.93 mmol, 486 µL) of Reaction Scheme 3 above were mixed with ethanol (5.00 mL), and then NaOH (235 mg, 5.90 mmol) in H₂O (2.00 mL) was added thereto at 0 °C in atmosphere including nitrogen. The mixture was stirred at 20 °C for 12 hours. It was confirmed by TLC (DCM: methanol = 10/1) that the reactants were fully consumed. It was confirmed by LCMS that the reactants were fully consumed, and it was confirmed by MS that Compound 4-3 was synthesized. The mixture was adjusted to pH 7 with 1N HCl, and then filtered and concentrated in a vacuum to thereby obtain Compound 4-3 (700 mg, 2.85 mmol, yield: 72.5 %) as a yellow solid.

### Step 2: Synthesis of 6-hydroxy-3-(5-methylthiazol-4-yl)indan-1-one (4-4)

Compound 4-3 (700 mg, 2.85 mmol) was dissolved in triflic acid (10 mL), and then the mixture was stirred at 80 °C for 16 hours. It was confirmed by TLC (PE: EA= 1/1) that the reactants were fully consumed. Water (30.0 mL) was added to the mixture, an aqueous NaHCO₃ solution was added thereto, the resulting solution was adjusted to pH 8, and then the mixture was filtered and concentrated in a vacuum to thereby obtain Compound 4-4 (700 mg, 2.85 mmol, yield: 100.00 %) as a brown solid.

### Step 3: Synthesis of [1-(5-methylthiazol-4-yl)-3-oxo-indan-5-yl] acetate (4-5)

Compound 4-4 (200 mg, 815 µmol) was dissolved in DCM (10.0 mL), and then Ac₂O (416.18 mg, 4.08 mmol, 381.82 µL, 5 eq), pyridine (322 mg, 4.08 mmol, 329 µL, 5 eq), and DMAP (99.6 mg, 815 µmol)were added thereto at 0 °C. The mixture was stirred at 20 °C for 24 hours. It was confirmed by TLC (PE: EA= 1/1) that the reactants were fully consumed. The mixture was diluted with DCM (100 mL) and extracted with brine (30.0 mL x 3). The resulting extract was dried with anhydrous Na₂SO₄, and then concentrated in a vacuum to thereby obtain a residue. The residue was purified by flash silica gel chromatography to thereby obtain Compound 4-5 (130 mg, 452 µmol, yield: 55.4 %) as a white solid.

### Step 4: Synthesis of [2-bromo-1-(5-methylthiazol-4-yl)-3-oxo-inden-5-yl] acetate (4-6)

Compound 4-5 (130 mg, 452 µmol)and NBS (177 mg, 995 µmol)were mixed with carbon tetrachloride (5.00 mL), and then AIBN (7.43 mg, 45.2 µmol) was added thereto in atmosphere including nitrogen. The reaction mixture was stirred at 400 W and 80 °C for 2 hours. It was confirmed by TLC (PE: EA= 2/1) that the reactants were fully consumed. The mixture was diluted with DCM (100 mL) and extracted with brine (30 mL x 3). The resulting extract was dried with anhydrous Na₂SO₄ and filtered, and then concentrated in a vacuum to thereby obtain Compound 4-6 (200 mg) as brown oil.

### Step 5: Synthesis of 2-bromo-6-hydroxy-3-(5-methylthiazol-4-yl)inden-1-one (4-7)

Compound 4-6 (200 mg, 549 µmol) was mixed with DCM (4.00 mL), and then DBU (83.6 mg, 549 µmol, 82.7 µL) was added thereto. The mixture was stirred at 20 °C for 12 hours. It was confirmed by TLC (PE: EA= 1/1) that the reactants were fully consumed. The mixture was diluted with DCM (100 mL) and extracted with brine (30 mL x 3). The resulting extract was dried with anhydrous Na₂SO₄ and filtered, and then concentrated in a vacuum to thereby obtain a residue. The residue was purified by flash silica gel chromatography to thereby obtain Compound 4-7 (50.0 mg, 155 µmol, yield: 28.26 %) as red oil.

### Step 6: Synthesis of 2-bromo-3-(5-methylthiazol-4-yl)-6-(3-morpholinopropoxy)-1H-inden-1-one (Compound 5)

Compound 4-7 (50.0 mg, 155 µmol)and 4-(3-chloropropyl)morpholine (30.5 mg, 186 µmol, 61.7 µL) as RX in Reaction Scheme 3 above were mixed with acetonitrile (2.00 mL), and then K₂CO₃ (64.3 mg, 465 µmol) and KI (25.7 mg, 155 µmol) were added thereto. The mixture was stirred at 60 °C for 2 hours. It was confirmed by LCMS that the reactants were fully consumed, and it was confirmed by MS that Compound 5 was synthesized. The mixture was diluted with EtOAc (50.0 mL) and extracted with brine (10 mL x 3). The resulting extract was dried with anhydrous Na₂SO₄, and then concentrated in a vacuum to thereby obtain a residue. The residue was purified by reversed-phase HPLC to thereby obtain Compound 5 (5.60 mg, 11.7 µmol, yield: 7.58 %) as a red solid.

### Step 7: Synthesis of 3-(5-methylthiazol-4-yl)-6-(3-morpholinopropoxy)-2-(pyridin-3-yl)-1H-inden-1-one (Compound 4)

Compound 5 (30.0 mg, 66.7 µmol) and pyridin-3-ylboronic acid (8.21 mg, 66.7 µmol) as R₁-B(OH)₂ in Reaction Scheme 3 above were mixed with dioxane (2.00 mL) and H₂O (0.50 mL), and then K₃PO₄ (42.5 mg, 200 µmol) and Pd(dtbpf)Cl₂ (4.35 mg, 6.68 µmol) were added thereto in atmosphere including nitrogen. The mixture was stirred with microwaves at 100 °C for 2 hours. It was confirmed by LCMS that the reactants were fully consumed, and it was confirmed by MS that Compound 4 was synthesized. The mixture was filtered and concentrated in a vacuum to thereby obtain a residue. The residue was purified by reversed-phase HPLC to thereby obtain Compound 4 (5.40 mg, 12.1 µmol, yield: 18.0 %) as a red solid.
¹H NMR (400 MHz, CD₃OD): *δ* 9.13 (s, 1H), 8.88 (s, 1H), 8.76 (d, *J* = 6.0 Hz, 1H), 8.38 (d, *J* = 8.4 Hz, 1H), 8.03 (dd, *J* = 6.0, 8.4 Hz, 1H), 7.29 (d, *J* = 2.4 Hz, 1H), 7.24 (d, *J* = 8.0 Hz, 1H), 7.06 (dd, *J* = 2.54, 8.4 Hz, 1H), 4.23 (t, *J* = 5.6 Hz, 2H), 4.06-4.11 (m, 2H), 3.83 (t, *J* = 12.0 Hz, 2H), 3.58 (d, *J* = 12.4 Hz, 2H), 3.38-3.44 (m, 2H), 3.17-3.26 (m, 2H), 2.29-2.36 (m, 2H), 2.24 (s, 3H)
MS found value: 448.0 [M+H]⁺

### Example 27. 2-bromo-3-(5-methylthiazol-4-yl)-6-(3-morpholinopropoxy)-1H-inden-1-one (Compound 5)

Compound 5 was synthesized according to Example 26.
¹H NMR (400 MHz, CD₃OD): *δ* 9.04 (s, 1H), 7.14 (d, *J* = 2.4 Hz, 1H), 6.95-7.01 (m, 1H), 6.89 (dd, *J* = 2.4, 8.0 Hz, 1H), 4.09 (t, *J* = 6.0 Hz, 2H), 3.72 (t, *J* = 4.8 Hz, 4H), 2.52-2.65 (m, 9H), 1.96-2.08 (m, 2H)
MS found value: 448.9 [M+H]⁺

### Example 28. 3-(5-methylthiazol-4-yl)-6-(3-phenylpropoxy)-2-(pyridin-3-yl)-1H-inden-1-one (Compound 8)

### Step 1: Synthesis of 2-bromo-3-(5-methylthiazol-4-yl)-6-(3-phenylpropoxy)-1H-inden-1-one (8-1)

Compound 4-7 (100 mg, 1.00 eq) synthesized according to Example 26 and (3-bromopropyl)benzene (88.0 mg, 1.5 eq) as RX in Reaction Scheme 3 above were mixed with DMF (1.00 mL), and then K₂CO₃ (70.0 mg, 1.83 eq) and Nal (5.00 mg, 0.01 eq) were added thereto at 20 °C to 25 °C in atmosphere including nitrogen. The mixture was stirred at 40 °C to 45 °C for 12 hours. It was confirmed by LCMS that the reactants were fully consumed. Water (10.0 mL) was added to the mixture, and the mixture was adjusted to pH 3 with 1 M HCl, and then extracted with EtOAc (10.0 mL X 3). The mixture was washed with brine (10.0 mL), dried with Na₂SO₄, and filtered and concentrated at 45 °C under reduced pressure. The residue was purified by column chromatography (SiO₂, PE/EA = 50/1 to 20/1) to thereby obtain Compound 8-1 (78.0 mg, yield: 61.5 %, purity: 97.4 %) as a red gel.

### Step 2: Synthesis of 3-(5-methylthiazol-4-yl)-6-(3-phenylpropoxy)-2-(pyridin-3-yl)-1H-inden-1-one (Compound 8)

Compound 8-1 (78.0 mg, 1.00 eq) and pyridin-3-ylboronic acid (32.0 mg, 1.5 eq) as R₁-B(OH)₂ in Reaction Scheme 3 above were mixed, and then added to K₂CO₃ (73.0 mg, 3.01 eq), Pd(t-Bu₃P)₂ (10.0 mg, 0.01 eq), and dioxane (0.80 mL) and water (0.20 mL) at 20 °C to 25 °C in atmosphere including nitrogen. The mixture was stirred at 80 °C to 85 °C for 12 hours. It was confirmed by LCMS that the reactants were fully consumed. Water (10.0 mL) was added to the mixture, and the mixture was adjusted to pH 7 with 1 M HCl, and then extracted with EtOAc (1.00 mL X 3). The organic layer was washed with brine (10.0 mL) and dried with Na₂SO₄, to thereby obtain a concentrated organic phase residue at 45 °C under reduced pressure. The residue was purified by prep-HPLC to thereby obtain Compound 8 (21.0 mg, yield: 26.2 %, purity: 94.2 %) as a red gum.
¹H NMR (400 MHz, DMSO-d6): *δ* ppm 9.23 (s, 1H), 8.72 (br d, *J* = 4.8 Hz, 1H), 8.61 (s, 1H), 7.98 - 8.07 (m, 1H), 7.77 - 7.85 (m, 1H), 7.18 - 7.30 (m, 7H), 7.00 - 7.04 (m, 1 H), 4.07 (br t, *J* = 6.0 Hz, 2H), 2.75 (br t, *J* = 7.6 Hz, 2H), 2.07 (s, 2H), 1.98 (s, 3H)
MS found value: 439.2 [M+H]⁺

### Example 29. 2-(4-methoxyphenyl)-3-(5-methylthiazol-4-yl)-6-phenethoxy-1H-inden-1-one (Compound 18)

### Step 1: Synthesis of 2-bromo-3-(5-methylthiazol-4-yl)-6-phenethoxy-1H-inden-1-one (18-1)

Compound 4-7 (1.5 g, 1.00 eq) synthesized according to Example 26 and (2-bromoethyl)benzene (9.00 g, 6.58 mL, 11.0 eq) as RX in Reaction Scheme 3 above were mixed with DMF (15.0 mL), and then K₂CO₃ (1.83 g, 3.00 eq) and Nal (133 mg, 0.20 eq) were added thereto at 15 °C to 20 °C in atmosphere including nitrogen. The mixture was stirred at 65 °C to 70 °C for 37 hours. It was confirmed by TLC (PE: EA = 3:1) that a desired compound was synthesized. The mixture was cooled with water (45.0 mL) at 15 °C to 20 °C, and diluted and extracted with EtOAc (40.0 mL X 3). The mixture was washed with brine (40.0 mL), dried with Na₂SO₄, and filtered and concentrated at 45 °C under reduced pressure. The residue was purified by column chromatography (SiO₂, PE/EA = 50/1 to 10/1) to thereby obtain Compound 18-1 as a red solid.

### Step 2: Synthesis of 2-(4-methoxyphenyl)-3-(5-methylthiazol-4-yl)-6-phenethoxy-1H-inden-1-one (Compound 18)

Compound 18-1 (100.0 mg, 1.00 eq) and (4-methoxyphenyl)boronic acid (67.0 mg, 2.05 eq) as R₁-B(OH)₂ in Reaction Scheme 3 above were mixed, and then added to K₂CO₃ (90.0 mg, 3.02 eq), Pd(t-Bu₃P)₂ (11.0 mg, 0.10 eq), and dioxane (2.0 mL) and water (0.5 mL) at 20 °C to 25 °C in atmosphere including nitrogen. The mixture was stirred at 80 °C to 85 °C for 19 hours. It was confirmed by LCMS that the reactants were fully consumed. The mixture was cooled with water (10.0 mL) at 15 °C to 20 °C, and diluted and extracted with EtOAc (10.0 mL X 3). The organic layer was washed with brine (10.0 mL), dried with Na₂SO₄, and filtered and concentrated under reduced pressure. The residue was purified by prep-HPLC to thereby obtain Compound 18 as a red solid.
¹H NMR (400 MHz, DMSO-d6): *δ* ppm 1.85 (s, 3 H) 3.05 (t, *J* = 6.8 Hz, 2 H) 3.75 (s, 3 H) 4.27 (t, *J* = 6.8 Hz, 2 H) 6.91 (d, *J* = 8.8 Hz, 2 H) 6.96 (dd, *J* = 8.0, 2.38 Hz, 1 H) 7.06 (d, *J* = 8.0 Hz, 1 H) 7.09 (d, *J* = 2.4 Hz, 1 H) 7.13 (d, *J* = 8.8 Hz, 2 H) 7.21 - 7.25 (m, 1 H) 7.31 - 7.35 (m, 4 H) 9.17 (s, 1 H)
MS found value: 454.2 [M+H]⁺

### Example 30. 2-(4-methoxyphenyl)-3-(5-methylthiazol-4-yl)-6-(4-phenylbutoxy)-1H-inden-1-one (Compound 19)

### Step 1: Synthesis of 2-bromo-3-(5-methylthiazol-4-yl)-6-(4-phenylbutoxy)-1H-inden-1-one (19-1)

Compound 4-7 (300 mg, 1.00 eq) synthesized according to Example 26 and (4-bromobutyl)benzene (239 mg, 1.20 eq) as RX in Reaction Scheme 3 above were mixed with DMF (3.00 mL), and then K₂CO₃ (194 mg, 1.51 eq) and Nal (28.0 mg, 0.02 eq) were added thereto at 20 °C to 25 °C in atmosphere including nitrogen. The mixture was stirred at 40 °C to 45 °C for 12 hours. It was confirmed by TLC (PE: EA = 1:1) that a desired compound was synthesized. The mixture was diluted with water (20.0 mL), adjusted to pH 3 with 1 M HCl, and then extracted with EtOAc (30.0 mL X 3). The mixture was separated, the organic layer was washed with brine (30.0 mL), dried with Na₂SO₄, and then filtered and concentrated at 45 °C under reduced pressure. The residue was purified by column chromatography (SiO₂, PE/EA = 100/1 to 20/1) to thereby obtain Compound 19-1 (275 mg, yield: 64.0 %, purity: 97.9 %) as a red gum.

### Step 2: Synthesis of 2-(4-methoxyphenyl)-3-(5-methylthiazol-4-yl)-6-(4-phenylbutoxy)-1H-inden-1-one (Compound 19)

Compound 19-1 (130 mg, 1.00 eq) and (4-methoxyphenyl)boronic acid (66.0 mg, 1.52 eq) as R₁-B(OH)₂ in Reaction Scheme 3 above were mixed, and then added to K₂CO₃ (118 mg, 3.01 eq), Pd(t-Bu₃P)₂ (15.0 mg, 0.10 eq), and dioxane (0.8 mL) and water (0.2 mL) at 20 °C to 25 °C in atmosphere including nitrogen. The mixture was stirred at 80 °C to 85 °C for 12 hours. It was confirmed by LCMS that the reactants were fully consumed. Water (10.0 mL) was added to the mixture, and the mixture was adjusted to pH 7 with 1 M HCl, and then extracted with EtOAc (10.0 mL X 3). The organic layer was washed with brine (10.0 mL), dried with Na₂SO₄, and filtered and concentrated at 45 °C under reduced pressure. The residue was purified by prep-HPLC to thereby obtain Compound 19 (58.0 mg, yield: 42.0 %, purity: 97.5 %) as a red gum.
¹H NMR (400 MHz, DMSO-d6): *δ* 9.11 - 9.23 (m, 1H), 7.04 - 7.30 (m, 9H), 6.85 - 6.97 (m, 3H), 4.00 - 4.11 (m, 2H), 3.75 (s, 3H), 2.61 - 2.68 (m, 2H), 1.85 (s, 3H), 1.73 (br s, 4H)
MS found value: 482.2 [M+H]⁺

### Example 31. 6-((benzyloxy)methoxy)-2-(4-methoxyphenyl)-3-(5-methylthiazol-4-yl)-1H-inden-1-one (Compound 20)

### Step 1: Synthesis of 6-((benzyloxy)methoxy)-2-bromo-3-(5-methylthiazol-4-yl)-1H-inden-1-one (20-1)

Compound 4-7 (500 mg, 1.00 eq) synthesized according to Example 26 and ((chloromethoxy)methyl)benzene (1.16 g, 5.02 eq) as RX in Reaction Scheme 3 above were mixed with DMF (5.0 mL), and then K₂CO₃ (618 mg, 3.03 eq) and Nal (48 mg, 0.20 eq) were added thereto at 25 °C in atmosphere including nitrogen. The mixture was stirred at 40 °C to 45 °C for 12 hours. It was confirmed by TLC (PE: EA = 3:1) that a desired compound was synthesized. Water (5.00 mL) was added to the mixture, and the mixture was extracted with EtOAc (2 mL X 1). The resulting extract was concentrated under reduced pressure. The residue was purified by column chromatography (SiO₂, PE/EA = 20/1 to 10/1) to thereby obtain Compound 20-1 (300 mg, yield: 34.0 %, purity: 74.4 %) as a red solid.

### Step 2: Synthesis of 6-((benzyloxy)methoxy)-2-(4-methoxyphenyl)-3-(5-methylthiazol-4-yl)-1H-inden-1-one (Compound 20)

Compound 20-1 (100.0 mg, 1.00 eq) and (4-methoxyphenyl)boronic acid (52.0 mg, 1.51 eq) as R₁-B(OH)₂ in Reaction Scheme 3 above were mixed, and then added to K₂CO₃ (94.0 mg, 3.01 eq), Pd(t-Bu₃P)₂ (12.0 mg, 0.10 eq), and dioxane (0.80 mL) and water (0.20 mL) at 25 °C in atmosphere including nitrogen. The mixture was stirred at 80 °C to 85 °C for 12 hours in atmosphere including nitrogen. It was confirmed by LCMS that the reactants were fully consumed. The mixture was diluted with water (3.0 mL) and extracted with EtOAc (1.00 mL), dried with Na₂SO₄, and then filtered and concentrated under reduced pressure. The residue was purified by prep-HPLC to thereby obtain Compound 20 (36.87 mg, yield: 34.5 %, purity: 99.5 %) as a red gum.
¹H NMR (400 MHz, DMSO-d6): *δ* 9.06 - 9.31 (m, 1 H), 7.25 - 7.41 (m, 5 H), 7.23 (s, 1 H), 7.04 - 7.18 (m, 4 H), 6.91 (d, *J* = 8.8 Hz, 2 H) 5.40 (s, 2 H), 4.70 (s, 2 H), 3.75 (s, 3 H), 1.86 (s, 3 H)
MS found value: 470.1 (M+1)

### Example 32. 2-(4-methoxyphenyl)-3-(5-methylthiazol-4-yl)-6-(2-phenoxyethoxy)-1H-inden-1-one (Compound 21)

### Step 1: Synthesis of 2-bromo-3-(5-methylthiazol-4-yl)-6-(2-phenoxyethoxy)-1H-inden-1-one (21-1)

Compound 4-7 (500 mg, 1.00 eq) synthesized according to Example 26 and (2-bromoethoxy)benzene (1.78 g, 6.00 eq) as RX in Reaction Scheme 3 above were mixed with DMF (5.00 mL), and then K₂CO₃ (306 mg, 1.50 eq) and Nal (45.0 mg, 0.20 eq) were added thereto at 15 °C to 20 °C in atmosphere including nitrogen. The mixture was stirred at 55-60 °C for 37 hours. It was confirmed that TLC (PE: EA = 3:1) that a desired compound was synthesized. The mixture was cooled with water (20.0 mL) at 15 °C to 20 °C, and diluted and extracted with EtOAc (20.0 mL X 3). The organic layer was washed with brine (20 mL), dried with Na₂SO₄, and then filtered and concentrated under reduced pressure. The residue was purified by column chromatography (SiO₂, PE/EA = 50/1 to 2/1) to thereby obtain Compound 21-1 as a red solid.

### Step 2: 2-(4-methoxyphenyl)-3-(5-methylthiazol-4-yl)-6-(2-phenoxyethoxy)-1H-inden-1-one (Compound 21)

Compound 21-1 (100.0 mg, 1.00 eq) and (4-methoxyphenyl)boronic acid (61.0 mg, 2.01 eq) as R₁-B(OH)₂ in Reaction Scheme 3 above were mixed, and then added to K₂CO₃ (83.0 mg, 3.01 eq), Pd(t-Bu₃P)₂ (11.0 mg, 0.10 eq), and dioxane (1.60 mL) and water (0.40 mL) at 20 °C to 25 °C in atmosphere including nitrogen. The mixture was stirred at 80 °C to 85 °C for 18 hours in atmosphere including nitrogen. It was confirmed by LCMS that the reactants were fully consumed. The mixture was cooled with water (10.0 mL) at 15 °C to 20 °C, and diluted and extracted with EtOAc (10.0 mL X 3). The organic layer was washed with brine (10.0 mL), dried with Na₂SO₄, and filtered and concentrated under reduced pressure. The residue was purified by prep-HPLC to thereby obtain Compound 21 as a red solid.
¹H NMR (400 MHz, DMSO-d6): *δ* ppm 1.85 (s, 3 H) 3.05 (t, *J* = 6.8 Hz, 2 H) 3.75 (s, 3 H) 4.27 (t, *J* = 6.8 Hz, 2 H) 6.91 (d, *J* = 8.8 Hz, 2 H) 6.96 (dd, *J* = 8.0, 2.4 Hz, 1 H) 7.06 (d, *J* = 8.0 Hz, 1 H) 7.09 (d, *J* = 2.4 Hz, 1 H) 7.13 (d, *J* = 8.8 Hz, 2 H) 7.21 - 7.25 (m, 1 H) 7.31 - 7.35 (m, 4 H) 9.17 (s, 1 H)
MS found value: 454.2 [M+H]⁺

### Example 33. 6-(2-(3,4-dichlorophenoxy)ethoxy)-3-(5-methylthiazol-4-yl)-2-(pyrimidin-5-yl)-1H-inden-1-one (Compound 26)

### Step 1: Synthesis of 2-bromo-6-(2-(3,4-dichlorophenoxy)ethoxy)-3-(5-methylthiazol-4-yl)-1H-inden-1-one (26-1)

Compound 4-7 (0.50 g, 1.00 eq) synthesized according to Example 26 and 4-(2-bromoethoxy)-1,2-dichlorobenzene(503 mg, 1.20 eq) as RX in Reaction Scheme 3 above were mixed with DMF (5 mL), and then K₂CO₃ (322 mg, 1.50 eq) and Nal (47 mg, 2.02 e-1eq) at 15 °C to 20 °C in atmosphere including nitrogen. The mixture was stirred at 40 °C to 45 °C for 18 hours. It was confirmed by TLC (PE: EA = 3:1) that a desired compound was synthesized. Water (20 mL) was added to the mixture, and the mixture was adjusted to pH 3 with 1 M HCl, and then extracted with EtOAc (30 mL X 3). The organic layer was washed with brine (30 mL), dried with Na₂SO₄, and filtered and concentrated at 45 °C under reduced pressure. The residue was purified by column chromatography (SiO₂, PE/EA = 10/1 to 3/1) to thereby obtain Compound 26-1 (401 mg, yield: 43.3 %, purity: 85.7 %) as a red solid.

### Step 2: Synthesis of 6-(2-(3,4-dichlorophenoxy)ethoxy)-3-(5-methylthiazol-4-yl)-2-(pyrimidin-5-yl)-1H-inden-1-one (Compound 26)

Compound 26-1 (100 mg, 1.00 eq) and pyrimidin-5-ylboronic acid (37 mg, 1.53 eq) as R₁-B(OH)₂ in Reaction Scheme 3 above were mixed, and then added to K₂CO₃ (81.0 mg, 586.08 µmol, 3 eq), Pd(t-Bu₃P)₂ (10.0 mg, 0.10 eq), and dioxane (2 mL) and water (0.5 mL) at 15 °C to 25 °C in atmosphere including nitrogen. The mixture was stirred at 80 °C to 85 °C for 12 hours in atmosphere including nitrogen. It was confirmed by LCMS that a desired compound was synthesized. Water (20 mL) was added to the mixture, and the mixture was extracted with EtOAc (30 mL X 3). The organic layer was washed with brine (30 mL), dried with Na₂SO₄, and filtered and concentrated at 45 °C under reduced pressure. The residue was purified by reversed-phase HPLC to thereby obtain Compound 26 (17.04 mg, yield: 19.17 %, purity: 95.2 %) as an orange solid.
¹H NMR (400 MHz, DMSO-d6): *δ* 9.22 (s, 1H), 9.10 (s, 1H), 8.60 (s, 2H), 7.54 (d, *J* = 8.8 Hz, 1H), 7.32 (d, *J* = 2.8 Hz, 1H), 7.26-7.20 (m, 2H), 7.06 (ddd, *J* = 2.8, 8.4, 17.2 Hz, 2H), 4.41 (br dd, *J* = 5.2, 14.4 Hz, 4H), 2.00 (s, 3H)
MS found value: 510.1 (M+1)

### Example 34. 6-(2-(3,4-difluorophenoxy)ethoxy)-3-(5-methylthiazol-4-yl)-2-(pyrimidin-5-yl)-1H-inden-1-one (Compound 27)

### Step 1: Synthesis of 2-bromo-6-(2-(3,4-difluorophenoxy)ethoxy)-3-(5-methylthiazol-4-yl)-1H-inden-1-one (27-1)

Compound 4-7 (1.00 g, 3.10 mmol, 1.00 eq) synthesized according to Example 26 and 4-(2-bromoethoxy)-1,2-dichlorobenzene (883 mg, 1.20 eq) as RX in Reaction Scheme 3 above were mixed with DMF (10 mL), and then K₂CO₃ (644 mg, 1.50 eq) and Nal (94 mg, 0.20 eq) at 15 °C to 20 °C in atmosphere including nitrogen. The mixture was stirred at 40 °C to 45 °C for 18 hours. It was confirmed by TLC (PE: EA = 3:1) that a desired compound was synthesized. Water (20 mL) was added to the mixture, and the mixture was adjusted to pH 3 with 1 M HCl, and then extracted with EtOAc (30 mL X 3). The organic layer was washed with brine (30 mL), dried with Na₂SO₄, and then filtered and concentrated at 45 °C under reduced pressure. The residue was purified by column chromatography (SiO₂, PE/EA = 10/1 to 3/1) to thereby obtain Compound 27-1 (690 mg, yield: 42.8 %, purity: 92.1 %) as a red solid.

### Step 2: Synthesis of 6-(2-(3,4-difluorophenoxy)ethoxy)-3-(5-methylthiazol-4-yl)-2-(pyrimidin-5-yl)-1H-inden-1-one (Compound 27)

Compound 27-1 (100 mg, 1.00 eq) and pyrimidin-5-ylboronic acid (39.0 mg, 1.51 eq) as R₁-B(OH)₂ in Reaction Scheme 3 above were mixed, and then added to K₂CO₃ (87.0 mg, 3.01 eq), Pd(t-Bu₃P)₂ (11 mg, 1.03 e-1 eq), and dioxane (2 mL) and water (0.5 mL) at 15 °C to 25 °C in atmosphere including nitrogen. The mixture was stirred at 80 °C to 85 °C for 12 hours in atmosphere including nitrogen. It was confirmed by TLC (PE: EA = 1:1) that a desired compound was synthesized. Water (20 mL) was added to the mixture, and the mixture was extracted with EtOAc (30 mL X 3). The organic layer was washed with brine (30 mL), dried with Na₂SO₄, and then filtered and concentrated at 45 °C under reduced pressure. The residue was purified by column chromatography (SiO₂, PE/EA = 10/1 to 2/1) to thereby obtain Compound 27 (31.0 mg, yield: 28.5 %, purity: 91.9 %) as a brown-red solid.
¹H NMR (400 MHz, DMSO-d6): *δ* 9.22 (s, 1H), 9.10 (s, 1H), 8.60 (s, 2H), 7.42-7.32 (m, 1H), 7.27-7.20 (m, 2H), 7.16 (ddd, *J* = 2.8, 6.8, 12.8 Hz, 1H), 7.07 (dd, *J* = 2.4, 8.0 Hz, 1H), 6.87-6.80 (m, 1H), 4.46-4.31 (m, 4H), 2.00 (s, 3H)
MS found value: 478.1 (M+1)

### Example 35. 6-(2-(4-methoxyphenoxy)ethoxy)-3-(5-methylthiazol-4-yl)-2-(4-(trifluoromethyl)phenyl)-1H-inden-1-one (Compound 32)

### Step 1: Synthesis of 2-bromo-6-(2-(4-methoxyphenoxy)ethoxy)-3-(5-methylthiazol-4-yl)-1H-inden-1-one (32-1)

Compound 4-7 (200 mg, 1.00 eq) synthesized according to Example 26 and 1-(2-bromoethoxy)-4-methoxybenzene (164 mg, 1.20 eq) as RX in Reaction Scheme 3 above were mixed with DMF (1.00 mL), and then K₂CO₃ (124 mg, 1.52 eq) and Nal (19.0 mg, 0.02 eq) at 20 °C to 25 °C in atmosphere including nitrogen. The mixture was stirred at 40 °C to 45 °C for 18 hours. It was confirmed by TLC (PE: EA = 3:1) that a desired compound was synthesized. Water (10.0 mL) was added to the mixture, and the mixture was adjusted to pH 3 with 1 M HCl, and then extracted with EtOAc (20.0 mL X 2). The resulting extract was dried with Na₂SO₄, and then concentrated. The residue was purified by column chromatography (SiO₂, PE/EA = 30/1 to 3/1) to thereby obtain Compound 32-1 (199 mg, yield: 70 %, purity: 100 %) as a red solid.

### Step 2: Synthesis of 6-(2-(4-methoxyphenoxy)ethoxy)-3-(5-methylthiazol-4-yl)-2-(4-(trifluoromethyl)phenyl)-1H-inden-1-one (Compound 32)

Compound 32-1 (90.0 mg, 1.00 eq) and ((4-(trifluoromethyl)phenyl)boronic acid (54.0 mg, 1.49 eq) as R₁-B(OH)₂ in Reaction Scheme 3 above were mixed, and then added to K₂CO₃ (79.0 mg, 3.00 eq), Pd(t-Bu₃P)₂ (10.0 mg, 0.02 eq), and dioxane(2.00 mL) and water (0.5 mL) at 20 °C to 25 °C in atmosphere including nitrogen. The mixture was stirred at 80 °C to 85 °C for 8 hours in atmosphere including nitrogen. It was confirmed by LCMS that a desired compound was synthesized. Water (20 mL) was added to the mixture, and the mixture was extracted with EtOAc (10.0 mL X 2). The organic layer was washed with brine (10.0 mL X 2), dried with Na₂SO₄, and then filtered and concentrated in a vacuum. The mixture was titrated with ACN/MeOH = 5/1 (3.00 mL) at 20 °C to 25 °C for 30 minutes and filtered to thereby obtain a residue. The residue was obtained as Compound 32 (26.4 mg, yield: 24.54 %, purity: 95.2 %) in the form of a red solid.
¹H NMR (400 MHz, DMSO-d6): *δ* ppm 9.21 (s, 1 H), 8.40 (br d, *J* = 6.0 Hz, 2 H), 7.72 (br d, *J* = 8.0 Hz, 2 H), 7.40 (br d, *J* = 8.0 Hz, 2 H), 7.12 - 7.29 (m, 2 H), 6.94 - 7.11 (m, 3 H), 4.44 (s, 4 H), 1.85 (s, 3 H)
MS found value: 509.3 (M+1)

### Example 36. 2-(4-fluorophenyl)-6-(2-(4-methoxyphenoxy)ethoxy)-3-(5-methylthiazol-4-yl)-1H-inden-1-one (Compound 22)

Compound 32-1 (100 mg, 1.00 eq) synthesized according to Example 35 and (4-fluorophenyl)boronic acid (45.0 mg, 1.52 eq) as R₁-B(OH)₂ in Reaction Scheme 3 above were mixed, and then added to K₂CO₃ (88.0 mg, 3.01 eq), Pd(t-Bu₃P)₂ (11.0 mg, 0.1 eq), and dioxane (2 mL) and water (0.5 mL) at 15 °C to 20 °C in atmosphere including nitrogen. The mixture was stirred at 80 °C to 85 °C for 14 hours in atmosphere including nitrogen. It was confirmed by LCMS that a desired compound was synthesized. Water (20 mL) was added to the mixture, and the mixture was extracted with EtOAc. The organic layer was washed with brine, dried with Na₂SO₄, and then filtered and concentrated under reduced pressure. The residue was purified by flash silica gel chromatography to thereby obtain Compound 22 (12.05 mg, yield: 11.59 %, purity: 99.3 %) as a red solid.
¹H NMR (400 MHz, DMSO-d6): *δ* ppm 9.20 (s, 1 H), 7.18 - 7.24 (m, 5 H), 7.12 - 7.16 (m, 1 H), 7.01 - 7.06 (m, 1 H), 6.91 - 6.95 (m, 2 H), 6.85 - 6.90 (m, 2 H), 4.36 - 4.41 (m, 2 H), 4.24 - 4.29 (m, 2 H), 3.71 (s, 3 H), 1.86 (s, 3 H)
MS found value: 488.3 (MS+1)

### Example 37. 6-(2-(3,4-dimethoxyphenoxy)ethoxy)-3-(5-methylthiazol-4-yl)-2-(4-(trifluoromethyl)phenyl)-1H-inden-1-one (Compound 33)

### Step 1: Synthesis of 2-bromo-6-(2-(3,4-dimethoxyphenoxy)ethoxy)-3-(5-methylthiazol-4-yl)-1H-inden-1-one (33-1)

Compound 4-7 (500 mg, 1.00 eq) synthesized according to Example 26 and 4-(2-bromoethoxy)-1,2-dimethoxybenzene (642 mg, 1.50 eq) as RX in Reaction Scheme 3 above were mixed with DMF (5.00 mL), and then K₂CO₃ (306 mg, 1.50 eq) and Nal (44.0 mg, 0.02 eq) were added thereto at 20 °C to 25 °C in atmosphere including nitrogen. The mixture was stirred at 40 °C to 45 °C for 18 hours. It was confirmed by LCMS that a desired compound was synthesized. Water (10.0 mL) was added to the mixture, and the mixture was adjusted to pH 3 with 1 M HCl, and then extracted with EtOAc (20.0 mL X 2). The resulting extract was dried with Na₂SO₄, and then concentrated. The residue was purified by column chromatography (SiO₂, EA/DCM = 0/1 to 1/20), and then concentrated to thereby obtain Compound 33-1 (172 mg, yield: 22.0 %, purity: 95.0 %) as a red solid.

### Step 2: Synthesis of 6-(2-(3,4-dimethoxyphenoxy)ethoxy)-3-(5-methylthiazol-4-yl)-2-(4-(trifluoromethyl)phenyl)-1H-inden-1-one (Compound 33)

Compound 33-1 (80.0 mg, 1.00 eq) and ((4-(trifluoromethyl)phenyl)boronic acid (45.0 mg, 1.49 eq) as R₁-B(OH)₂ in Reaction Scheme 3 above were mixed, and then added to a solution obtained by dissolving K₂CO₃ (66.0 mg, 3.00 eq) in water (0.25 mL), Pd(t-Bu₃P)₂ (16.0 mg, 0.02 eq), and dioxane (1.00 mL) at 20 °C to 25 °C in atmosphere including nitrogen. The mixture was stirred at 80 °C to 85 °C for 8 hours in atmosphere including nitrogen. It was confirmed by LCMS that a desired compound was synthesized. Water (20.0 mL) was added to the mixture, and the mixture was extracted with EtOAc (10.0 mL X 2). The organic layer was washed with brine (10.0 mL X 2), dried with Na₂SO₄, and then filtered and concentrated in a vacuum. The residue was purified by reversed-phase HPLC under 0.10 % HCl conditions to thereby obtain Compound 33 (17.3 mg, yield: 18.6 %, purity: 97.4 %) as a red solid.
¹H NMR (400 MHz, DMSO-d6): *δ* ppm 9.21 (s, 1 H), 7.72 (d, *J* = 8.4 Hz, 2 H), 7.41 (d, *J* = 8.0 Hz, 2 H), 7.15 - 7.25 (m, 2 H), 7.06 (dd, *J* = 8.4, 2.4 Hz, 1 H), 6.86 (d, *J* = 9.2 Hz, 1 H), 6.62 (d, *J* = 2.8 Hz, 1 H), 6.48 (dd, *J* = 8.8, 3.2 Hz, 1 H), 4.40 (dd, *J* =5.6 , 2.8 Hz, 2 H), 4.20 - 4.33 (m, 2 H), 3.74 (s, 3 H), 3.69 (s, 3 H), 1.85 (s, 3 H)
MS found value: 568.3 (M+1)

### Example 38. 6-(2-(3,4-dimethoxyphenoxy)ethoxy)-2-(4-fluorophenyl)-3-(5-methylthiazol-4-yl)-1H-inden-1-one (Compound 23)

Compound 33-1 (80.0 mg, 1.00 eq) synthesized according to Example 37 and (4-fluorophenyl)boronic acid (34.0 mg, 1.53 eq) as R₁-B(OH)₂ in Reaction Scheme 3 above were mixed, and then added to K₂CO₃ (67.0 mg, 3.04 eq), Pd(t-Bu₃P)₂ (10.0 mg, 0.12 eq), and dioxane (2 mL) and water (0.5 mL) at 15 °C to 20 °C in atmosphere including nitrogen. The mixture was stirred at 80 °C to 85 °C for 14 hours in atmosphere including nitrogen. It was confirmed by LCMS that a desired compound was synthesized. Water (20 mL) was added to the mixture, and the mixture was extracted with EtOAc. The organic layer was washed with brine, dried with Na₂SO₄, and then filtered and concentrated under reduced pressure. The residue was purified by prep-HPLC to thereby obtain Compound 23 (13.60 mg, purity: 95.4 %) as a red solid.
¹H NMR (400 MHz, DMSO-d6): *δ* ppm 9.20 (s, 1 H), 7.17 - 7.27 (m, 5 H), 7.15 (d, *J* = 8.0 Hz, 1 H), 7.04 (dd, *J* = 8.0, 2.4 Hz, 1 H), 6.86 (d, *J* = 8.8 Hz, 1 H), 6.63 (d, *J* = 2.4 Hz, 1 H), 6.49 (dd, *J* = 8.8, 2.75 Hz, 1 H), 4.38 (br d, *J* = 4.4 Hz, 2 H), 4.27 (br d, *J* = 2.4 Hz, 2 H), 3.74 (s, 3 H), 3.69 (s, 3 H), 1.86 (s, 3 H)
MS found value: 518.3 (MS+1)

### Example 39. 6-(2-(benzo[d][1,3]dioxol-5-yloxy)ethoxy)-3-(5-methylthiazol-4-yl)-2-(4-(trifluoromethyl)phenyl)-1H-inden-1-one (Compound 34)

### Step 1: Synthesis of 6-(2-(benzo[d][1,3]dioxol-5-yloxy)ethoxy)-2-bromo-3-(5-methylthiazol-4-yl)-1H-inden-1-one (34-1)

Compound 4-7 (200 mg, 1.00 eq) synthesized according to Example 26 and 5-(2-bromoethoxy)benzo[d][1,3]dioxole (173 mg, 1.19 eq) as RX in Reaction Scheme 3 above were mixed with DMF (1.00 mL), and then K₂CO₃ (124 mg, 1.52 eq) and Nal (18.0 mg, 0.02 eq) were added thereto at 20 °C to 25 °C in atmosphere including nitrogen. The mixture was stirred at 40 °C to 45 °C for 18 hours. It was confirmed by TLC (PE/EA = 3/1) that a desired compound was synthesized. Water (10.0 mL) was added to the mixture, and the mixture was adjusted to pH 3 with 1 M HCl, and then extracted with EtOAc (20.0 mL X 2). The resulting extract was dried with Na₂SO₄, and then concentrated. The residue was purified by column chromatography (SiO₂, PE/EA = 30/1 to 3/1), and then concentrated to thereby obtain Compound 34-1 (150 mg, yield: 48.0 %, purity: 91.8 %) as a red solid.

### Step 2: Synthesis of 6-(2-(benzo[d][1,3]dioxol-5-yloxy)ethoxy)-3-(5-methylthiazol-4-yl)-2-(4-(trifluoromethyl)phenyl)-1H-inden-1-one (Compound 34)

Compound 34-1 (40.0 mg, 1.00 eq) and ((4-(trifluoromethyl)phenyl)boronic acid (24.0 mg, 1.54 eq) as R₁-B(OH)₂ in Reaction Scheme 3 above were mixed, and then added to a solution obtained by dissolving K₂CO₃ (34.0 mg, 2.99 eq) in water (0.25 mL), Pd(t-Bu₃P)₂ (5.00 mg, 0.02 eq), and dioxane (1.00 mL) at 20 °C to 25 °C in atmosphere including nitrogen. The mixture was stirred at 80 °C to 85 °C for 8 hours in atmosphere including nitrogen. It was confirmed by LCMS that a desired compound was synthesized. Water (20.0 mL) was added to the mixture, and the mixture was extracted with EtOAc (10.0 mL X 2). The organic layer was washed with brine (10.0 mL X 2), dried with Na₂SO₄, and then filtered and concentrated in a vacuum. The residue was purified by reversed-phase HPLC under 0.10 % HCl conditions to thereby obtain Compound 34 (24.1 mg, yield: 53.1 %, purity: 99.6 %) as a red solid.
¹H NMR (400 MHz, DMSO-d6): *δ* ppm 9.21 (s, 1 H), 7.72 (d, *J* = 8.4 Hz, 2 H), 7.41 (d, *J* = 8.0 Hz, 2 H) ,7.15 - 7.25 (m, 2 H), 7.06 (dd, *J* = 8.4, 2.4 Hz, 1 H), 6.86 (d, *J* = 9.2 Hz, 1 H), 6.62 (d, *J* = 2.8 Hz, 1 H), 6.48 (dd, *J* = 8.8, 3.2 Hz, 1 H), 4.40 (dd, *J* =5.6 , 2.8 Hz, 2 H), 4.20 - 4.33 (m, 2 H), 3.74 (s, 3 H), 3.69 (s, 3 H), 1.85 (s, 3 H)
MS found value: 552.3 (M+1)

### Example 40. 6-(2-(benzo[d][1,3]dioxol-5-yloxy)ethoxy)-2-(4-fluorophenyl)-3-(5-methylthiazol-4-yl)-1H-inden-1-one (Compound 24)

Compound 34-1 (90.0 mg, 1.00 eq) synthesized according to Example 39 and (4-fluorophenyl)boronic acid (40.0 mg, 1.54 eq) as R₁-B(OH)₂ in Reaction Scheme 3 above were mixed, and then added to K₂CO₃ (78.0 mg, 3.05 eq), Pd(t-Bu₃P)₂ (10.0 mg, 0.1 eq), and dioxane (2 mL) and water (0.5 mL) at 15 °C to 20 °C in atmosphere including nitrogen. The mixture was stirred at 80 °C to 85 °C for 14 hours in atmosphere including nitrogen. It was confirmed by LCMS that a desired compound was synthesized. Water (20 mL) was added to the mixture, and the mixture was extracted with EtOAc. The organic layer was washed with brine, dried with Na₂SO₄, and then filtered and concentrated under reduced pressure to thereby obtain a residue. The residue was purified by flash silica gel chromatography to thereby obtain Compound 24 (14.72 mg, yield: 15.8 %, purity: 99.8 %) as a red solid.
¹H NMR (400 MHz, DMSO-d6): *δ* ppm 9.20 (s, 1 H), 7.17 - 7.24 (m, 5 H), 7.14 (d, *J*=8.0 Hz, 1 H), 7.03 (dd, *J*=8.0, 2.4 Hz, 1 H), 6.83 (d, *J*=8.4 Hz, 1 H), 6.69 (d, *J*=2.8 Hz, 1 H), 6.43 (dd, *J*=8.8, 2.8 Hz, 1 H), 5.97 (s, 2 H), 4.37 (m, 2 H), 4.23 - 4.28 (m, 2 H), 1.86 (s, 3 H)
MS found value: 502.2 (MS+1)

### Example 41. 3-(5-methylthiazol-4-yl)-6-(2-(pyridin-4-yloxy)ethoxy)-2-(4-(trifluoromethyl)phenyl)-1H-inden-1-one (Compound 35)

### Step 1: Synthesis of 2-bromo-6-(2-((t-butyldimethylsilyl)oxy)ethoxy)-3-(5-methylthiazol-4-yl)-1H-inden-1-one (35-1)

Compound 4-7 (1.00 g, 1.00 eq) synthesized according to Example 26 and (2-bromoethoxy)(tert-butyl)dimethylsilane (1.06 g, 1.50 eq) were mixed with DMF (10.0 mL), and then K₂CO₃ (613 mg, 1.50 eq) and Nal (89.0 mg, 0.02 eq) were added thereto at 20 °C to 25 °C in atmosphere including nitrogen. The mixture was stirred at 40 °C to 45 °C for 18 hours. It was confirmed by LCMS that a desired compound was synthesized. Water (50.0 mL) was added to the mixture, and the mixture was adjusted to pH 3 with 1 M HCl, and then extracted with EtOAc (50.0 mL X 2). The resulting extract was dried with Na₂SO₄, and then concentrated. The residue was purified by column chromatography (SiO₂, PE/EA = 30/1 to 3/1), and then concentrated to thereby obtain Compound 35-1 (723 mg, yield: 50.9 %) as a red solid.

### Step 2: Synthesis of 2-bromo-6-(2-hydroxyethoxy)-3-(5-methylthiazol-4-yl)-1H-inden-1-one (Compound 35-2)

Compound 35-1 (723 mg, 1.00 eq) and HCl (2.00 M, 1.50 mL, 2.00 eq) were added to THF (4.00 mL), followed by stirring at 20 °C to 25 °C for 12 hours. It was confirmed by TLC (PE/EA = 1/1) that a desired compound was synthesized. Water (10.0 mL) was added to the mixture, and the mixture was adjusted to pH 8-9 with NaHCO₃, and then extracted with EtOAc (20.0 mL X 2). The resulting extract was concentrated, and then was not purified, to thereby obtain Compound 35-2 (521 mg, yield: 94.5 %) as a red solid.

### Step 3: Synthesis of 6-(2-hydroxyethoxy)-3-(5-methylthiazol-4-yl)-2-(4-(trifluoromethyl)phenyl)-1H-inden-1-one (Compound 35-3)

Compound 35-2 (270 mg, 1.00 eq) and ((4-(trifluoromethyl)phenyl)boronic acid (210 mg, 1.50 eq) as R₁-B(OH)₂ in Reaction Scheme 3 above were mixed, and then added to a solution obtained by dissolving K₂CO₃ (307 mg, 3.01 eq) in water (0.7 mL), Pd(t-Bu₃P)₂ (75.0 mg, 0.02 eq), and dioxane (2.70 mL) at 20 °C to 25 °C in atmosphere including nitrogen. The mixture was stirred at 80 °C to 85 °C for 8 hours in atmosphere including nitrogen. It was confirmed by TLC that a desired compound was synthesized. Water (20.0 mL) was added to the mixture, and the mixture was extracted with EtOAc (10.0 mL X 2). The organic layer was washed with brine (10.0 mL X 2), dried with anhydrous Na₂SO₄, and then filtered and concentrated in a vacuum. The residue was purified by column chromatography (SiO₂, PE/EA = 10/1 to 0/1), and then concentrated to thereby obtain Compound 35-3 (219 mg, yield: 62.4 %, purity: 90.6 %) as a red solid.

### Step 4: Synthesis of 3-(5-methylthiazol-4-yl)-6-(2-(pyridin-4-yloxy)ethoxy)-2-(4-(trifluoromethyl)phenyl)-1H-inden-1-one (Compound 35)

Compound 35-3 (110 mg, 1.00 eq) and pyridin-4-ol (29.0 mg, 1.50 eq) instead of RX in Reaction Scheme 3 were mixed with DCM (0.50 mL), and then PPh₃ (118 mg, 1.76 eq) was added thereto at 20 °C to 25 °C in atmosphere including nitrogen. The mixture was cooled at 0-5 °C, and then DIAD (91.0 mg, 1.77 eq) was added thereto, followed by stirring for 2 hours in atmosphere including nitrogen. It was confirmed by TLC (PE/EA = 1/2) that a desired compound was synthesized. Water (20.0 mL) was added to the mixture, and the mixture was extracted with EtOAc (50.0 mL X 3). The resulting extract was dried with Na₂SO₄, and then concentrated. The residue was purified by prep-TLC (EA: THF = 1:1), and titrated with 3 mL of PE/MTBE = 10/1 at 20 °C to 25 °C for 30 minutes. The residue was filtered and concentrated, to thereby obtain Compound 35 as an orange solid.
¹H NMR (400 MHz, DMSO-d6): *δ* ppm 9.21 (s, 1 H), 8.40 (br d, *J* = 6.0 Hz, 2 H), 7.72 (br d, *J* = 8.0 Hz, 2 H), 7.40 (br d, *J* = 8.0 Hz, 2 H), 7.12 - 7.29 (m, 2 H), 6.94 - 7.11 (m, 3 H), 4.44 (s, 4 H), 1.85 (s, 3 H)
MS found value: 509.3 (M+1)

### Example 42. 2-(4-fluorophenyl)-3-(5-methylthiazol-4-yl)-6-(2-(pyridin-4-yloxy)ethoxy)-1H-inden-1-one (Compound 25)

### Step 1: Synthesis of 2-(4-fluorophenyl)-6-(2-hydroxyethoxy)-3-(5-methylthiazol-4-yl)-1H-inden-1-one (25-1)

Compound 35-2 (250 mg, 1.00 eq) synthesized according to Example 41 and (4-fluorophenyl)boronic acid (146 mg, 1.53 eq) as R₁-B(OH)₂ in Reaction Scheme 3 above were mixed, and then added to a solution obtained by dissolving K₂CO₃ (287 mg, 3.04 eq) in water (1 mL), Pd(t-Bu₃P)₂ (43.0 mg, 0.12 eq), and dioxane (4 mL) at 15 °C to 20 °C in atmosphere including nitrogen. The mixture was stirred at 80 °C to 85 °C for 14 hours in atmosphere including nitrogen. It was confirmed by LCMS that a desired compound was synthesized. Water (20.0 mL) was added to the mixture, and the mixture was extracted with EtOAc. The organic layer was washed with brine, dried with Na₂SO₄, and then filtered and concentrated under reduced pressure. The residue was purified by column chromatography (SiO₂, PE/EA = 10/1 to 3/1), and then concentrated to thereby obtain Compound 25-1 (103 mg, yield: 35.9 %, purity: 90.8 %) as a red solid.

### Step 2: Synthesis of 2-(4-fluorophenyl)-3-(5-methylthiazol-4-yl)-6-(2-(pyridin-4-yloxy)ethoxy)-1H-inden-1-one (Compound 25)

Compound 25-1 (100.0 mg, 1.00 eq) and pyridin-4-ol (25.0 mg, 1.00 eq) instead of RX in Reaction Scheme 3 above were mixed with THF (5 mL), and then PPh₃ (103 mg, 1.50 eq) and DIAD (80.0 mL, 1.51 eq) were added thereto at 0-10 °C in atmosphere including nitrogen. The mixture was stirred at 15 °C to 25 °C for 14 hours in atmosphere including nitrogen. It was confirmed by LCMS that a desired compound was synthesized. Water (20.0 mL) was added to the mixture, and the mixture was extracted with EtOAc. The organic layer was washed with brine, dried with Na₂SO₄, and then filtered and concentrated under reduced pressure. The resulting extract was dried with Na₂SO₄, and then concentrated. The residue was purified by column chromatography (SiO₂, PE/EA = 10/1 to 3/1) to thereby obtain Compound 25 (27.58 mg, yield: 20.72 %, purity: 90.3 %) as a red gum.
¹H NMR (400 MHz, DMSO-d6): *δ* ppm 9.20 (s, 1 H), 8.39 - 8.43 (m, 2 H), ppm 7.13 - 7.25 (m, 6 H), 7.02 - 7.06 (m, 3 H), 4.44 (s, 4 H), 1.87 (s, 3 H)
MS found value: 459.1 (MS+1)

### Example 43. 3-(5-methylthiazol-4-yl)-6-phenethoxy-2-(thiophen-2-yl)-1H-inden-1-one (Compound 28)

Compound 18-1 (150 mg, 1.00 eq) synthesized according to Example 29 and tributyl(thiophen-2-yl)stannane (157 mg, 1.30 eq) instead of R₁-B(OH)₂ in Reaction Scheme 3 above were mixed, and then added to Pd₂(dba)₃ (60.0 mg, 0.20 eq) and dioxane (4.00 mL) at 20 °C to 25 °C in atmosphere including nitrogen. The mixture was stirred at 115-120 °C for 2 hours in atmosphere including nitrogen. It was confirmed by LCMS that the reactants were fully consumed. The mixture was cooled with water (15.0 mL) at 15 °C to 20 °C, and extracted with EtOAc (15.0 mL X 3). The organic layer was washed with brine (15.0 mL), dried with Na₂SO₄, and then filtered and concentrated under reduced pressure to thereby obtain a residue. The residue was purified by prep-HPLC to thereby obtain Compound 28 as a dark brown solid.
¹H NMR (400 MHz, DMSO-d6): *δ* ppm 2.17 (s, 3 H) 3.04 (t, *J* = 6.8 Hz, 2 H) 4.26 (t, *J* = 6.8 Hz, 2 H) 6.85 - 6.89 (m, 1 H) 6.92 - 6.96 (m, 1H) 7.04 - 7.10 (m, 2 H) 7.26 - 7.36 (m, 6 H) 7.56 (m, 1 H) 9.21 (s, 1 H)
MS found value: 430.1 [M+H]⁺

### Example 44. 3-(5-methylthiazol-4-yl)-6-(4-phenylbutoxy)-2-(thiophen-2-yl)-1H-inden-1-one (Compound 29)

Compound 19-1 (50.0 mg, 1.00 eq) synthesized according to Example 30 and tributyl(thiophen-2-yl)stannane (55.0 mg, 1.31 eq) instead of R₁-B(OH)₂ in Reaction Scheme 3 above were mixed, and then added to Pd₂(dba)₃ (20.0 mg, 0.02 eq) and dioxane (1.50 mL) at 25-30 °C in atmosphere including nitrogen. The mixture was stirred with microwaves at 115-120 °C for 2 hours. It was confirmed by LCMS that the reactants were fully consumed. Water (10.0 mL) was added to the mixture at 15 °C to 20 °C, and the mixture was extracted with EtOAc (10.0 mL X 3). The organic layer was washed with brine (10.0 mL), dried with Na₂SO₄, and then filtered and concentrated at 45 °C under reduced pressure, to thereby obtain a residue. The residue was purified by prep-HPLC to thereby obtain Compound 29 (58.0 mg, yield: 58.7 %, purity: 99.5 %) as red oil.
¹H NMR (400 MHz, DMSO-d6): *δ* 9.22 (s, 1H), 7.55 - 7.57 (m, 1H), 7.18 - 7.31 (m, 6H), 7.05 - 7.10 (m, 2H), 6.85 - 6.94 (m, 2H), 4.03 - 4.07 (m, 2H), 2.62 - 2.67 (m, 2H), 2.18 (s, 3H), 1.70 - 1.75 (m, 4H)
MS found value: 458.1 [M+H]⁺

### Example 45. 6-((benzyloxy)methoxy)-3-(5-methylthiazol-4-yl)-2-(thiophen-2-yl)-1H-inden-1-one (Compound 30)

Compound 20-1 (100 mg, 1.00 eq) synthesized according to Example 31 and tributyl(thiophen-2-yl)stannane (110 mg, 1.30 eq) instead of R₁-B(OH)₂ in Reaction Scheme 3 above were mixed, and then added to Pd₂(dba)₃ (42.0 mg, 0.20 eq) and dioxane (1.00 mL) at 20 °C to 25 °C in atmosphere including nitrogen. The mixture was stirred at 120 °C for 2 hours in atmosphere including nitrogen. It was confirmed by LCMS that the reactants were fully consumed. Water (3.0 mL) was added to the mixture, and the mixture was extracted with EtOAc (1.0 mL X 1). The resulting extract was dried with Na₂SO₄, and then filtered and concentrated under reduced pressure, to thereby obtain a residue. The residue was purified by prep-HPLC to thereby obtain Compound 30 (51.82 mg, yield: 51.0 %, yield: 99.3 %) as a dark purple solid.
¹H NMR (400 MHz, DMSO-d6): *δ* 9.23 (s, 1 H), 7.58 (dd, *J* = 5.2, 1.2 Hz, 1 H), 7.26 - 7.39 (m, 6 H), 7.23 (d, *J* = 2.4 Hz, 1 H), 7.02 - 7.14 (m, 2 H), 6.91 (d, *J* = 8.0 Hz, 1 H), 5.40 (s, 2 H), 4.70 (s, 2 H), 2.12 - 2.28 (m, 3 H)
MS found value: 446.0 (M+1)

### Example 46. 3-(5-methylthiazol-4-yl)-6-(2-phenoxyethoxy)-2-(thiophen-2-yl)-1H-inden-1-one (Compound 31)

Compound 21-1 (150 mg, 1.00 eq) synthesized according to Example 32 and tributyl(thiophen-2-yl)stannane (146 mg, 1.30 eq) instead of R₁-B(OH)₂ in Reaction Scheme 3 above were mixed, and then added to Pd₂(dba)₃ (55.0 mg, 0.20 eq) and dioxane (4.50 mL) at 20 °C to 25 °C in atmosphere including nitrogen. The mixture was stirred at 115-120 °C for 2 hours in atmosphere including nitrogen. It was confirmed by LCMS that the reactants were fully consumed. The mixture was cooled with water (15.0 mL) at 15 °C to 20 °C, and extracted with EtOAc (15.0 mL X 3). The organic layer was washed with brine (15.0 mL), dried with Na₂SO₄, and then filtered and concentrated under reduced pressure to thereby obtain a residue. The residue was purified by prep-HPLC to thereby obtain Compound 31 as a dark brown solid.
¹H NMR (400 MHz, DMSO-d6): *δ* ppm 2.19 (s, 3 H) 4.29 - 4.34 (m, 2 H) 4.40 (dd, *J* = 5.6, 2.8 Hz, 2 H) 6.89 - 7.02 (m, 5 H) 7.07 (m, 1 H) 7.17 (d, *J*=2.4 Hz, 1 H) 7.27 - 7.33 (m, 3 H) 7.57 (dd, *J* = 5.2, 1.2 Hz, 1 H) 9.23 (s, 1 H)
MS found value: 446.0 [M+H]⁺

### Example 47. 6-(2-(3,4-dichlorophenoxy)ethoxy)-3-(5-methylthiazol-4-yl)-2-(thiophen-2-yl)-1H-inden-1-one (Compound 36)

Compound 26-1 (100 mg, 1.00 eq) synthesized according to Example 33 and tributyl(thiophen-2-yl)stannane (96.0 mg, 1.32 eq) instead of R₁-B(OH)₂ in Reaction Scheme 3 above were mixed, and then added to Pd₂(dba)₃ (36.0 mg, 39.31 µmol, 0.20 eq) and dioxane (3 mL) at 15 °C to 25 °C in atmosphere including nitrogen. The mixture was stirred at 145-150 °C for 2 hours in atmosphere including nitrogen. It was confirmed by LCMS that a desired compound was synthesized. Water (20 mL) was added to the mixture, and the mixture was extracted with EtOAc (30 mL X 3). The organic layer was washed with brine (30 mL), dried with Na₂SO₄, and then filtered and concentrated at 45 °C under reduced pressure, to thereby obtain a residue. The residue was purified by reversed-phase HPLC to thereby obtain Compound 36 (50.2 mg, yield: 49.5 %, purity: 99.4 %) as a dark purple solid.
¹H NMR (400 MHz, DMSO-d6): *δ* 9.23 (s, 1H), 7.64 - 7.48 (m, 2H), 7.36-7.25 (m, 2H), 7.16 (d, *J* = 2.4 Hz, 1H), 7.09-6.97 (m, 3H), 6.90 (d, *J* = 8.0 Hz, 1H), 4.38 (br d, *J* = 2.8 Hz, 4H), 2.19 (s, 3H)
MS found value: 514.0 (M+1)

### Example 48. 6-(2-(3,4-difluorophenoxy)ethoxy)-3-(5-methylthiazol-4-yl)-2-(thiophen-2-yl)-1H-inden-1-one (Compound 37)

Compound 27-1 (100 mg, 1.00 eq) synthesized according to Example 34 and tributyl(thiophen-2-yl)stannane (103 mg, 1.32 eq) instead of R₁-B(OH)₂ in Reaction Scheme 3 above were mixed, and then added to Pd₂(dba)₃ (39 mg, 0.20 eq) and dioxane (3 mL) at 15 °C to 25 °C in atmosphere including nitrogen. The mixture was stirred at 145-150 °C for 2 hours in atmosphere including nitrogen. It was confirmed by LCMS that a desired compound was synthesized. Water (20 mL) was added to the mixture, and the mixture was extracted with EtOAc (30 mL X 3). The organic layer was washed with brine (30 mL), dried with Na₂SO₄, and then filtered and concentrated at 45 °C under reduced pressure, to thereby obtain a residue. The residue was purified by reversed-phase HPLC to thereby obtain Compound 37 (30.4 mg, yield: 29.9 %, purity: 99.0 %) as a dark purple solid.
¹H NMR (400 MHz, DMSO-d6): *δ* 9.22 (s, 1H), 9.10 (s, 1H), 8.60 (s, 2H), 7.42-7.32 (m, 1H), 7.27-7.20 (m, 2H), 7.16 (ddd, *J* = 2.8, 6.8, 12.8 Hz, 1H), 7.07 (dd, *J* = 2.4, 8.0 Hz, 1H), 6.87-6.80 (m, 1H), 4.46-4.31 (m, 4H), 2.00 (s, 3H)
MS found value: 478.1 (M+1)

### Example 49. 6-(3-phenylpropoxy)-2-(pyridin-3-yl)-3-(thiophen-2-yl)-1H-inden-1-one (Compound 6)

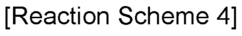

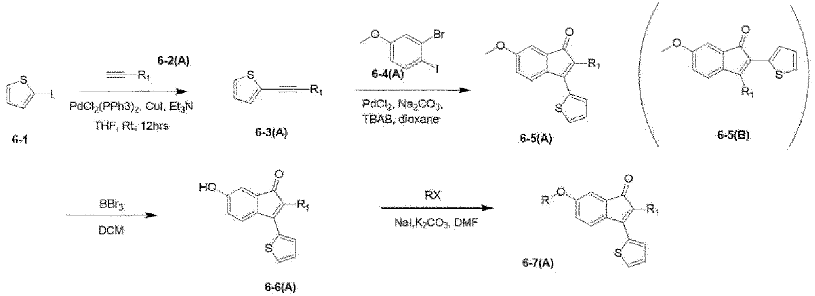

### Step 1: Synthesis of 3-(thiophen-2-ylethynyl)pyridine (6-3)

Compound 6-1 (1.70 g, 8.08 mmol, 823 µL) of Reaction Scheme 4 above and pyridine as R₁ and 3-ethynylpyridine (1.00 g, 9.70 mmol, 558 µL) as Compound 6-2(A) in Reaction Scheme 4 above were mixed with THF (10.0 mL), and then Et₃N (2.45 g, 24.2 mmol, 3.37 mL), Cul (769 mg, 4.04 mmol), and PdCl₂(PPh3)₂ (567 mg, 808 umol) were added thereto at 25 °C in atmosphere including nitrogen. The mixture was stirred at 25 °C for 12 hours. It was confirmed by LCMS that Compound 6-3 was synthesized, and it was confirmed by TLC (PE: EA = 3/1) that the reactants were fully consumed. The residue was diluted with EtOAc (200 mL). The combined organic phase layer was extracted with brine (20 mL x 3). The resulting extract was dried with anhydrous Na₂SO₄ and filtered, and then concentrated under reduced pressure to thereby obtain a residue. The residue was purified by flash silica gel chromatography to obtain Compound 6-3, i.e., 3-(thiophen-2-ylethynyl)pyridine (1.16 g, 6.26 mmol, yield: 77.49 %) as yellow oil.

### Step 2: Synthesis of 6-methoxy-2-(pyridin-3-yl)-3-(thiophen-2-yl)-1H-inden-1-one (6-5)

3-(thiophen-2ylethynyl)pyridine (600 mg, 3.24 mmol) as Compound 6-3 synthesized according to step 1 and Compound 6-4A (2.03 g, 6.48 mmol) were mixed with dioxane (5.00 mL), and then TBAB (1.04 g, 3.24 mmol), Na₂CO₃ (686 mg, 6.48 mmol), and PdCl₂ (57.4 mg, 323 µmol) were added thereto at 25 °C under carbon monoxide (15 psi) conditions. The mixture was stirred at 100 °C for 12 hours. It was confirmed by TLC (PE: EA= 1/1) that the reactants were fully consumed. The residue was diluted with EtOAc (200 mL). The combined organic phase layer was extracted with brine (20.0 mL x 3). The resulting extract was dried with anhydrous Na₂SO₄ and filtered, and then concentrated under reduced pressure to thereby obtain a residue. The residue was purified by flash silica gel chromatography, and then purified by prep-HPLC, to thereby obtain Compound 6-5 as a red solid (6-methoxy-2-(pyridin-3-yl)-3-(thiophen-2-yl)-1H-inden-1-one, 100 mg, 313 µmol, yield: 9.67 %) and 6-methoxy-3-(pyridin-3-yl)-2-(thiophen-2-yl)-1H-inden-1-one (140 mg, 438.35 µmol, yield: 13.53 %) as purple solid-type Compound 6-5(B).

### Step 3: Synthesis of 6-hydroxy-2-(pyridin-3-yl)-3-(thiophen-2-yl)-1H-inden-1-one (6-6)

6-methoxy-2-(pyridin-3-yl)-3-(thiophen-2-yl)-1H-inden-1-one (70.0 mg, 219.18 µmol) as Compound 6-5 was mixed with DCM (2.00 mL), and then BBr₃ (55.0 mg, 219 µmol, 21.1 µL) was added thereto at 0 °C in atmosphere including nitrogen. The mixture was stirred at 25 °C for 12 hours. It was confirmed by TLC (PE: EA= 1/1) that the reactants were fully consumed. The reaction mixture was cooled with an aqueous NaHCO₃ solution (10.0 mL), no additional reaction (work up) was performed, and Compound 6-6 (6-hydroxy-2-(pyridin-3-yl)-3-(thiophen-2-yl)-1H-inden-1-one, 150mg) was obtained as red oil.

### Step 4: Synthesis of 6-(3-phenylpropoxy)-2-(pyridin-3-yl)-3-(thiophen-2-yl)-1H-inden-1-one (Compound 6)

6-hydroxy-2-(pyridin-2-yl)-3-(thiophen-2-yl)-1H-inden-1-one (70.0 mg, 229 µmol) as Compound 6-6 and (3-bromopropyl)benzene (68.5 mg, 343 µmol, 51.8 µL) as RX in Reaction Scheme 4 above were mixed with acetonitrile (3.00 mL), and then K₂CO₃ (79.2 mg, 573 µmol) was added thereto at 25 °C in atmosphere including nitrogen. The mixture was stirred at 60 °C for 12 hours. It was confirmed by LCMS that the reactants were fully consumed. The residue was diluted with EtOAc (200 mL). The combined organic phase layer was extracted with brine (20.0 mL x 3). The resulting extract was dried with anhydrous Na₂SO₄ and filtered, and then concentrated under reduced pressure to thereby obtain a residue. The residue was purified by prep-HPLC to thereby obtain Compound 6 (5.00 mg, 11.6 µmol, yield: 5.05 %, purity: 98.15 %) as a red solid.
¹H NMR (400 MHz, CD₃OD): *δ* 8.53-8.56 (m, 2H), 7.97-8.0 (m, 1H), 7.74 (dd, *J* = 8.0, 5.2 Hz, 1H), 7.59-7.62 (m, 1H), 7.48-7.55 (m, 2H), 7.15-7.33 (m, 7H), 6.97 (dd, *J* = 2.4, 8.0 Hz, 1H), 4.04 (t, *J* = 6.0 Hz, 2H), 2.82 (t, *J* = 7.6 Hz, 2H), 2.04-2.18 (m, 2H)
MS found value: 424.1 [M+H]⁺

### Example 50. 6-(3-phenylpropoxy)-3-(1H-pyrazol-3-yl)-2-(pyridin-3-yl)-1H-inden-1-one (Compound 7)

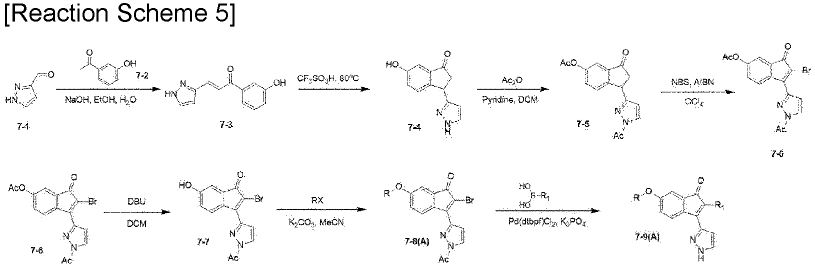

### Step 1: Synthesis of (E)-1-(3-hydroxyphenyl)-3-(1H-pyrazol-5-yl)prop-2-en-1-one (7-3)

Compound 7-1 (5.00 g, 52.0 mmol) of Reaction Scheme 5 above and Compound 7-2 (7.08 g, 52.0 mmol) of Reaction Scheme 5 above were mixed with ethanol (75.0 mL), and then NaOH (3.12 g, 78.05 mmol) in H₂O (30.0 mL) was added thereto at 0 °C. The reaction mixture was stirred at 25 °C for 16 hours. It was confirmed by TLC (PE: EA= 3/1) that the reactants were fully consumed. The residue was diluted with H₂O (50 mL) and extracted with EtOAc (50.0 mL x 2). The combined organic phase layer was extracted with brine (50 mL x 2). The resulting extract was dried with Na₂SO₄ and filtered, and then concentrated under reduced pressure to thereby obtain a residue. The residue was purified by column chromatography (SiO₂, PE/EA = 1/0 to 3/1) to thereby obtain Compound 7-3 (1.25 g) as a yellow solid.

### Step 2: Synthesis of 6-hydroxy-3-(1H-pyrazol-5-yl)-2,3-dihydro-1H-inden-1-one (7-4)

Compound 7-3 (1.10 g, 5.13 mmol) was mixed with triflic acid (10.0 mL), and then the mixture was stirred at 80 °C for 16 hours. It was confirmed by TLC (PE: EA= 1/1) that the reactants were fully consumed. The residue was diluted with H₂O (50.0 mL) and extracted with EtOAc (50.0 mL x 2). The combined organic phase layer was extracted with brine (50.0 mL x 2). The resulting extract was dried with Na₂SO₄ and filtered, and then concentrated under reduced pressure to thereby obtain a residue. The residue was purified by column chromatography (SiO₂, PE/EA = 1/0 to 1/1) to thereby obtain Compound 7-4 (0.80 g, 3.73 mmol, yield: 72.7 %) as a dark brown solid.

### Step 3: Synthesis of [1-(1-acetylpyrazol-3-yl)-3-oxo-indan-5-yl] acetate (7-5)

Compound 7-4 (500 mg, 2.33 mmol) and Ac₂O (1.19 g, 11.6 mmol) were mixed with DCM (10 mL), and then pyridine (923 mg, 11.6 mmol) and DMAP (285 mg, 2.33 mmol) were added thereto at 0 °C in atmosphere including nitrogen. The mixture was stirred at 20 °C for 12 hours. It was confirmed by TLC (PE: EA= 1/1) that the reactants were fully consumed. The reaction mixture was diluted with DCM (100 mL) and extracted with brine (30.0 mL x 3). The resulting extract was dried with anhydrous Na₂SO₄, and then concentrated in a vacuum to thereby obtain a residue. The residue was purified by flash silica gel chromatography to thereby obtain Compound 7-5 (540 mg, 1.81 mmol, yield: 77.5 %) as a yellow solid.

### Step 4: Synthesis of [1-(1-acetylpyrazol-3-yl)-2-bromo-3-oxo-inden-5-yl] acetate (7-6)

Compound 7-5 (540 mg, 1.81 mmol) and NBS (708 mg, 3.98 mmol) were dissolved in carbon tetrachloride (10.0 mL), and then AIBN (29.7 mg, 181 µmol) was added thereto in atmosphere including nitrogen. The mixture was stirred at 400 W and 80 °C for 2 hours. It was confirmed by TLC (PE: EA= 1/1) that the reactants were fully consumed. The mixture was diluted with DCM (100 mL) and extracted with brine (30.0 mL x 3). The resulting extract was dried with anhydrous Na₂SO₄ and filtered, and then concentrated in a vacuum to thereby obtain Compound 7-6 (800 mg) as red oil.

### Step 5: Synthesis of 3-(1-acetylpyrazol-3-yl)-2-bromo-6-hydroxy-inden-1-one (7-7)

Compound 7-6 (800 mg, 2.13 mmol) was dissolved in DCM (10.0 mL), and then DBU (324 mg, 2.13 mmol) was added thereto at 0 °C. The mixture was stirred at 20 °C for 12 hours. It was confirmed by TLC (PE: EA= 2/1) that the reactants were fully consumed. The mixture was adjusted to pH 6 with 1N HCl, and then the mixture was extracted with DCM (50.0 mL x 3). The combined organic phase layer was dried with anhydrous Na₂SO₄ and filtered, and then concentrated in a vacuum to thereby obtain a residue. The residue was purified by flash silica gel chromatography to thereby obtain Compound 7-7 (40.0 mg, 120 µmol, yield: 5.63 %) as a red solid.

### Step 6: Synthesis of 3-(1-acetyl-1H-pyrazol-3-yl)-2-bromo-6-(3-phenylpropoxy)-1H-inden-1-one (Compound 7-8)

Compound 7-7 (40.0 mg, 120 µmol) and (3-bromopropyl)benzene (35.8 mg, 180 µmol) as RX in Reaction Scheme 5 above were mixed with acetonitrile (3.00 mL), and then K₂CO₃ (49.8 mg, 360 µmol) was added thereto at 20 °C, and the mixture was stirred at 20 °C for 3 hours. It was confirmed by TLC (PE: EA= 2/1) that the reactants were fully consumed. The mixture was diluted with EtOAc (50.0 mL) and extracted with brine (20.0 mL x 3). The resulting extract was dried with anhydrous Na₂SO₄, and then concentrated in a vacuum to thereby obtain a residue. The residue was purified by flash silica gel chromatography to thereby obtain Compound 7-8 (3-(1-acetyl-1H-pyrazol-3-yl)-2-bromo-6-(3-phenylpropoxy)-1H-inden-1-one, 6.00 mg, 12.37 µmol, yield: 10.30 %) as yellow oil.

### Step 7: Synthesis of 6-(3-phenylpropoxy)-3-(1H-pyrazol-3-yl)-2-(pyridin-3-yl)-1H-inden-1-one (Compound 7)

Compound 7-8 (6.00 mg, 12.37 µmol) and pyridin-3-ylboronic acid (1.82 mg, 14.8 µmol) wherein pyridine was used as R₁ in B(OH)₂-R₁ of Reaction Scheme 5 above were mixed with dioxane (1.50 mL) and H₂O (0.50 mL), and then K₃PO₄ (7.88 mg, 37.11 µmol) and Pd(dtbpf)Cl₂ (806 µg, 1.24 µmol) were added thereto. The mixture was stirred at 100 °C for 10 hours in atmosphere including nitrogen. It was confirmed by TLC (DCM: methanol = 10/1) that the reactants were fully consumed. The mixture was diluted with H₂O (10.0 mL) and extracted with EtOAc (20.0 mL x 3). The combined organic phase layer was dried with anhydrous Na₂SO₄ and filtered, and then concentrated in a vacuum to thereby obtain a residue. The residue was purified by prep-TLC (SiO₂, DCM: methanol = 10/1) to thereby obtain Compound 7 (3.50 mg, 8.59 µmol, yield: 64.61 %, purity: 89.36 %) as a red solid.
¹H NMR (400 MHz, CD₃OD): *δ* 8.45-8.52 (m, 2H), 7.80-7.90 (m, 2H), 7.63 (d, *J* = 2.4 Hz, 1H), 7.41-7.49 (m, 1H), 7.26-7.33 (m, 2H,), 7.13-7.22 (m, 3H), 7.00 (d, *J* = 2.4 Hz, 1H), 6.83 (dd, *J* = 2.4, 8.0 Hz, 1H), 6.18 (d, *J* = 2.4 Hz, 1H), 4.24 (t, *J* = 6.8 Hz, 2H), 2.63 (t, *J*=7.6 Hz, 2H), 2.18-2.25 (m, 2H)
MS found value: 452.8 [M+H]⁺

### Experimental Examples

### Experimental Example 1. Amyloid-beta (Aβ) Disaggregation Test

### 1.1. Sample Preparation

Each compound was dissolved in DMSO to a concentration of 10 mM. Subsequently, the compound was diluted with 6 % DMSO (in DW) and used.

Specifically, amyloid-beta and tau solutions were prepared by dissolving Aβ₁₋₄₂ or Tau-RD3 (Tau repeat domain 3) monomer in DMSO to a concentration of 10 mM. Thereafter, each solution was diluted with DW to a concentration of 100 µM and stored on ice. Meanwhile, thioflavin-T was dissolved in 50 mM glycine buffer (pH 8.5) to a concentration of 5 mM. Thereafter, the resulting solution was diluted with 50 mM glycine buffer (pH 8.5) to a concentration of 5 µM and stored in a dark room while light was blocked.

### 1.2. Amyloid-beta and Tau Disaggregation Test by Thioflavin-T Assay

Each of the amyloid-beta and tau solutions prepared according to Experimental Example 1.1 was added to a 1.5 mL microcentrifuge tube to a concentration of 50 µM or 35 µM, and then a reaction was allowed to occur therebetween at 37 °C for 24 hours. Each of the 1.5 mL microcentrifuge tubes in which amyloid-beta aggregation and tau aggregation had been completed was treated with each compound at a concentrated of 500 µM to 0 µM through serial dilution, and then a further reaction was allowed to occur therebetween at 37 °C for 48 hours. 25 µL of the reaction solution in which the reaction had been completed was added to each well of a 96-well plate for fluorescence analysis, and 75µL of the previously prepared thioflavin-T solution was added to each well. After a reaction was allowed to occur at room temperature in the dark for 5 minutes, fluorescence values were measured at excitation 450 nm and emission 485 nm by using a multi-mode microplate reader.

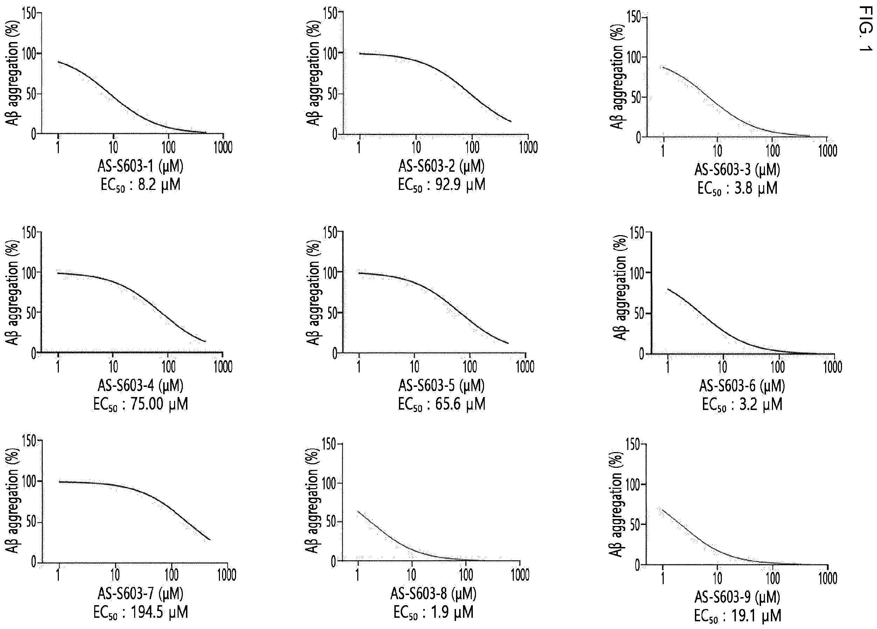

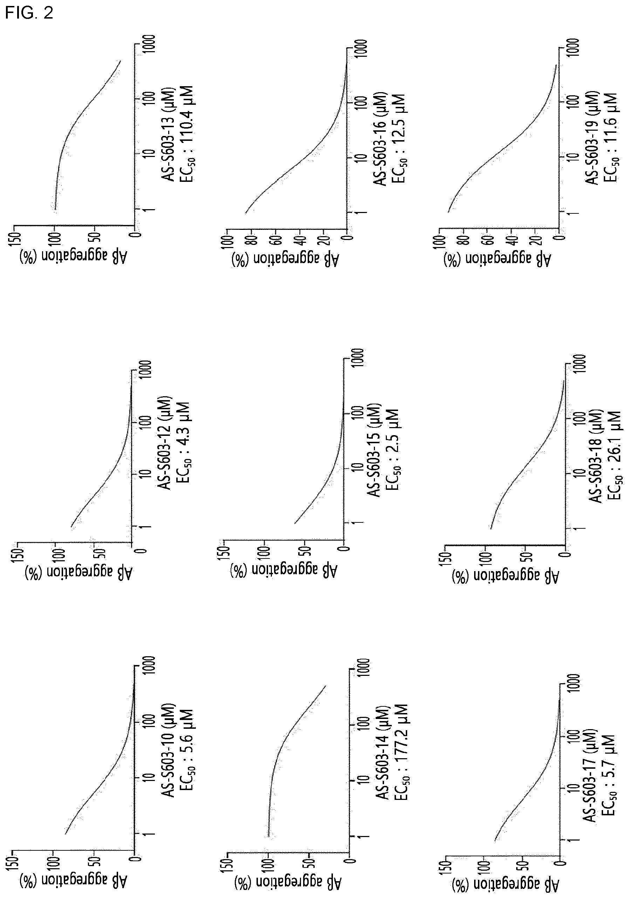

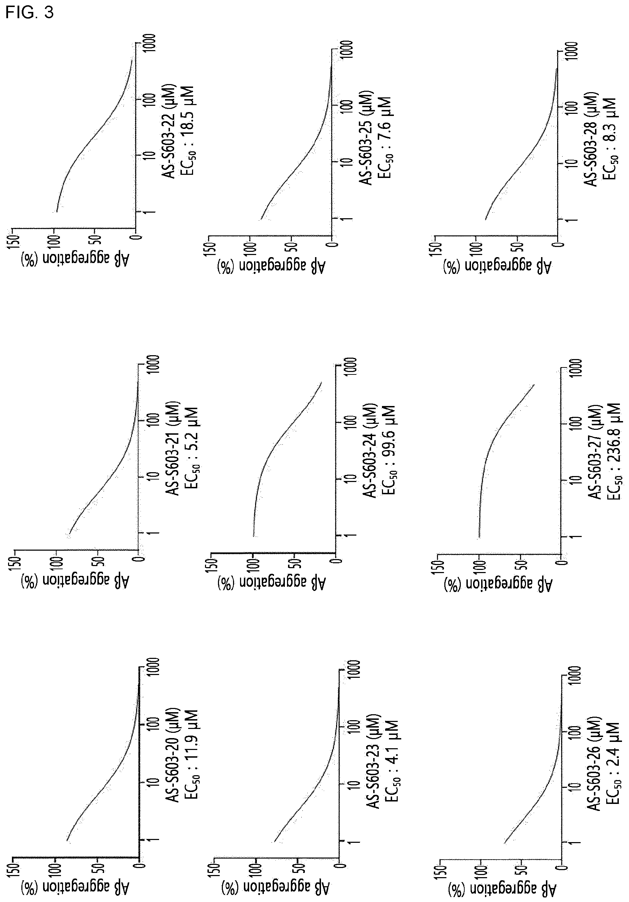

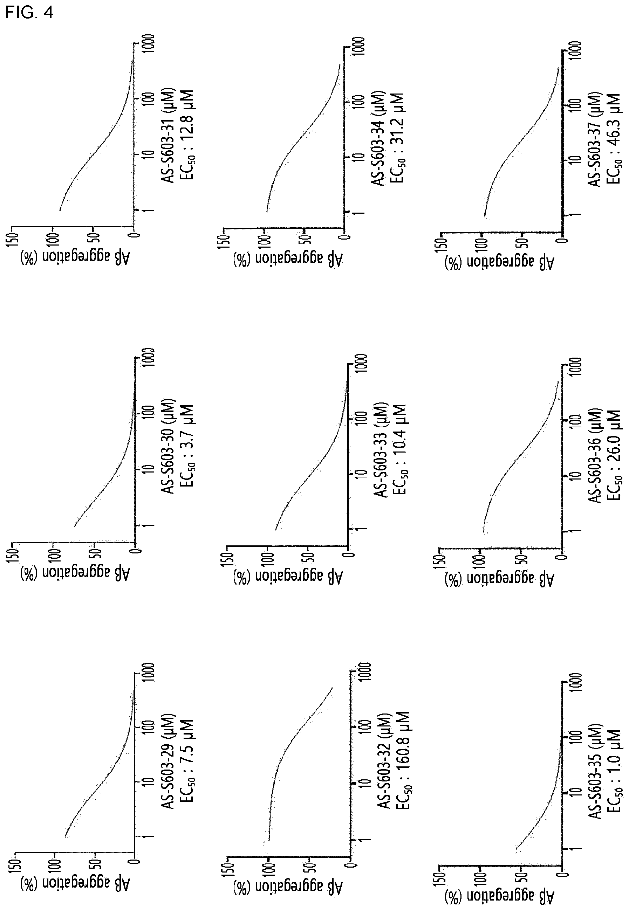

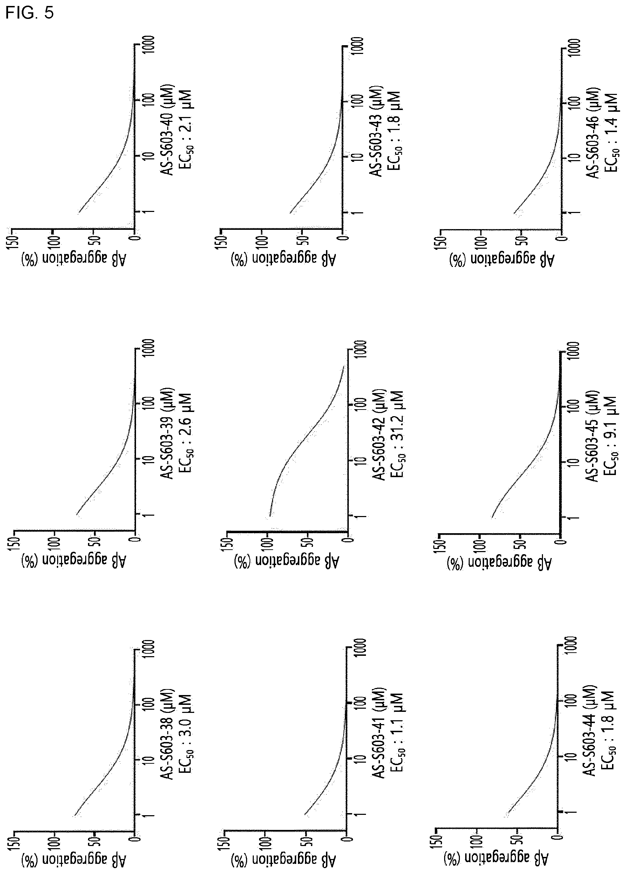

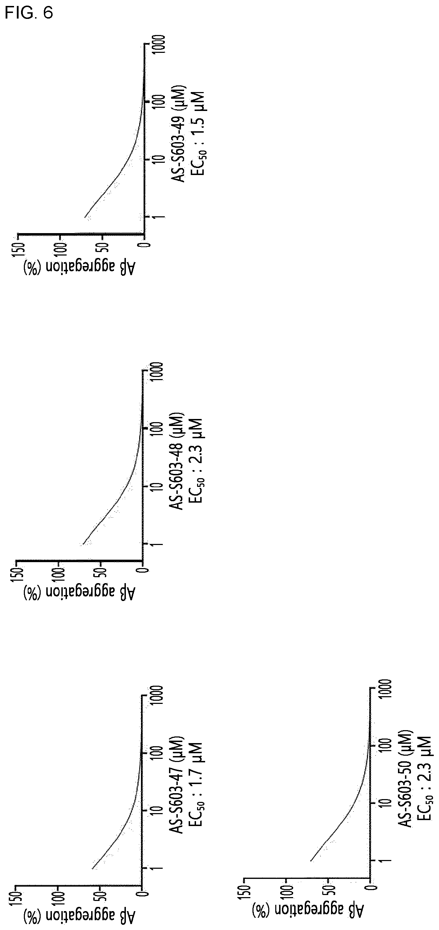

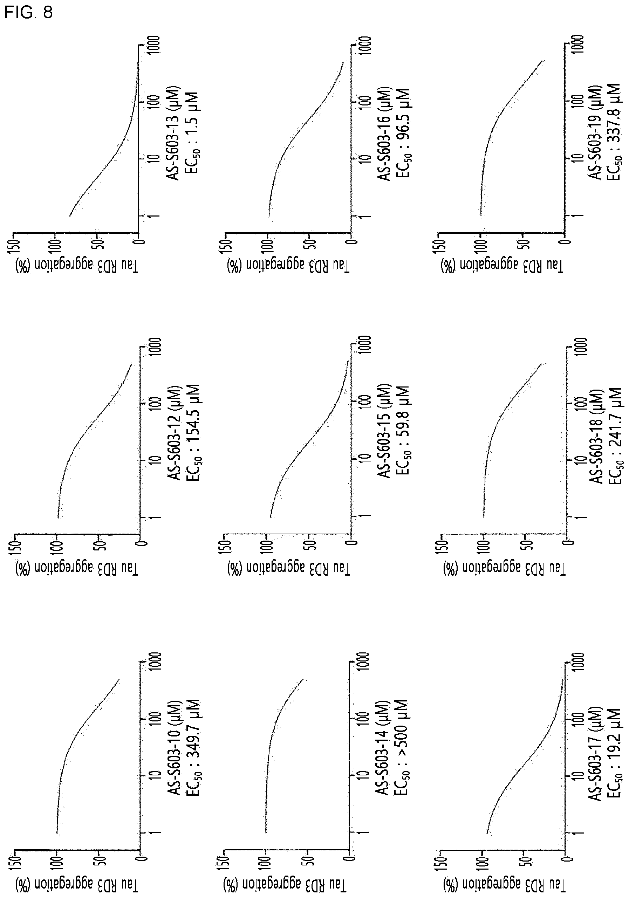

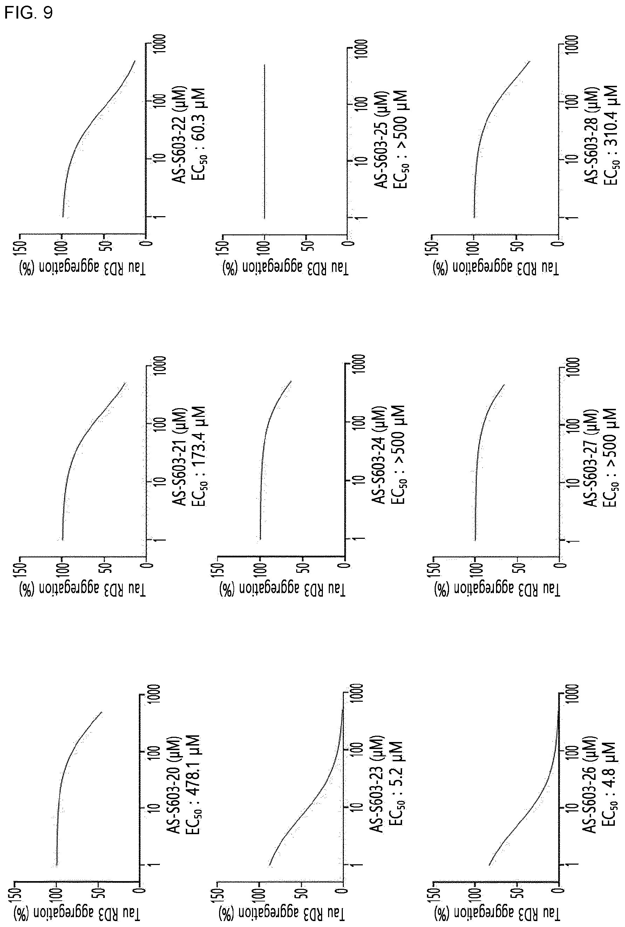

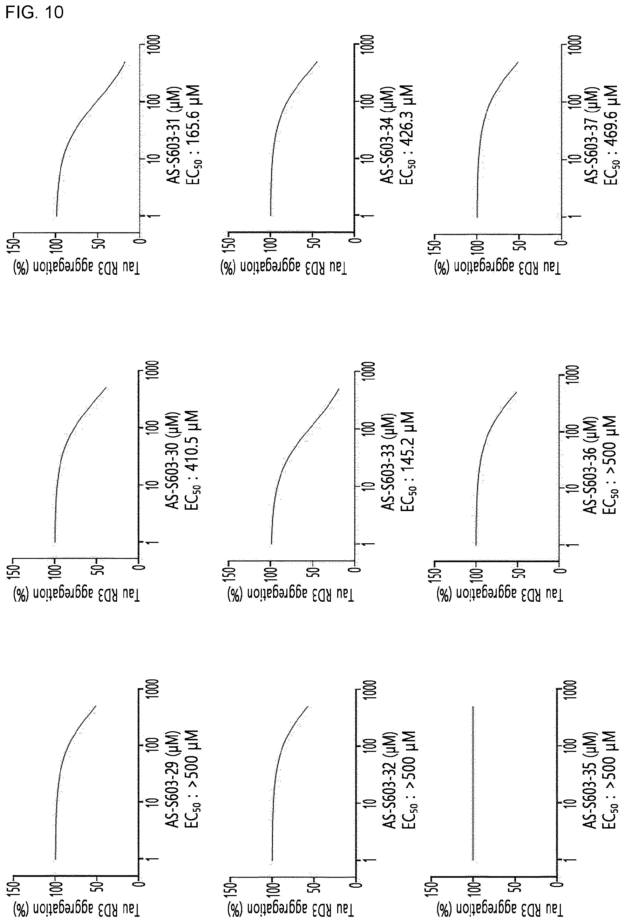

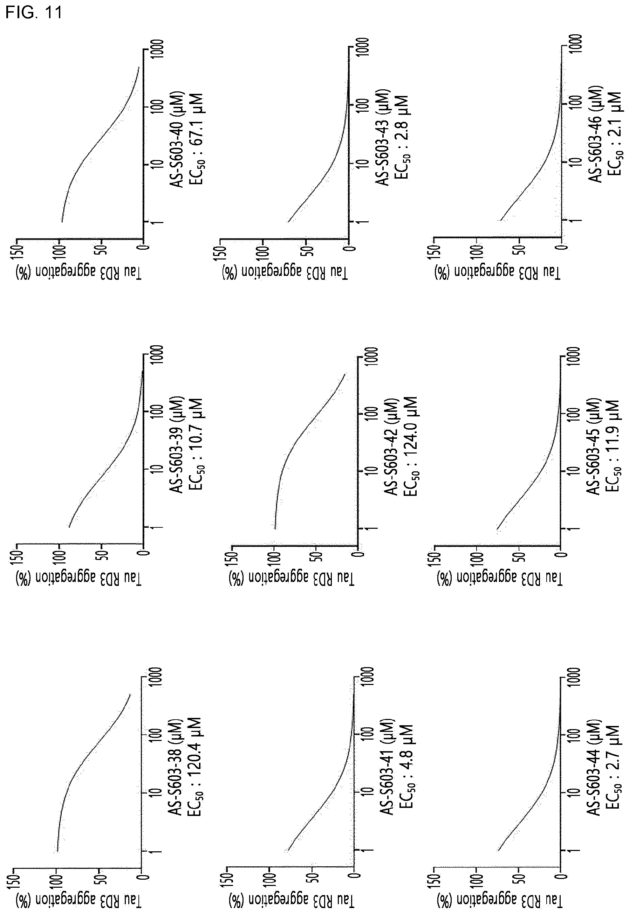

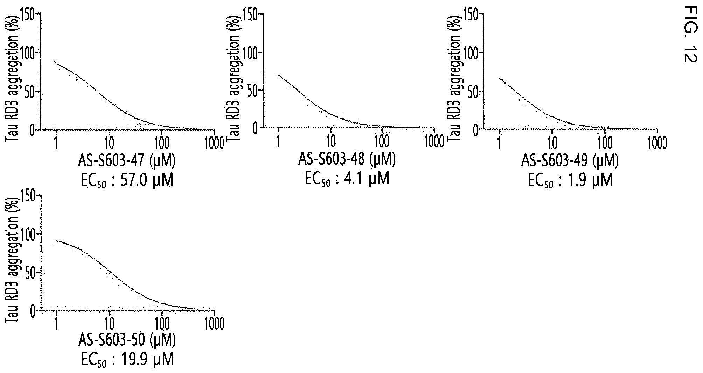

The fluorescence value of a group (control) treated with amyloid-beta or tau alone, followed by aggregation for 72 hours was converted into a percentage value and expressed as 100, and amyloid-beta and tau aggregates, in which aggregation had been completed upon reaction for 24 hours, were treated with each compound at each concentration and a further reaction was allowed to occur therebetween for 48 hours, and then fluorescence values thereof were measured and converted into relative values. The results thereof are illustrated in FIGS. 1 to 6 and 7 to 12. In addition, EC₅₀ values of the compounds of the present disclosure for amyloid-beta and tau, respectively are summarized in Table 1.

**Table 1**

| **Compound No.** | **EC₅₀ (µM)** | | **Compound No.** | **EC₅₀ (µM)** | |
|---|---|---|---|---|---|
| | **Amyloid-beta** | **Tau** | | **Amyloid-beta** | **Tau** |
| **1** | 8.2 | 19.2 | **27** | 236.8 | >500 |
| **2** | 92.9 | 321.1 | **28** | 8.3 | 310.4 |
| **3** | 3.8 | 8.5 | **29** | 7.5 | >500 |
| **4** | 75 | 156.3 | **30** | 3.7 | 410.5 |
| **5** | 65.6 | 344.1 | **31** | 12.8 | 165.6 |
| **6** | 3.2 | 16.2 | **32** | 160.8 | >500 |
| **7** | 194.5 | 132.7 | **33** | 10.4 | 145.2 |
| **8** | 1.9 | 4.2 | **34** | 31.2 | 426.3 |
| **9** | 19.1 | 53.6 | **35** | 1.0 | >500 |
| **10** | 5.6 | 349.7 | **36** | 26.0 | >500 |
| **12** | 4.3 | 154.5 | **37** | 46.3 | 469.6 |
| **13** | 110.4 | 1.5 | **38** | 3.0 | 120.4 |
| **14** | 177.2 | >500 | **39** | 2.6 | 10.7 |
| **15** | 2.5 | 59.8 | **40** | 2.1 | 67.1 |
| **16** | 12.5 | 96.5 | **41** | 1.1 | 4.8 |
| **17** | 5.7 | 19.2 | **42** | 31.2 | 124.0 |
| **18** | 26.1 | 241.7 | **43** | 1.8 | 2.8 |
| **19** | 11.6 | 337.8 | **44** | 1.8 | 2.7 |
| **20** | 11.9 | 478.1 | **45** | 9.1 | 11.9 |
| **21** | 5.2 | 173.4 | **46** | 1.4 | 2.1 |
| **22** | 18.5 | 60.3 | **47** | 1.7 | 57.0 |
| **23** | 4.1 | 5.2 | **48** | 2.3 | 4.1 |
| **24** | 99.6 | >500 | **49** | 1.5 | 1.9 |
| **25** | 7.6 | >500 | **50** | 2.3 | 19.9 |
| **26** | 2.4 | 4.8 | | | |

FIGS. 1 to 6 illustrate: the amyloid-beta disaggregation effect of each of indenone derivative compounds 1 to 50 according to treatment concentration (0-500 µM) (control: 50 µM Aβ₁₋₄₂-treated group with aggregation for 72 hours; and treated groups: aggregation of 50 µM Aβ₁₋₄₂ for 24 hours + treated with derivative compounds at each concentration and reaction for 48 hours); and a concentration (EC₅₀) at which, upon treatment with each compound, amyloid-beta can be disaggregated by 50 % compared to the control. This means that each compound exhibits a disaggregation effect in a concentration-dependent manner.

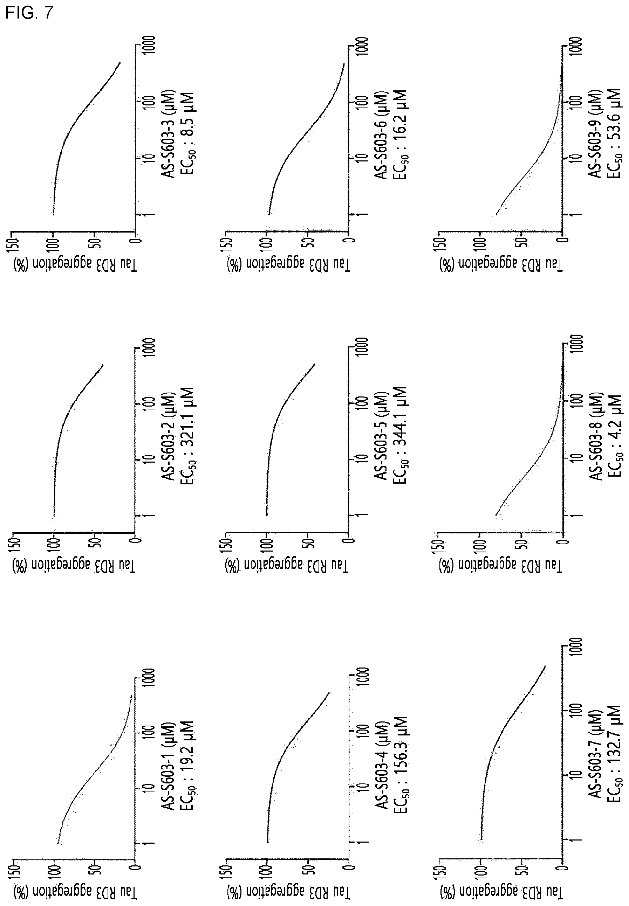

FIGS. 7 to 12 illustrate: the tau disaggregation effect of each compound according to treatment concentration (0-500 µM) (control: 35µM Tau-RD3-treated group with aggregation for 72 hours; and treated groups: aggregation of 35 µM Tau-RD3 for 24 hours + treated with derivative compounds at each concentration and reaction for 48 hours); and a concentration (EC₅₀) at which, upon treatment with each compound, tau can be disaggregated by 50 % compared to the control. This means that each compound exhibits a disaggregation effect in a concentration-dependent manner.

### Experimental Example 2. Confirmation of Effect of Reducing Amyloid Plaques by Compound Treatment in 5xFAD TG Mice

### 2.1. Preparation of 5xFAD TG Mouse Model

Transgenic mice (5xFAD TG; Alzheimer's disease animal model; B6SJL-Tg (APPSwFILon, PSEN1*M146L*L286V)6799Vas/Mmjax) and wild-type mice were obtained from Jackson Laboratory (Bar Harbor, Maine, USA). The 5xFAD TG mice were crossed with wild-type mice and maintained as double hemizygotes. All genotypes were confirmed through PCR analysis using tail DNA according to the standard PCR conditions of Jackson Laboratory. The TG mice used in the present experiment, which are a model in which cognitive decline occurs due to deposition of amyloid-beta in the brain, are known to have a deposition pattern of amyloid-beta, which is a substance deposited in the brain of actual Alzheimer's patients, that is similar to that of patients, and thus were selected due to making it easy to interpret and evaluate the experimental results. 1 mouse per plastic cage was accommodated in an animal breeding room, maintained at 21±1° C under 12 h light/12 h dark cycles, and freely fed food and water.

### 2.2. Administration of Compounds to Mouse Model

Each compound was orally administered daily at each concentration to 10-month-old 5xFAD TG mice.

A total of 6 experimental groups consisted of a wildtype (WT) group and 5 5xFAD (TG) groups, and the TG mouse groups consisted of a vehicle-administered group as a negative control and groups administered with 3 mg/kg, 10 mg/kg, 30 mg/kg, and 100 mg/kg of Compound 1.

### 2.3. Preparation of Brain Tissue Sample

After administration for 4 weeks, mice were anesthetized using 2% avertine (20 mg/g, i.p.). The mice were perfused with 0.9 % NaCl and the brains thereof were excised, and the hemibrains were fixed overnight at 4 °C in 4 % paraformaldehyde (pH 7.4).

### 2.4. Immunohistochemical Staining

The fixed brain tissues prepared in Experimental Example 2.3 were sectioned to a thickness of 30 µm to perform immunohistochemical staining. The fixed tissues were reacted with a 0.5% thioflavin-S solution for 10 minutes to stain amyloid plaques. After washing twice with 50 % ethanol and washing once with DPBS, the stained tissues were mounted on slide glasses and observed with a laser scanning confocal microscope. In addition, immunostaining was performed using 6E10 antibody, which is a specific antibody against amyloid-beta.

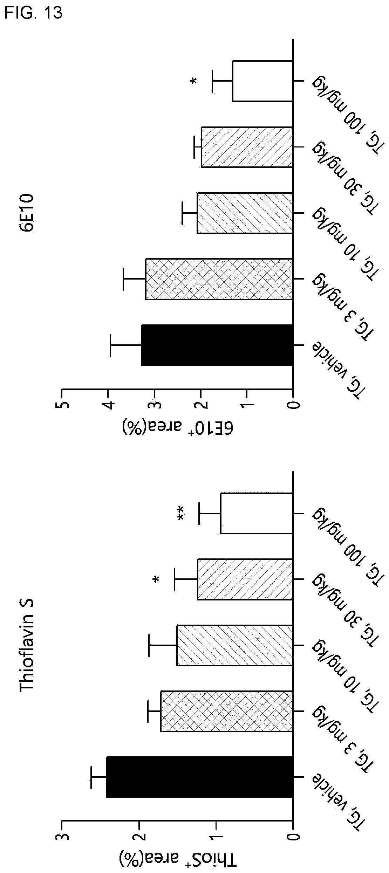

FIG. 13 illustrates graphs showing the effect of reducing amyloid plaques according to treatment with indenone derivative compounds in a 5xFAD TG mouse Alzheimer model according to an aspect.

As shown in the groups of FIG. 13 showing values obtained by calculating the total area of amyloid-beta aggregation in the brain hemisphere, it was confirmed that, compared to control mice (vehicle), the area of plaques was significantly reduced in the groups administered with the compound in a dose-dependent manner.

### Experimental Example 3. Confirmation of Effect of Reducing Tau Aggregates by Compound Treatment in PS19 TG Mice

### 3.1. Preparation of PS19 TG Mouse Model

Transgenic mice (PS19 TG; Alzheimer's disease animal model; B6; C3-Tg (Prnp-MAPT*P301S)PS19Vle/J) and wild-type mice were obtained from Jackson Laboratory (Bar Harbor, Maine, USA). The PS19 TG mice were crossed with wild-type mice and maintained as double hemizygotes. All genotypes were confirmed through PCR analysis using tail DNA according to the standard PCR conditions of Jackson Laboratory. The TG mice used in the present experiment, which are a model in which cognitive decline occurs due to deposition of tau aggregates in the brain, are known to have a deposition pattern of tau aggregates, which are a substance deposited in the brain of actual Alzheimer's patients, that is similar to that of patients, and thus were selected due to making it easy to interpret and evaluate the experimental results. 1 mouse per plastic cage was accommodated in an animal breeding room, maintained at 21±1° C under 12 h light/12 h dark cycles, and freely fed food and water.

### 3.2. Administration of Compound to Mouse Model

The compound was orally administered daily at each concentration to 6-month-old PS19 TG mice.

A total of 4 experimental groups consisted of a wildtype (WT) group and 3 PS19 (TG) groups, and the TG mouse groups consisted of a vehicle-administered group as a negative control and groups administered with 3 mg/kg and 10 mg/kg of Compound 1.

### 3.3. Preparation of Brain Tissue Sample

After administration for 12 weeks, mice were anesthetized using 2% avertine (20 mg/g, i.p.). The mice were perfused with 0.9 % NaCl and the brains thereof were excised, and the hemibrains were fixed overnight at 4 °C in 4 % paraformaldehyde (pH 7.4).

### 3.4. Immunohistochemical Staining

The fixed brain tissues prepared in Experimental Example 3.3 were sectioned to a thickness of 30 µm to perform immunohistochemical staining. The fixed tissues were reacted with a 0.5 % thioflavin-S solution for 10 minutes to stain tau aggregates. After washing twice with 50% ethanol and washing once with DPBS, the stained tissues were mounted on slide glasses and observed with a laser scanning confocal microscope. In addition, immunostaining was performed using neurofibrillary tangles (NFT) antibody, which is a specific antibody against tau aggregates.

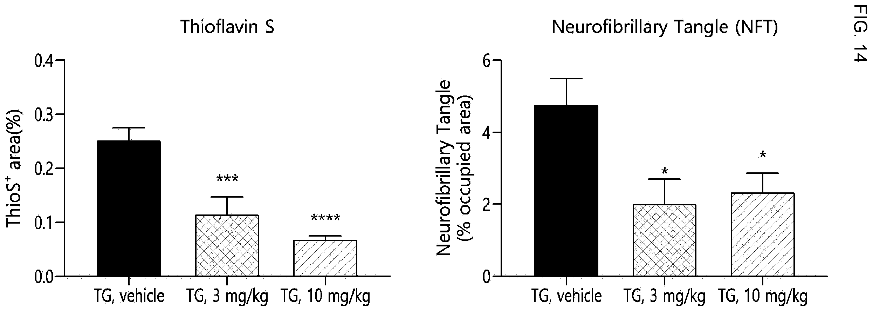

FIG. 14 illustrates graphs showing the effect of reducing amyloid plaques according to treatment with indenone derivative compounds in a PS19 TG mouse Alzheimer model according to an aspect.

As shown in the groups of FIG. 14 showing values obtained by calculating the total area of tau aggregation in the brain hemisphere, it was confirmed that, compared to control mice (vehicle), the area of tau aggregates was significantly reduced in the mice administered with the compound in a dose-dependent manner.

The above description of the present disclosure is provided only for illustrative purposes, and it will be understood by one of ordinary skill in the art to which the present disclosure pertains that the disclosure may be embodied in various modified forms without departing from the technical spirit or essential characteristics thereof. Thus, the embodiments described herein should be considered in an illustrative sense only and not for the purpose of limitation.

## Claims

1. A compound of Formula 1 below or an isomer or pharmaceutically acceptable salt thereof: 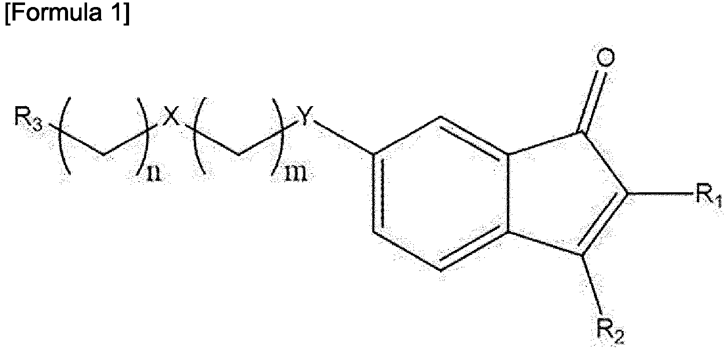 wherein, in Formula 1,
X is a direct bond or O;
Y is O or S;
n is an integer from 0 to 5;
m is an integer from 1 to 5;
R₁ is halogen, C₁₋₆ alkyl, C₆₋₁₀ aryl, -C₁₋₆ alkyl-C₆₋₁₀ aryl, or 5- or 6-membered heteroaryl, wherein the C₁₋₆ alkyl, the C₆₋₁₀ aryl, the -C₁₋₆ alkyl-C₆₋₁₀ aryl, and the 5- or 6-membered heteroaryl may each independently be unsubstituted or substituted with 1 to 4 halogens, hydroxy, CN, -NH₂, -CF₃, C₁₋₆ alkyl, -C₁₋₆ alkyl-CN, -O-C₁₋₆ alkyl, -NH-C₁₋₆ alkyl, or -N(C₁₋₆ alkyl)₂;
R₂ is 5- or 6-membered heteroaryl, wherein the 5- or 6-membered heteroaryl may be unsubstituted or substituted with 1 to 4 halogens, -CF₃, or C₁₋₆ alkyl;
R₃ is C₆₋₁₀ aryl, 5- or 6-membered heteroaryl, or 5- to 10-membered heterocyclyl, wherein the C₆₋₁₀ aryl, the 5- or 6-membered heteroaryl, and the 5-to 10-membered heterocyclyl may each independently be unsubstituted or substituted with 1 to 4 halogens, hydroxy, CN, -NH₂, -CF₃, C₁₋₆ alkyl, -C₁₋₆ alkyl-CN, -O-C₁₋₆ alkyl, -NH-C₁₋₆ alkyl, or -N(C₁₋₆ alkyl)₂; and
the heteroaryl is an aromatic heterocycle containing 1 to 4 heteroatoms selected from N, O, and S, and the heterocyclyl is an aliphatic heterocycle containing 1 to 4 heteroatoms selected from N, O, and S.

2. The compound or the isomer or pharmaceutically acceptable salt thereof of claim 1, wherein R₁ is halogen, phenyl, pyridinyl, pyrimidinyl, or thiophenyl, wherein the phenyl, the pyridinyl, the pyrimidinyl, and the thiophenyl may each independently be unsubstituted or substituted with 1 to 4 halogens, hydroxy, CN, -CF₃, C₁₋₆ alkyl, -C₁₋₆ alkyl-CN, or -O-C₁₋₆ alkyl.

3. The compound or the isomer or pharmaceutically acceptable salt thereof of claim 1 or 2, wherein R₂ is furanyl, thiophenyl, thiazolyl, or pyrazolyl, wherein the thiazolyl, the furanyl, the thiophenyl, and the pyrazolyl may each independently be unsubstituted or substituted with 1 to 4 halogens, -CF₃, or C₁₋₆ alkyl.

4. The compound or the isomer or pharmaceutically acceptable salt thereof of any one of claims 1 to 3, wherein R₃ is phenyl, pyridinyl, morpholinyl, or benzodioxolyl, wherein the phenyl, the pyridinyl, the morpholinyl, and the benzodioxolyl may each independently be unsubstituted or substituted with 1 to 4 halogens, hydroxy, -NH₂, -CF₃, C₁₋₆ alkyl, -O-C₁₋₆ alkyl, -NH-C₁₋₆ alkyl, or -N(C₁₋₆ alkyl)₂.

5. The compound or the isomer or pharmaceutically acceptable salt thereof of any one of claims 1 to 4, wherein
R₁ is halogen, phenyl, pyridinyl, pyrimidinyl, or thiophenyl, wherein the phenyl, the pyridinyl, the pyrimidinyl, and the thiophenyl may each independently be unsubstituted or substituted with 1 to 4 halogens, hydroxy, CN, -CF₃, C₁₋₆ alkyl, -C₁₋₆ alkyl-CN, or -O-C₁₋₆ alkyl,
R₂ is furanyl, thiophenyl, thiazolyl, or pyrazolyl, wherein the thiazolyl, the furanyl, the thiophenyl, and the pyrazolyl may each independently be unsubstituted or substituted with 1 to 4 halogens, -CF₃, or C₁₋₆ alkyl, and
R₃ is phenyl, pyridinyl, morpholinyl, or benzodioxolyl, wherein the phenyl, the pyridinyl, the morpholinyl, and the benzodioxolyl may each independently be unsubstituted or substituted with 1 to 4 halogens, hydroxy, -NH₂, -CF₃, C₁₋₆ alkyl, -O-C₁₋₆ alkyl, -NH-C₁₋₆ alkyl, or -N(C₁₋₆ alkyl)₂.

6. The compound or the isomer or pharmaceutically acceptable salt thereof of any one of claims 1 to 5,
wherein the compound is any one selected from the group consisting of the following compounds:
(1) 3-(4-methylthiazol-5-yl)-6-(3-phenylpropoxy)-2-(pyridin-3-yl)-1H-inden-1-one;
(2) 2-(2-(3-(4-methylthiazol-5-yl)-1-oxo-6-(3-phenylpropoxy)-1H-inden-2-yl)phenyl)acetonitrile;
(3) 2-bromo-3-(4-methylthiazol-5-yl)-6-(3-phenylpropoxy)-1H-inden-1-one;
(4) 3-(5-methylthiazol-4-yl)-6-(3-morpholinopropoxy)-2-(pyridin-3-yl)-1H-inden-1-one;
(5) 2-bromo-3-(5-methylthiazol-4-yl)-6-(3-morpholinopropoxy)-1H-inden-1-one;
(6) 6-(3-phenylpropoxy)-2-(pyridin-3-yl)-3-(thiophen-2-yl)-1H-inden-1-one;
(7) 6-(3-phenylpropoxy)-3-(1H-pyrazol-3-yl)-2-(pyridin-3-yl)-1H-inden-1-one;
(8) 3-(5-methylthiazol-4-yl)-6-(3-phenylpropoxy)-2-(pyridin-3-yl)-1H-inden-1-one;
(9) 3-(furan-3-yl)-6-(3-phenylpropoxy)-2-(pyridin-3-yl)-1H-inden-1-one;
(10) 3-(4-methylthiazol-5-yl)-2-phenyl-6-(3-phenylpropoxy)-1H-inden-1-one;
(11) 3-(4-methylthiazol-5-yl)-6-(3-phenylpropoxy)-2-(pyrimidin-5-yl)-1H-inden-1-one;
(12) 3-(4-methylthiazol-5-yl)-6-(3-phenylpropoxy)-2-(thiophen-2-yl)-1H-inden-1-one;
(13) 3-(4-methylthiazol-5-yl)-2-(pyridin-3-yl)-6-(3-(pyridin-4-yl)propoxy)-1H-inden-1-one;
(14) 3-(4-methylthiazol-5-yl)-6-phenethoxy-2-(pyridin-3-yl)-1H-inden-1-one;
(15) 3-(4-methylthiazol-5-yl)-6-(4-phenylbutoxy)-2-(pyridin-3-yl)-1H-inden-1-one;
(16) 6-((benzyloxy)methoxy)-3-(4-methylthiazol-5-yl)-2-(pyridin-3-yl)-1H-inden-1-one;
(17) 3-(4-methylthiazol-5-yl)-6-(2-phenoxyethoxy)-2-(pyridin-3-yl)-1H-inden-1-one;
(18) 2-(4-methoxyphenyl)-3-(5-methylthiazol-4-yl)-6-phenethoxy-1H-inden-1-one;
(19) 2-(4-methoxyphenyl)-3-(5-methylthiazol-4-yl)-6-(4-phenylbutoxy)-1H-inden-1-one;
(20) 6-((benzyloxy)methoxy)-2-(4-methoxyphenyl)-3-(5-methylthiazol-4-yl)-1H-inden-1-one;
(21) 2-(4-methoxyphenyl)-3-(5-methylthiazol-4-yl)-6-(2-phenoxyethoxy)-1H-inden-1-one;
(22) 2-(4-fluorophenyl)-6-(2-(4-methoxyphenoxy)ethoxy)-3-(5-methylthiazol-4-yl)-1H-inden-1-one;
(23) 6-(2-(3,4-dimethoxyphenoxy)ethoxy)-2-(4-fluorophenyl)-3-(5-methylthiazol-4-yl)-1H-inden-1-one;
(24) 6-(2-(benzo[d][1,3]dioxol-5-yloxy)ethoxy)-2-(4-fluorophenyl)-3-(5-methylthiazol-4-yl)-1H-inden-1-one;
(25) 2-(4-fluorophenyl)-3-(5-methylthiazol-4-yl)-6-(2-(pyridin-4-yloxy)ethoxy)-1H-inden-1-one;
(26) 6-(2-(3,4-dichlorophenoxy)ethoxy)-3-(5-methylthiazol-4-yl)-2-(pyrimidin-5-yl)-1H-inden-1-one;
(27) 6-(2-(3,4-difluorophenoxy)ethoxy)-3-(5-methylthiazol-4-yl)-2-(pyrimidin-5-yl)-1H-inden-1-one;
(28) 3-(5-methylthiazol-4-yl)-6-phenethoxy-2-(thiophen-2-yl)-1H-inden-1-one;
(29) 3-(5-methylthiazol-4-yl)-6-(4-phenylbutoxy)-2-(thiophen-2-yl)-1H-inden-1-one;
(30) 6-((benzyloxy)methoxy)-3-(5-methylthiazol-4-yl)-2-(thiophen-2-yl)-1H-inden-1-one;
(31) 3-(5-methylthiazol-4-yl)-6-(2-phenoxyethoxy)-2-(thiophen-2-yl)-1H-inden-1-one;
(32) 6-(2-(4-methoxyphenoxy)ethoxy)-3-(5-methylthiazol-4-yl)-2-(4-(trifluoromethyl)phenyl)-1H-inden-1-one;
(33) 6-(2-(3,4-dimethoxyphenoxy)ethoxy)-3-(5-methylthiazol-4-yl)-2-(4-(trifluoromethyl)phenyl)-1H-inden-1-one;
(34) 6-(2-(benzo[d][1,3]dioxol-5-yloxy)ethoxy)-3-(5-methylthiazol-4-yl)-2-(4-(trifluoromethyl)phenyl)-1H-inden-1-one;
(35) 3-(5-methylthiazol-4-yl)-6-(2-(pyridin-4-yloxy)ethoxy)-2-(4-(trifluoromethyl)phenyl)-1H-inden-1-one;
(36) 6-(2-(3,4-dichlorophenoxy)ethoxy)-3-(5-methylthiazol-4-yl)-2-(thiophen-2-yl)-1H-inden-1-one;
(37) 6-(2-(3,4-difluorophenoxy)ethoxy)-3-(5-methylthiazol-4-yl)-2-(thiophen-2-yl)-1H-inden-1-one;
(38) 3-(furan-3-yl)-6-phenethoxy-2-(pyridin-3-yl)-1H-inden-1-one;
(39) 3-(furan-3-yl)-6-(4-phenylbutoxy)-2-(pyridin-3-yl)-1H-inden-1-one;
(40) 6-((benzyloxy)methoxy)-3-(furan-3-yl)-2-(pyridin-3-yl)-1H-inden-1-one;
(41) 3-(furan-3-yl)-6-(2-phenoxyethoxy)-2-(pyridin-3-yl)-1H-inden-1-one;
(42) 3-(furan-3-yl)-6-(2-(4-methoxyphenoxy)ethoxy)-2-(pyridin-3-yl)-1H-inden-1-one;
(43) 6-(2-(3,4-dimethoxyphenoxy)ethoxy)-3-(furan-3-yl)-2-(pyridin-3-yl)-1H-inden-1-one;
(44) 6-(2-(benzo[d][1,3]dioxol-5-yloxy)ethoxy)-3-(furan-3-yl)-2-(pyridin-3-yl)-1H-inden-1-one;
(45) 3-(furan-3-yl)-2-(pyridin-3-yl)-6-(2-(pyridin-4-yloxy)ethoxy)-1H-inden-1-one;
(46) 6-(2-(3,4-dichlorophenoxy)ethoxy)-3-(furan-3-yl)-2-(pyridin-3-yl)-1H-inden-1-one;
(47) 6-(2-(3,4-difluorophenoxy)ethoxy)-3-(furan-3-yl)-2-(pyridin-3-yl)-1H-inden-1-one;
(48)6-(2-(4-(dimethylamino)phenoxy)ethoxy)-3-(furan-3-yl)-2-(pyridin-3-yl)-1H-inden-1-one;
(49) 3-(furan-3-yl)-6-(2-(4-isopropylphenoxy)ethoxy)-2-(pyridin-3-yl)-1H-inden-1-one; and
(50) 3-(furan-3-yl)-6-(((4-methoxybenzyl)oxy)methoxy)-2-(pyridin-3-yl)-1H-inden-1-one.

7. A pharmaceutical composition for preventing or treating a neurodegenerative brain disease, comprising, as an active ingredient, the compound or the isomer or pharmaceutically acceptable salt thereof according to any one of claims 1 to 6.

8. The pharmaceutical composition of claim 7, wherein the pharmaceutical composition is administered to a subject selected from:
(1) subjects who have a higher level or a higher risk of amyloid-beta aggregation than normal individuals not having a neurodegenerative brain disease;
(2) subjects who have a higher level or a higher risk of tau protein aggregation than normal individuals not having a neurodegenerative brain disease;
(3) subjects who have a higher level or a higher risk of phosphorylation of tau protein than normal individuals not having a neurodegenerative brain disease; and
(4) subjects corresponding to one or more of (1) to (3).

9. The pharmaceutical composition of claim 7 or 8, wherein the neurodegenerative brain disease is any one selected from the group consisting of dementia, Alzheimer's disease, preclinical Alzheimer's disease, Parkinson's disease, Huntington's disease, mild cognitive impairment, cerebral amyloid angiopathy, Down's syndrome, amyloid stroke, systemic amyloid disease, Dutch amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

10. A health functional food for preventing or ameliorating a neurodegenerative brain disease, comprising, as an active ingredient, the compound or the isomer or sitologically acceptable salt thereof according to any one of claims 1 to 6.

11. A composition comprising the compound or the isomer or acceptable salt thereof according to any one of claims 1 to 6.

12. A method of inhibiting amyloid-beta aggregation or disaggregating amyloid-beta aggregates, the method comprising administering, to a subject in need thereof, the compound or the isomer or pharmaceutically acceptable salt thereof according to any one of claims 1 to 6.

13. A method of inhibiting tau protein aggregation, disaggregating tau protein aggregates, or inhibiting the phosphorylation of tau protein, the method comprising administering, to a subject in need thereof, the compound or the isomer or pharmaceutically acceptable salt thereof according to any one of claims 1 to 6.

14. A method of treating or preventing a neurodegenerative brain disease, the method comprising administering, to a subject in need thereof, the compound or the isomer or pharmaceutically acceptable salt thereof according to any one of claims 1 to 6.

15. Use of the compound or the isomer or pharmaceutically acceptable salt thereof according to any one of claims 1 to 6 for inhibiting amyloid-beta aggregation or disaggregating amyloid-beta aggregates.

16. Use of the compound or the isomer or pharmaceutically acceptable salt thereof according to any one of claims 1 to 6 for inhibiting tau protein aggregation, disaggregating tau protein aggregates, or inhibiting the phosphorylation of tau protein.

17. Use of the compound or the isomer or pharmaceutically acceptable salt thereof according to any one of claims 1 to 6 for treating or preventing a neurodegenerative brain disease.
